(19)

Europäisches
Patentamt

European
Patent Office

Office européen
des brevets

(11) **EP 4 451 275 B1**

(12) **EUROPEAN PATENT SPECIFICATION**

(45) Date of publication and mention
of the grant of the patent:
**17.09.2025 Bulletin 2025/38**

(21) Application number: **24154637.3**

(22) Date of filing: **30.01.2024**

(51) International Patent Classification (IPC):
**G16B 20/00** (2019.01)    **G16B 40/20** (2019.01)
**G16H 50/20** (2018.01)    **G16H 50/30** (2018.01)

(52) Cooperative Patent Classification (CPC):
**G16B 40/20; G16B 20/00; G16H 50/20;
G16H 50/30**

(54) **METHODS AND SYSTEMS FOR PREDICTING A CATEGORY OF MAMMOGRAPHIC BREAST
DENSITY FOR A SUBJECT**

VERFAHREN UND SYSTEME ZUR VORHERSAGE EINER KATEGORIE VON
MAMMOGRAPHISCHER BRUSTDICHTE FÜR EINE PERSON

PROCÉDÉS ET SYSTÈMES DE PRÉDICTION D'UNE CATÉGORIE DE DENSITÉ MAMMAIRE
MAMMOGRAPHIQUE POUR UN SUJET

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **19.04.2023 IN 202321028614**

(43) Date of publication of application:
**23.10.2024 Bulletin 2024/43**

(73) Proprietor: **Tata Consultancy Services Limited
Maharashtra (IN)**

(72) Inventors:
• **BOSE, CHANDRANI
411013 Pune - Maharashtra (IN)**
• **HAQUE, MOHAMMED MONZOORUL
411013 Pune - Maharashtra (IN)**
• **SINGH, RASHMI
411013 Pune - Maharashtra (IN)**
• **MANDE, SHARMILA SHEKHAR
411013 Pune - Maharashtra (IN)**
• **CHENNAREDDY, VENKATA SIVA KUMAR REDDY
411013 Pune - Maharashtra (IN)**

(74) Representative: **Goddar, Heinz J.
Boehmert & Boehmert
Anwaltspartnerschaft mbB
Pettenkoferstrasse 22
80336 München (DE)**

(56) References cited:
• **JONES GIEIRA S. ET AL: "Mammographic breast
density and its association with urinary
estrogens and the fecal microbiota in
postmenopausal women", PLOS ONE, vol. 14,
no. 5, 8 May 2019 (2019-05-08), US, pages
e0216114, XP093181481, ISSN: 1932-6203, DOI:
10.1371/journal.pone.0216114**
• **YOON LARA S. ET AL: "The Association
Between Breast Density and Gut Microbiota
Composition at 2 Years Post-Menarche: A Cross-
Sectional Study of Adolescents in Santiago,
Chile", FRONTIERS IN CELLULAR INFECTION
MICROBIOLOGY, vol. 11, 17 December 2021
(2021-12-17), CH, XP093181483, ISSN: 2235-2988,
DOI: 10.3389/fcimb.2021.794610**
• **YAGHJYAN LUSINE ET AL: "Gut microbiome,
body weight, and mammographic breast density
in healthy postmenopausal women", CANCER
CAUSES, SPRINGER INTERNATIONAL
PUBLISHING, CHAM, vol. 32, no. 7, 27 March
2021 (2021-03-27), pages 681 - 692,
XP037474804, ISSN: 0957-5243, [retrieved on
20210327], DOI: 10.1007/S10552-021-01420-6**
• **ÁLVAREZ-MERCADO ANA ISABEL ET AL: "Gut
Microbiota and Breast Cancer: The Dual Role of
Microbes", CANCERS, vol. 15, no. 2, 10 January
2023 (2023-01-10), CH, pages 443, XP093181618,
ISSN: 2072-6694, DOI: 10.3390/cancers15020443**

EP 4 451 275 B1

**(Cont. next page)**

- MANN RITSE M ET AL: "Breast cancer screening in women with extremely dense breasts recommendations of the European Society of Breast Imaging (EUSOBI)", EUROPEAN RADIOLOGY, SPRINGER BERLIN HEIDELBERG, BERLIN/HEIDELBERG, vol. 32, no. 6, 8 March 2022 (2022-03-08), pages 4036 - 4045, XP037839416, DOI: 10.1007/S00330-022-08617-6
- AN JEONGSHIN ET AL: "Prediction of breast cancer using blood microbiome and identification of foods for breast cancer prevention", vol. 13, no. 1, 29 March 2023 (2023-03-29), US, XP093181479, ISSN: 2045-2322, Retrieved from the Internet <URL:https://www.nature.com/articles/s41598-023-32227-x> DOI: 10.1038/s41598-023-32227-x

Remarks:
The complete document including Reference Table(s) and the Sequence Listing(s) can be downloaded from the EPO website

**Description**

TECHNICAL FIELD

The disclosure herein generally relates to the field of breast cancer and, more particularly, to methods and systems for predicting a category of mammographic breast density (MBD) for a subject using a microbial profile obtained from a biological sample of the subject.

BACKGROUND

Breast Cancer is world's most prevalent cancer amongst women. With close to eight million active cases worldwide, breast cancer currently stands as the world's most prevalent cancer. In fact, approximately one-fourth of these cases were diagnosed in the year 2020 alone. The most important strategy to fight breast cancer lies in its "early detection", like most other forms of cancer. Early screening/ detection and treatment of affected individuals are known to significantly increase the chance of survival. However, lack of any clear symptoms in most cases makes the early detection/ early diagnosis/ early screening challenging. Although detection of breast cancer (in early stages of the disease) can help physicians to take appropriate therapeutic steps to cure the disease, detecting the disease at an advanced stage makes the therapy and disease management difficult and advanced stages also significantly impacts the quality and expectancy of life of affected individuals. In the past decade, the role of 'human microbiota' (i.e., the assembly/ community of microbes present on and within the human body) has been significantly discussed in the context of etiology, severity, and the progression dynamics) of several diseases including breast cancer. For instance, imbalance in gut microbiota has been shown to contribute to the development of breast cancer through estrogen-dependent mechanisms.

In the context of risk factors for breast cancer, besides genetics and other aspects, high breast density is considered as one of the independent risk factors for breast cancer. Women with high Mammographic Breast Density (MBD) are reported to have a three times increased risk of breast cancer than women with low MBD. Dense breast tissue also leads to high false positives (due to obscured visibility of cancerous breast lesions) and limits the sensitivity of mammography (which is the most widely used imaging-based method for breast cancer screening for detection of the presence of cancerous breast lesions. Furthermore, recent clinical guidelines also restrain the employment of mammography (as a screening technique for detecting cancerous lesions in the breast) in women under 40 years of age (unless they have a familial history of breast cancer) due to concerns over excessive (or unnecessary/ unintended) levels of radiation exposure. In addition to the proposed association between microbiome and breast cancer, a few recent studies have also suggested that an altered gut microbiome pattern is observed in women having varied breast density.

The state-of-art diagnostic/ screening methods (or strategies or tests or downstream methods) employed/ clinically suggested for detecting breast cancer vary depending on the type of exhibited symptoms, cancer type suspected by oncologist/ physician as well as other factors like age, medical condition, familial history, etc. The currently available tests belong to two broad categories - (i) imaging-based tests viz., - mammography, ultrasound, and MRI; (ii) biopsy or histopathology-based tests. Among mentioned image-based breast cancer screening methods, mammography i.e., analyzing x-rays of the breast for identifying anomalous (non-palpable) changes in breast tissue indicating possible risk of breast cancer, is currently the most widely used.

While the imaging-based tests are prescribed for initial diagnosis of any abnormal growth (or lesions) in the breast, biopsy is mandatory for further confirmation/ tumor characterization of test positive cases. Each of the mentioned imaging-based methods/ tests, although widely prescribed/ recommended by clinicians world-wide, are limited by one or more of factors like technical shortcomings, concerns about radiation exposure, associated costs, and physical (and psychological) discomfort to the subject while undergoing the screening procedure.

The above-mentioned factors contribute towards significantly delayed diagnosis of breast cancer. Lack of awareness and cultural hindrance towards the imaging-based methodologies also limit the overall utility of available early screening tests/ methods. For example, although mammography as a technique has improvised over the years, a few grave limitations still exist. These include reporting of false positives (thereby causing immense psychological stress and anxiety in healthy women who end up getting wrongly flagged as suspect cases), over-diagnosis (i.e., detection of benign lesions which would never have affected woman's health/ life, and such diagnosis resulting in subjecting women to needless additional imaging, biopsies, therapies, and all related side effects), and problems due to excessive (and unwanted) radiation exposure

False positives in mammography are more common in women with high mammographic breast density (MBD). In women with higher MBD, the higher proportion of connective and epithelial tissue in their breasts as compared to radiologically translucent adipose tissue (i.e., fat) obscures the visibility (and thereby hinders detection) of breast cancer lesions (if existing). These lesions generally appear as whiter areas on mammograms (similar in contrast to the relatively light background created by dense breast tissues). In this context, it may also be noted that apart from the problem of false positives, pain due to breast compression and the need for women to undress (from the waist up) during mammography makes it an uncomfortable and rather embarrassing choice for women in several socio-cultural contexts across the world. Furthermore, routine check-ups via mammography are also not medically advised given associated hazards of excessive radiation exposure.

On a different note, MBD-associated false positive calls (via mammography) are also likely to be more in younger women (<40 years) given a lower ratio of adipose tissue to other breast tissues (as compared to relatively older women). Several studies have indicated that the sensitivity of mammography remains significantly lower (around only 70%) in cases of high MBD. The mentioned aspects therefore make it practically infeasible to employ mammography as an "early" and "routine" screening technique in younger (premenopausal) women too. In fact, recent guidelines restrain the employment of mammography in women under 40 years unless they have a familial history of breast cancer or have detected physical abnormalities necessitating advanced mammographic examination. In this context, it is interesting to note that women with high MBD (as compared to baseline ranges of similar-aged peers) were observed to have a 5-fold higher risk of breast cancer. In fact, MBD is now listed as an independent risk factor for breast cancer, and it is increasingly being viewed as a potential surrogate marker for breast cancer development.

Overall, the above context indicates the need for newer techniques that can reliably indicate breast cancer risk in women (irrespective of their age/ menopausal status) via assessment of MBD. To facilitate its applicability in a mass breast cancer 'risk-screening' context, the technique should ideally be non-invasive in nature and non-radiation based (unlike mammography). Ensuring the latter aspect would render the technique amenable for routine application in clinical (and/or non-clinical) settings as a non-invasive, non-harmful pre-adjunct (screening/ diagnostic) to mammography. In other words, only the subset of women identified as 'at-risk' (via such a companion technique) can technically be advised to undergo mammography (or advanced ultrasound/ MRI methods) based on the MBD dependent 'risk-score' categorization/ assessment.

Of late, a large corpus of recent research points towards several tractable (and potentially translatable) associations between the structure of human microbiota with various states of human health and disease. Briefly, the human microbiota refers to microbial communities (comprised of diverse kinds of bacterial, archaeal species, etc. in varying proportions) that live on or within us. In the specific context of breast cancer, there are several reports indicating distinct associations between the structure and function of microbiota resident at two specific body sites (viz. breast tissues and the gut) with the presence and/ or stage of breast cancer. For instance, a recent study by Goedert et al., (2016) demonstrates compositional differences and a statistically significant reduction in diversity of gut microbiota in post-menopausal women diagnosed with breast cancer.

Since MBD status is a potential proxy for assessing breast cancer risk, a possible solution would be building a machine learning model (based on microbiome abundance patterns from training biological samples collected from a subject cohort having varying status/ levels of MBD) that would help categorizing women (undergoing a microbiota-based screening test) into three risk categories viz., low, medium, and high). The latter two categories would indicate possible high MBD or disease risk status for the tested individual and can be used for recommending the individual to go for 3D-mammography or other suitable (advanced) imaging-based techniques. On the other hand, for women categorized as low risk via this 'non-invasive' microbiota-based technique, mammography can be suggested as the sensitivity of mammography is higher in case of low breast density (as described in previous paragraphs).

In summary, microbiota-analysis mediated assessment of MBD status and possible breast cancer risk has the following advantages over existing breast cancer risk/ MBD status ascertaining techniques.

(a) Non-invasive methodology: Unlike invasive techniques like mammography or biopsies, microbiota sampling is non-invasive (involving just the collection of a stool sample from the individual undergoing a test to assess breast cancer risk). This therefore renders the method feasible for routine (mass) screening.

(b) Non-harmful: Microbiota-based method also does not pose any harm to the users either physically (e.g., radiation hazard) or psychologically (e.g., disrobing-related embarrassment, as in mammography).

(c) Ease of microbiota sample collection makes it potentially deployable as a DIY home-based collection method. Availability of specially designed stool sample collection tubes (with pre-made reagents & buffers mix) makes it feasible to collect & transfer stool samples from even remote rural settings.

(d) Economic screening method upon large scale deployment: Once transferred, multiple samples can be pooled, sequenced, and analyzed in batch mode to generate risk assessment reports within a couple of days. With the costs of sequencing coming down, the cost of a microbiota analysis-based screening test (in near future) should ideally be not more than INR 2000-3000 (if not lesser, when done in a mass scale).

(e) No-age restrictions for testing: Given that the method involves a non-invasive sampling procedure, the method can technically be employed on women across any age groups. The method therefore clearly scores over other existing screening methods, wherein existing medical guidelines suggest screening of women only after 40-45 years of age. A microbiota analysis mediated 'non-invasive' method especially becomes useful in case of younger women (a huge chunk of population), wherein currently there is limited availability of safe and early breast cancer risk screening methods. Overall, the method caters to both pre/ peri as well as post-menopausal women.

(f) Enables increased frequency of testing: The method, being non-invasive can be used multiple times (as and when required) for screening risk. This feature is especially advantageous for routinely monitoring risk in women who are known to have a family history of breast cancer or are known to be genetically susceptible.

In addition to its utility as a possible screening methodology by itself (or as a companion diagnostic to mammography), a 'microbiota analysis' based MBD and breast cancer risk assessment method finds additional application as a potential surrogate method for tracking treatment outcomes. Given that higher MBD is recognized as a strong risk factor for breast cancer, MBD assessment (via a microbiota-based approach) can potentially and routinely be employed as a non-invasive technique to indicate the effectiveness of breast cancer therapy methods in providing better prognosis/ treatment outcomes.

Jones Gieira S. ET AL: "Mammographic breast density and its association with urinary estrogens and the fecal microbiota in postmenopausal women" discloses breast density, as estimated by mammography, is a strong risk factor for breast cancer in pre- and postmenopausal women, but the determinants of breast density have not yet been established. The aim of this study was to assess if urinary estrogens or gut microbiota alterations are associated with mammographic density in postmenopausal women. Among 54 cancer-free, postmenopausal controls in the Breast and Colon Health study, we classified low- versus high-density women with Breast Imaging Reporting and Data System (BI-RADS, 5th edition) mammographic screening data, then assessed associations with urinary estrogens and estrogen metabolites (determined by liquid chromatography/tandem mass spectrometry), and fecal microbiota alpha and beta diversity (using Illumina sequencing of 16S rRNA amplicons). Multiple logistic regression revealed no significant association between breast density and fecal microbiota metrics (PD_tree P-value = 0.82; un-weighted and weighted UniFrac P = 0.92 and 0.83, respectively, both by MiRKAT). In contrast, total urinary estrogens (and all 15 estrogens/estrogen metabolites) were strongly and inversely associated with breast density (P = 0.01) after adjustment for age and body mass index. Mammographic density was not associated with the gut microbiota, but it was inversely associated with urinary estrogen levels (Abstract).

YAGHJYAN LUSINE ET AL: "Gut microbiome, body weight, and mammographic breast density in healthy postmenopausal women" discloses examined gut microbiome (GM) profiles in relation to mammographic breast density (BD) and body mass index (BMI) in healthy postmenopausal women. Eligible women were postmenopausal, had a BMI ≤ 35 kg/m$^2$, and had not recently taken oral/IV antibiotics. All women provided a fecal sample and information on breast cancer risk factors. Mammographic BD was classified with the American College of Radiology's BI-RADS BD classification system. Bacterial DNA was isolated from fecal samples and the V1-V2 hypervariable regions of 16S rRNA were sequenced on the Illumina MiSeq platform. We examined associations of GM with indices of within-sample (alpha) diversity and the ratio of the two main phyla (Firmicutes and Bacteroidetes; F/B ratio) with BD and BMI. Among 69 women with BD data, 39 had low BD (BI-RADS I/II) and 30 had high BD (BI-RADS III/IV). BMI was inversely associated with BD (mean BMI = 23.8 and 28.0 in women with high and low BD, respectively, p = 1.07 × 10$^{-5}$). Similar levels of GM diversity were found across weight groups according to Shannon (p = 0.83); Inverse Simpson (p = 0.97); and Chao1 (p = 0.31) indices. F/B ratio and microbiota diversity were suggestively greater in women with high vs. low BD (p = 0.35, 0.14, 0.15, and 0.17 for F/B ratio, Shannon, Inverse Simpson and Chao1, respectively). Suggestive differences observed in women with high and low BD with respect to GM alpha diversity and prevalence of specific GM taxa need to be confirmed in larger studies (Abstract).

SUMMARY

Embodiments of the present disclosure present technological improvements as solutions to one or more of the above-mentioned technical problems recognized by the inventors in conventional systems.

In one embodiment, a method for predicting a category of mammographic breast density (MBD) for a subject is provided. The invention is set out in the appended set of claims 1-12.

In another aspect, a kit for predicting a category of mammographic breast density (MBD) for a subject is provided. The invention is set out in the appended claim 13.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for predicting a category of mammographic breast density (MBD) for a subject, according to some embodiments of the present disclosure.

FIGS. 2A and 2B are flowcharts illustrating a method for predicting a category of mammographic breast density (MBD) for a subject, according to some embodiments of the present disclosure.

FIG. 3 illustrates an exemplary probe and multiplexed qPCR design for detecting and determining a quantitative abundance of each of a plurality of predetermined microbes associated with the biological sample, according to some embodiments of the present disclosure.

FIGS. 4A, 4B and 4C are flowcharts illustrating steps involved in building a pre-determined machine learning model, according to some embodiments of the present disclosure.

FIGS. 5A, 5B and 5C are flowcharts illustrating steps involved in calculating a pair of predefined threshold values, according to some embodiments of the present disclosure.

FIG. 6 illustrates an exemplary block diagram of a kit for predicting a category of mammographic breast density (MBD) for a subject, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Microbiota: The collection of microorganisms, such as, bacteria, archaea, protists, fungi, and virus, that inhabit a particular niche or geographical site.

Microbiome: The collection of genetic material of micro-organisms that reside in a particular geographical niche.

Mammographic breast Density (MBD): the amount of dense tissue of an entire breast as detected by mammography. It allows physicians to evaluate the proportion of dense to non-dense tissue.

Probiotics: A micro-organism or a collection of micro-organisms introduced into the body for its beneficial qualities.

Prebiotics: A non-digestible food or food component that promotes the growth of beneficial micro-organisms in the gut.

The conventional techniques used for evaluating breast density primarily include mammography-based techniques. Once a set of images is generated through mammography, a tool that is widely used to infer the results is called BI-RADS (Breast imaging-reporting and data system) and thus, BI-RADS categorization of mammographic breast density is a universally accepted measurement unit. BI-RADS tool can also be used to interpret the outcomes of other imaging techniques such as, ultrasound and MRI. Thus, in context of evaluating breast density, the limitations of the state-of-art imaging techniques mentioned above also applies to the breast density categorization through BI-RADS. In addition, since the final categorization depends on visual review of the mammogram by a radiologist the report may vary when inspected by a different radiologist.

The present disclosure provides methods and systems for predicting a risk category of mammographic breast density (MBD) for a subject, where the subject is a woman. The prediction of breast density is microbiota-based method which can help in choosing the appropriate downstream imaging technique for detection of cancerous lesions.

The present disclosure, primarily being non-invasive and having no radiation hazards (which is one of the major limitations of mammography), is utilized for providing early information (and assessment) with respect to MBD, a known risk factor to breast cancer predisposition. This helps an individual or care provider to take or undergo or provide precautionary/ corrective medical advice/ procedures to reduce/ obviate the risk as well as it might also help in prognosis of the cancer patients with ongoing cancer treatments. With these advantages of the present disclosure, it may prove its appropriateness to be employed as an optimal method in a mass screening setup/context.

The present disclosure utilizes the gut microbiome, i.e., the assemblage of the genetic material of the microbes residing in gut, which is not dependent on any imaging techniques. The present invention does not base its predictions on the mere abundance changes of any/ specific bacterial pathogens. The methodology is adapted for computation/ quantification of the microbial variation involved in breast cancer aetiology and deriving a personalized prediction score for assessing the risk of breast cancer as well as categorization of breast density.

Further, the present disclosure proposes an approach for early and non-invasive methods for predicting/ assessing the risk of breast cancer in a woman by analyzing the gut microbiota (represented via a stool sample). The invention extends to determining breast density category and recommending an appropriate imaging technique for detection of any possible cancerous lesion. The present disclosure further provides a scheme that can be followed to better understand the requirement of microbiome test or the type of diagnostic technique for optimal outcome.

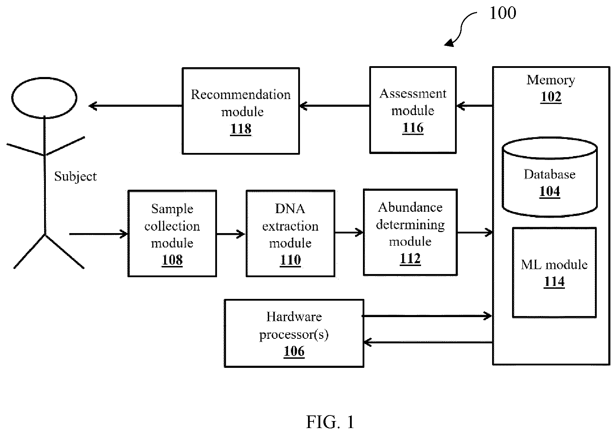

Referring now to the drawings, and more particularly to FIG. 1 through FIG. 6, where similar reference characters denote corresponding features consistently throughout the figures, there are shown preferred embodiments and these embodiments are described in the context of the following exemplary system and/or method.

FIG. 1 illustrates an exemplary block diagram of a system 100 for predicting a category of mammographic breast density (MBD) for a subject, according to some embodiments of the present disclosure. In an embodiment, the system 100 includes a memory 102, a database 104, one or more hardware processors 106, a sample collection module 108, a DNA extraction module 110, an abundance determining module 112, a machine learning (ML) module 114, an assessment module 116, and a recommendation module 118. In an embodiment, the database 104 and the machine learning (ML) module 114 are stored in the memory 102.

In an embodiment, the sample collection module 108 is configured to collect a biological sample of the subject whose category of mammographic breast density (MBD) is to be assessed. The subject is being a woman. The DNA extraction module 110 is configured to extract microbial deoxyribonucleic acid (DNA) sequences from the biological sample. The abundance determining module 112 is configured to determine a quantitative abundance of each of a plurality of predetermined microbes associated with the biological sample, using the microbial DNA.

The machine learning (ML) module 114 is configured to determine a model score based on a microbial abundance matrix which is obtained by collating the quantitative abundances of each of the plurality of predetermined microbes. The assessment module 116 is configured to predict the category of mammographic breast density (MBD) for the subject, based on the model score. Lastly, the recommendation module 118 is configured to design a personalized therapeutic recommendation for the subject based on the risk category (healthy or having breast cancer) assessed for the subject.

In an embodiment, the one or more hardware processors 106 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 106 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 104 configured to include information regarding risk assessment of breast cancer present in the subject. The memory 102 may comprise information pertaining to input(s)/output(s) of each step performed by the one or more hardware processors 106 of the system 100 and methods of the present disclosure. In an embodiment, the database 104 may be external (not shown) to the system 100 and coupled to the system 100 via the I/O interfaces (not shown in FIG. 1).

In an embodiment, one or more data storage devices or the memory 102 operatively coupled to the one or more hardware processors 106. The system 100 with the one or more hardware processors 106 is configured to execute functions of one or more functional modules of the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

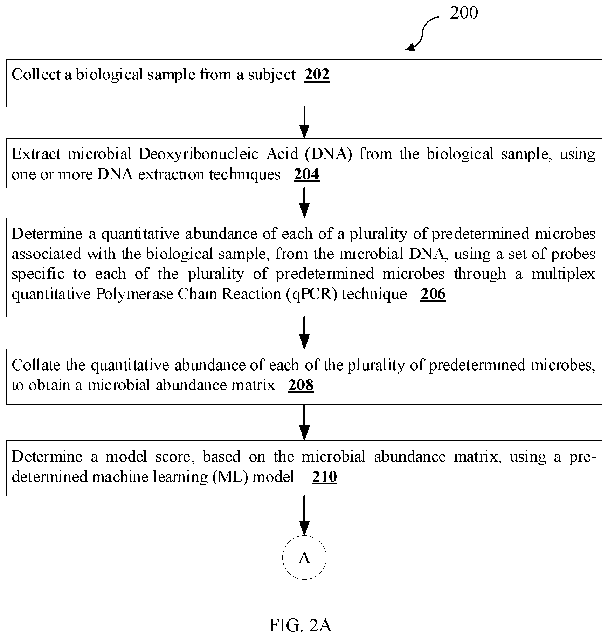

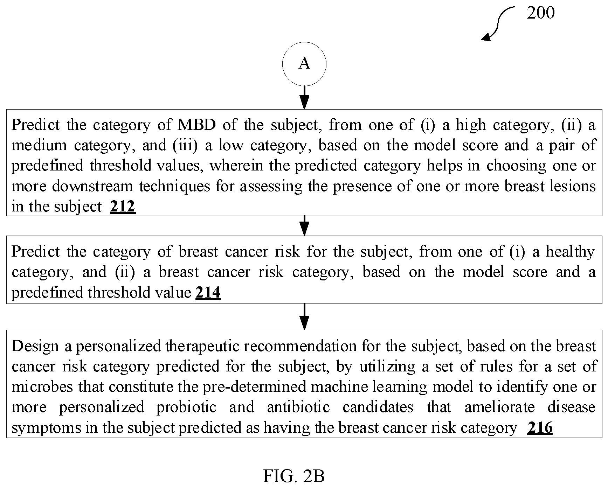

In an embodiment, the memory 106 comprises one or more data storage devices operatively coupled to the one or more hardware processors 106 and is configured to store instructions for execution of steps of the method depicted in FIGS. 2A and 2B by the one or more hardware processors 106. FIGS. 2A and 2B are flowcharts illustrating a method 200 for predicting the category of mammographic breast density (MBD) for the subject, according to some embodiments of the present disclosure.

The steps of the method 200 of the present disclosure will now be explained with reference to the components or blocks of the system 100 as depicted in FIG. 1 and the steps of flow diagrams as depicted in FIGS. 2A and 2B. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the biological sample of the subject whose category of mammographic breast density (MBD) is to be predicted, is collected through the sample collection module 108. In an embodiment, the subject or the individual is a human being and particularly a woman. The biological sample is a test biological sample which is tested for predicting the category of the subject. The biological sample is at least one of a stool sample, a gastrointestinal tract (gut) sample, a saliva sample, and a urine sample. The type of sample is collected based on the subject belonging to a specific region, geography, and ethnicity, and further based on the standard medical procedure followed in the specific region.

In an embodiment, the biological sample can be collected from a body site/location other than gut such as saliva, urine etc. Samples from healthy and diseased individuals, from any other mammalian organism are covered in the scope of this invention.

Further, at step 204 of the method 200, microbial deoxyribonucleic acid (DNA) or DNA sequences from the biological sample collected at step 202 of the method 200, is extracted through the DNA extraction module 110. The DNA extraction module 110 includes one or more DNA extraction techniques. In an embodiment, the extraction of microbial DNA from the biological sample is performed by amplification of 16S rRNA marker genes (either full-length or specific variable regions of the gene) using one or more of: a next-generation sequencing (NGS) platform, Oxford nanopore sequencing or any other DNA sequencing technique and platform (including a classical Sanger sequencing).

In another embodiment, the NGS platforms include any one of whole genome sequencing, CPN60 gene-based amplicon sequencing, other phylogenetically conserved genetic region-based amplicon sequencing, sequencing using approaches which involve either a fragment library or a mate-pair library or a paired-end library or a combination of the same.

Further, at step 206 of the method 200, the quantitative abundance of each of a plurality of predetermined microbes associated with the biological sample, is determined, from the microbial DNA or DNA sequences extracted at step 204 of the method 200. The plurality of predetermined microbes is the set of microbes whose quantification (i.e., determining the abundance) is performed in the corresponding sample.

In an embodiment, the plurality of predetermined microbes associated with the biological sample comprises of *Murimonas, Clostridium sensu stricto, Clostridium XIVa, Lachnospiracea incertae sedis, Blautia, Bacteroides, Intestinibacter,* and *Bilophila*. The microbes having 16S (or any other phylogenetic marker) gene sequences of >=95% sequence similarity and >=95% coverage with the corresponding 16S (or any other phylogenetic marker) gene sequences of the predetermined microbes are also under the scope of this invention. Newly assigned nomenclature of the predetermined microbes due to modifications in the classification database are also under the scope of the present invention.

The sequence listing corresponding to a set of microbial organisms belonging to the predetermined microbes are listed below:

>NR_134772.1 Murimonas intestini strain SRB-530-5-H 16S ribosomal RNA, partial sequence:

TGCAGTCGAGCGGAGTATTTAACGGAAGTCTTCGGATGGAAGTTTG
GATACTTAGCGGCGGACGGGTGAG

TAACGCGTGGGCAACCTGCCCCATACCGGGGGATAACAGCCAGAA
ATGGCTGCTAATACCGCATAAGCGC

ACAGCGCCACATGGCGCGGTGTGAAAAACTCCGGTGGTATGGGAT
GGGCCCGCGTCTGATTAGGTAGTTG

GCGGGGTAACGGCCCACCAAGCCGACGATCAGTAGCCGACCTGAG
AGGGTGACCGGCCACATTGGGACTG

AGACACGGCCCAAACTCCTACGGGAGGCAGCAGTGGGGAATATTG
CACAATGGGGGAAACCCTGATGCAG

CGACGCCGCGTGAGCGAAGAAGTATTTCGGTATGTAAAGCTCTAT
CAGCAGGGAAGAAAATGACGGTACC

TGACTAAGAAGCCCCGGCTAACTACGTGCCAGCAGCCGCGGTAAT
ACGTAGGGGGCAAGCGTTATCCGGA

TTTACTGGGTGTAAAGGGAGCGTAGACGGCCAGGCAAGTCTGATG
TGAAAGGCAGGGGCTCAACCCCTGG

ACTGCATTGGAAACTGTCAGGCTGGAGTGCCGGAGAGGTAAGCGG
AATTCCTAGTGTAGCGGTGAAATGC

GTAGATATTAGGAGGAACACCAGTGGCGAAGGCGGCTTACTGGAC
GGTAACTGACGTTGATGCTCGAAAG

CGTGGGGAGCAAACAGGATTAGATACCCTGGTAGTCCACGCCGTA
AACGATGAATACTAGGTGTCGGGGG

GCAAAGCCCTTCGGTGCCGCCGCAAACGCATTAAGTATTCCACCTG
GGGAGTACGTTCGCAAGAATGAAA

CTCAAAGGAATTGACGGGGACCCGCACAAGCGGTGGAGCATGTGG
TTTAATTCGAAGCAACGCGAAGAAC

CTTACCAAGCCTTGACATCCCATTGAAGCCAGAGTAACGTCTGACG
GCCTTCGGGACAATGGAGACAGGT

GGTGCATGGTTGTCGTCAGCTCGTGTCGTGAGATGTTGGGTTAAGT
CCCGCAACGAGCGCAACCCTTATC

TTCAGTAGCCAGCACGCGATGGTGGGCACTCTGGAGAGACTGCCA
GGGACAACCTGGAGGAAGGTGGGGA

TGACGTCAAATCATCATGCCCCTTATGGCTTGGGCTACACACGTGC
TACAATGGCGTAAACAGAGGGAAG

CAGCCCTGCGAAGGTGAGCGAATCCCAAAAATAACGTCTCAGTTC
GGATTGTAGTCTGCAACTCGACTAC

ATGAAGCTGGAATCGCTAGTAATCGCGAATCAGAATGTCGCGGTG
AATACGTTCCCGGGTCTTGTACACA

CCGCCCGTCACACCATGGGAGTCAGTAACGCCCGAAGTCAGTGAC
CTAAACCGAAAGGAAGGAGCTGCCG

AAGGCGGGACTGGTAACTGGGGTGAAGTCGTAA

>MK087656.1 Clostridium perfringens strain A 16S ribosomal RNA gene, partial sequence:

ATGCAAGTCGTAACAAGGTAGCCGTTATAGGGAACGGATTAGCGG
CGGACGGGTGAGTAACACGTGGGTA

ACCTGCCTCATAGAGTGGAATAGCCTTCCGAAAGGAAGATTAATA
CCGCATAATGTTGAAAGATGGCATC

ATCATTCAACCAAAGGAGCAATCCGCTATGAGATGGACCCGCGGC
GCATTAGCTAGTTGGTGGGGTAACG

GCCTACCAAGGCGACGATGCGTAGCCGACCTGAGAGGGTGATCGG
CCACATTGGGACTGAGACACGGCCC

AGACTCCTACGGGAGGCAGCAGTGGGGAATATTGCACAATGGGGG
AAACCCTGATGCAGCAACGCCGCGT

GAGTGATGAAGGTTTTCGGATCGTAAAGCTCTGTCTTTGGGGAAGA
TAATGACGGTACCCAAGGAGGAAG

CCACGGCTAACTACGTGCCAGCAGCCGCGGTAATACGTAGGTGGC
GAGCGTTATCCGGATTTACTGGGCG

TAAAGGGAGCGTAGGCGGATGATKWRGKGGRATGTGAAATAGCC
CGGKCTCAACTTGGGTGCTGCATTAC
CAAACTSGTTATCTAGAGTGCAGGAGAGGAGAGTRGAATTCSTAGT
GTAGCGGTGAAATGCGTAKAGATT
AKGAAGAACACCAGTGGCGAAGGCGACTCTCTGGACTGTAACTGA
CGCTGAGGCTCGAAAGCGTGGGGAG
CAAACAGGATTAGATACCCTGGTAGTCCACGCCGTAWACGATGAA
TACTAGGTGTGGGGGGTTTCAACAC
CTCCGTGCCGCCGCTAACGCATTWAGTATTCCGTCCTGGRGAAGTA
CGGTCGCARRATTAMAACTCRAAG
GAATTTGACGGGGASCTCGCAACAAAGTTAGCGGAGCATGTSGTTT
AATTCGAAGCAACGCGAAGAACCT
TACCTACACTTGACATCCCTTGCATTACTCTTAATCGAGGAAATCC
CTTCGGGGACAAGGTGACAGGTGG
TGCATGGTTGTCGTCAGCTCGTGTCGTGAGATGTTGGGTTAAGTCC
CGCAACGAGCGCAACCCTTGTCGT
TAGTTACTACCATTAAGTTGAGGACTCTAGCGAGACTGCCTGGGTT
AACCAGGAGGAAGGTGGGGATGAC
GTCAAATCATCATGCCCCTTATGTGTAGGGCTACACACGTGCTACA
ATGGCTGGTACAGAGAGATGCAAT
ACCGCGAGGTGGAGCCTAACTTAAAAACCAGTCTCAGTTCGGATT
GTAGGCTGAAACTCGCCTACATGAA
GCTGGAGTTACTAGTAATCGCGAATCAGAATGTCGCGGTGAATAC
GTTCCCGGGTCTTGTACACACCGCC
CGTCACACCATGAGAGTTGGCAATACCCGAAGTTCGTAGAGTTTG
ATCCCTGGCTCAGATAACG

>NR_036800.1 [Ruminococcus] gnavus ATCC 29149 16S ribosomal RNA, partial sequence:

TGAACGCTGGCGGCGTGCTTAACACATGCAAGTCGAGCGAAGCAC
CTTGACGGATTTCTTCGGATTGAAG

CCTTGGTGACTGAGCGGCGGACGGGTGAGTAACGCGTGGGTAACC
TGCCTCATACAGGGGGATAACAGTT

GGAAACGNCTGCTAATACCGCATAAGCGCACAGTACCGCATGGTA
CGGTGTGAAAAACTCCGGTGGTATG

AGATGGACCCGCGTCTGATTAGGTAGTTGGTGGGGTAACGGCCTA
CCAAGCCGACGATCAGTAGCCGACC

TGAGAGGGTGACCGGCCACATTGGGACTGAGACACGGCCCAAACT
CCTACGGGAGGCAGCAGTGGGGAAT

ATTGCACAATGGGGGAAACCCTGATGCAGCGACGCCGCGTGAGCG
ATGAAGTATTTCGGTATGTAAAGCT

CTATCAGCAGGGAAGAAAATGACGGTACCTGACTAAGAAGCCCCG
GCTAACTACGTGCCAGCAGCCGCGG

TAATACGTAGGGGGCAAGCGTTATCCGGATTTACTGGGTGTAAAG
GGAGCGTAGACGGCATGGCAAGCCA

GATGTGAAAGCCCGGGGCTCAACCCCGGGACTGCATTTGGAACTG
TCAGGCTAGAGTGTCGGAGAGGAAA

GCGGAATTCCTAGTGTAGCGGTGAAATGCGTAGATATTAGGAGGA
ACACCAGTGGCGAAGGCGGCTTTCT

GGACGATGACTGACGTTGAGGCTCGAAAGCGTGGGGAGCAAACAG
GATTAGATACCCTGGTAGTCCACGC

CGTAAACGATGAATACTAGGTGTCGGGTGGCAAAGCCATTCGGTG
CCGCAGCAAACGCAATAAGTATTCC

ACCTGGGGAGTACGTTCGCAAGAATGAAACTCAAAGGAATTGACG
GGGACCCGCACAAGCGGTGGAGCAT

GTGGTTTAATTCGAAGCAACGCGAAGAACCTTACCTGGTCTTGACA TCCCTCTGACCGCTCTTTAATCGG AGCTTTCCTTCGGGACAGAGGAGACAGGTGGTGCATGGTTGTCGTC AGCTCGTGTCGTGAGATGTTGGGT TAAGTCCCGCAACGAGCGCANCCCCTATCTTTAGTAGCCAGCATTT TGGATGGGCACTCTAGAGAGACTG CCAGGGATAACCTGGAGGAAGGTGGGGATGACGTCAAATCATCAT GCCCCTTATGACCAGGGCTACACAC GTGCTACAATGGCGTAAACAAAGGGAAGCGAGCCCGCGAGGGGG AGCAAATCCCAAAAATAACGTCTCAG TTCGGATTGTAGTCTGCAACTCGACTACATGAAGCTGGAATCGCTA GTAATCGCGAATCAGAATGTCGCG GTGAATACGTNCCCGGGTCTTGTACACACCGCCCGTCACACCATGG GAGTCAGTAACGCCCGAAGTCAGT GACCCAACCGCAAGGAGGGAGCTGCCGAAGGTGGGACCGATAACT GGGGTGAAGTCGTAACAAGGTAGCC GTATCGGAAGG

>NR_044049.1 Coprococcus eutactus strain ATCC 27759 16S ribosomal RNA, partial sequence:

GATGAACGCTGGCGGCGTGCTTAACACATGCAAGTCGAACGATTG AAGCTGGAGCTTGCTCCGGCCGATT TAGTGGCGGACGGGTGAGTAACGCGTGGGTAACCTGCCTCATACA GGGGGATAGCAGTTGGAAACGACTG GTAAAACCGCATAAGCGCACRGTATCGCATGATACAGTGTGAAAA CACTCCGGTGGTATGAGATGGACCC GCGTCTGATTAGCTAGTTGGTGAGGTAACGGCCCACCAAGGCGAC GATCAGTAGCCGGCCTGAGAGGGTG

AACGGCCACATTGGGACTGAGACACGGCCCAAACTCCTACGGGAG
GCAGCAGTGGGGAATATTGCACAAT

GGGGGAAACCCTGATGCAGCGACGCCGCGTGAGTGAAGAAGTATT
TCGGTATGTAAAGCTCTATCAGCAG

GGAAGATAATGACGGTACCTGACTAAGAAGCACCGGCTAAATACG
TGCCAGCAGCCGCGGTAATACGTAT

GGTGCAAGCGTTATCCGGATTTACTGGGTGTAAAGGGTGCGTAGGT
GGCAAGGCAAGTCTGAAGTGAAAA

TCCGGGGCTCAACCCCGGAACTGCTTTGGAAACTGTTTAGCTAGAG
TACAGGAGAGGTAAGTGGAATTCC

TAGTGTAGCGGTGAAATGCGTAGATATTAGGAGGAACACCAGTGG
CGAAGGCGACTTACTGGACTGCTAC

TGACACTGAGGCACGAAAGCGTGGGGAGCAAACAGGATTAGATAC
CCTGGTAGTCCACGCCGTAAACGAT

GAATACTAGGTGTTGGGTTCCAAAGGGACTCGGTGCCGTCGCAAA
CGCATTAAGTATTCCACCTGGGGAG

TACGTTCGCAAGAATGAAACTCAAAGGAATTGACGGGGACCCGCA
CAAGCGGTGGAGCATGTGGTTTAAT

TCGAAGCAACGCGAAAAACCTTACCAAGTCTTGACATCCTGATGA
CGGTTCCTTAACCGGAACTTCTCTT

CGGAGCATCAGAGACAGGTGGTGCATGGTTGTCGTCAGCTCGTGT
CGTGAGATGTTGGGTTAAGTCCCGC

AACGAGCGCAACCCCTATTTCCAGTAGCCAGCAGTAAGATGGGCA
CTCTGGAGAGACTGCCGGGGATAAC

CCGGAGGAAGGTGGGGATGACGTCAAATCATCATGCCCCTTATGA
TTTGGGCTACACACGTGCTACAATG

GCAGTTACAAAGAGAAGCRAACCTGTGAAGGCGAGCAAACCTCAA
AAAGGCTGTCTCAGTTCGGATTGTA

GTCTGCAACTCGACTACATGAAGCTGGAATCGCTAGTAATCGCAG
ATCAGCATGCTGCGGTGAATACGTT

CCCGGGTCTTGTACACACCGCCCGTCACACCATGGGAGTCGGTAAC
GCCCGAAGTCAGTGACCCAACCGC

AAGGAGGGAGCTGCCGAAGGCAGGATCGATAACTGGGGTG

>NR_074634.1 [Eubacterium] rectale ATCC 33656 16S ribosomal RNA, partial sequence:

AGAGTTTGATCCTGGCTCAGGATGAACGCTGGCGGCGTGCTTAAC
ACATGCAAGTCGAACGAAGCACTTT
ATTTGATTTCCTTCGGGACTGATTATTTTGTGACTGAGTGGCGGAC
GGGTGAGTAACGCGTGGGTAACCT
GCCTTGTACAGGGGGATAACAGTTGGAAACGGCTGCTAATACCGC
ATAAGCGCACGGCATCGCATGATGC
AGTGTGAAAACTCCGGTGGTATAAGATGGACCCGCGTTGGATTA
GCTAGTTGGTGAGGTAACGGCCCAC
CAAGGCGACGATCCATAGCCGACCTGAGAGGGTGACCGGCCACAT
TGGGACTGAGACACGGCCCAAACTC
CTACGGGAGGCAGCAGTGGGGAATATTGCACAATGGGCGAAAGCC
TGATGCAGCGACGCCGCGTGAGCGA
AGAAGTATTTCGGTATGTAAAGCTCTATCAGCAGGGAAGATAATG
ACGGTACCTGACTAAGAAGCACCGG
CTAAATACGTGCCAGCAGCCGCGGTAATACGTATGGTGCAAGCGT
TATCCGGATTTACTGGGTGTAAAGG
GAGCGCAGGCGGTGCGGCAAGTCTGATGTGAAAGCCCGGGGCTCA
ACCCCGGTACTGCATTGGAAACTGT
CGTACTAGAGTGTCGGAGGGGTAAGCGGAATTCCTAGTGTAGCGG
TGAAATGCGTAGATATTAGGAGGAA

CACCAGTGGCGAAGGCGGCTTACTGGACGATAACTGACGCTGAGG
CTCGAAAGCGTGGGGAGCAAACAGG
ATTAGATACCCTGGTAGTCCACGCCGTAAACGATGAATACTAGGT
GTTGGGAAGCATTGCTTCTCGGTGC
CGTCGCAAACGCAGTAAGTATTCCACCTGGGGAGTACGTTCGCAA
GAATGAAACTCAAAGGAATTGACGG
GGACCCGCACAAGCGGTGGAGCATGTGGTTTAATTCGAAGCAACG
CGAAGAACCTTACCAAGTCTTGACA
TCCTTCTGACCGGTACTTAACCGTACCTTCTCTTCGGAGCAGGAGT
GACAGGTGGTGCATGGTTGTCGTC
AGCTCGTGTCGTGAGATGTTGGGTTAAGTCCCGCAACGAGCGCAA
CCCTTATCTTTAGTAGCCAGCGGTT
CGGCCGGGCACTCTAGAGAGACTGCCAGGGATAACCTGGAGGAAG
GCGGGGATGACGTCAAATCATCATG
CCCCTTATGACTTGGGCTACACACGTGCTACAATGGCGTAAACAAA
GGGAAGCAAAGCTGTGAAGCCGAG
CAAATCTCAAAAATAACGTCTCAGTTCGGACTGTAGTCTGCAACCC
GACTACACGAAGCTGGAATCGCTA
GTAATCGCAGATCAGAATGCTGCGGTGAATACGTTCCCGGGTCTTG
TACACACCGCCCGTCACACCATGG
GAGTTGGGAATGCCCGAAGCCAGTGACCTAACCGAAAGGAAGGA
GCTGTCGAAGGCAGGCTCGATAACTG
GGGTGAAGTCGTAACAAGGTAGCCGTATCGGAAGGTGCGGCTGGA
TCACCT

>NR_104687.1 Blautia hansenii DSM 20583 strain JCM 14655 16S ribosomal RNA, partial sequence:

AGAGTTTGATCCTGGCTCAGGATGAACGCTGGCGGCGTGCTTAAC
ACATGCAAGTCGAGCGAAGCACTTA

TCATTGACTCTTCGGAAGATTTGATATTTGACTGAGCGGCGGACGG
GTGAGTAACGCGTGGGTAACCTGC

CTCATACAGGGGAATAACAGTTAGAAATGGCTGCTAATGCCGCAT
AAGCGCACAGGACCGCATGGTCTGG

TGTGAAAAACTGAGGTGGTATGAGATGGACCCGCGTCTGATTAGG
TAGTTGGTGGGGTAACGGCCTACCA

AGCCGACGATCAGTAGCCGGCCTGAGAGGGTGAACGGCCACATTG
GGACTGAGACACGGCCCAGACTCCT

ACGGGAGGCAGCAGTGGGGAATATTGCACAATGGGGGAAACCCTG
ATGCAGCGACGCCGCGTGAAGGAAG

AAGTATCTCGGTATGTAAACTTCTATCAGCAGGGAAGAAAATGAC
GGTACCTGACTAAGAAGCCCCGGCT

AACTACGTGCCAGCAGCCGCGGTAATACGTAGGGGGCAAGCGTTA
TCCGGATTTACTGGGTGTAAAGGGA

GCGTAGACGGAAGAGCAAGTCTGATGTGAAAGGCTGGGGCTTAAC
CCCAGGACTGCATTGGAAACTGTTT

TTCTAGAGTGCCGGAGAGGTAAGCGGAATTCCTAGTGTAGCGGTG
AAATGCGTAGATATTAGGAGGAACA

CCAGTGGCGAAGGCGGCTTACTGGACGGTAACTGACGTTGAGGCT
CGAAAGCGTGGGGAGCAAACAGGAT

TAGATACCCTGGTAGTCCACGCCGTAAACGATGAATACTAGGTGTC
GGGGTGCAAAGCAGTTCGGTGCCG

CAGCAAACGCAATAAGTATTCCACCTGGGGAGTACGTTCGCAAGA
ATGAAACTCAAAGGAATTGACGGGG

ACCCGCACAAGCGGTGGAGCATGTGGTTTAATTCGAAGCAACGCG
AAGAACCTTACCAAGTCTTGACATC

TGCCTGACCGTTCCTTAACCGGAGCTTTCCTTCGGGACAGGCAAGA

CAGGTGGTGCATGGTTGTCGTCAG

CTCGTGTCGTGAGATGTTGGGTTAAGTCCCGCAACGAGCGCAACCC

CTATCCTTAGTAGCCAGCAGTCCG

GCTGGGCACTCTAGGGAGACTGCCGGGGATAACCCGGAGGAAGGC

GGGGACGACGTCAAATCATCATGCC

CCTTATGATTTGGGCTACACACGTGCTACAATGGCGTAAACAAAG

GGAAGCGAAGCGGTGACGCTTAGCA

AATCTCAAAAATAACGTCCCAGTTCGGACTGCAGTCTGCAACTCGA

CTGCACGAAGCTGGAATCGCTAGT

AATCGCGAATCAGAATGTCGCGGTGAATACGTTCCCGGGTCTTGTA

CACACCGCCCGTCACACCATGGGA

GTCAGTAACGCCCGAAGTCAGTGACCCAACCTTATGGAGGGAGCT

GCCGAAGGCGGGACCGATAACTGGG

GTGAAGTCGTAACAAGGTAACC

>NR_109014.1 Blautia faecis strain M25 16S ribosomal RNA, partial sequence:

ATAACAGCCAGAAATGACTGCTAATACCGCATAAGCGCACAGAAC

CGCATGGTTCGGTGTGAAAAACTCC

GGTGGTATAAGATGGACCCGCGTTGGATTAGCTAGTTGGCAGGGC

AGCGGCCTACCAAGGCGACGATCCA

TAGCCGGCCTGAGAGGGTGAACGGCCACATTGGGACTGAGACACG

GCCCAGACTCCTACGGGAGGCAGCA

GTGGGGAATATTGCACAATGGGGGAAACCCTGATGCAGCGACGCC

GCGTGAAGGAAGAAGTATCTCGGTA

TGTAAACTTCTATCAGCAGGGAAGATAATGACGGTACCTGACTAA

GAAGCCCCGGCTAACTACGTGCCAG

CAGCCGCGGTAATACGTAGGGGGCAAGCGTTATCCGGATTTACTG GGTGTAAAGGGAGCGTAGACGGCGC

AGCAAGTCTGATGTGAAAGGCAGGGGCTTAACCCCTGGACTGCAT TGGAAACTGCTGTGCTTGAGTGCCG

GAGGGGTAAGCGGAATTCCTAGTGTAGCGGTGAAATGCGTAGATA TTAGGAGGAACACCAGTGGCGAAGG

CGGCTTACTGGACGGTAACTGACGTTGAGGCTCGAAAGCGTGGGG AGCAAACAGGATTAGATACCCTGGT

AGTCCACGCCGTAAACGATGAATACTAGGTGTCAGGGAGCACAGC TCTTTGGTGCCGCCGCAAACGCATT

AAGTATTCCACCTGGGGAGTACGTTCGCAAGAATGAAACTCAAAG GAATTGACGGGGACCCGCACAAGCG

GTGGAGCATGTGGTTTAATTCGAAGCAACGCGAAGAACCTTACCA AATCTTGACATCCCTCTGACCGGGA

CTTAACCGTCCCTTTCCTTCGGGACAGGGGAGACAGGTGGTGCATG GTTGTCGTCAGCTCGTGTCGTGAG

ATGTTGGGTTAAGTCCCGCAACGAGCGCAACCCCTATCCTTAGTAG CCAGCACGCARTGGTGGGCACTCT

GAGGAGACTGCCAGGGATAACCTGGAGGAAGGCGGGGATGACGT CAAATCATCATGCCCCTTATGATTTG

GGCTACACACGTGCTACAATGGCGTAAACAAAGGGAAGCGAACCC GCGAGGGTGGGCAAATCTCAAAAAT

AACGTCCCAGTTCGGACTGCAGTCTGCAACTCGACTGCACGAAGCT GGAATCGCTAGTAATCGCGGATCA

GAATGCCGCGGTGAATACGTTCCCGGGTCTTGTACACACCGCCCGT CACACCATGGGAGTCAGTAACGCC

CG

>NR_144607.1 Blautia caecimuris strain SJ18 16S ribosomal RNA, partial sequence:

ATTTTGACAGAGACTTCGGTTGAAGTCGTTATAATCCTAGTGGCGG

ACGGGTGAGTAACGCGTGGGTAAC

CTGCCTCACACTGGGGGATAACAGTCAGAAATGACTGCTAATACC

GCATAAGCGCACGGGATTGCATGAT

CCAGTGTGAAAAACTCCGGTGGTGTGAGATGGACCCGCGTTGGAT

TAGCCAGTTGGCAGGGTAACGGCCT

ACCAAAGCGACGATCCATAGCCGGCCTGAGAGGGTGGACGGCCAC

ATTGGGACTGAGACACGGCCCAGAC

TCCTACGGGAGGCAGCAGTGGGGAATATTGCACAATGGGGGAAAC

CCTGATGCAGCGACGCCGCGTGAAG

GAAGAAGTATCTCGGTATGTAAACTTCTATCAGCAGGGAAGAAAA

TGACGGTACCTGACTAAAAGCCCC

GGCTAACTACGTGCCAGCAGCCGCGGTAATACGTAGGGGGCAAGC

GTTATCCGGATTTACTGGGTGTAAA

GGGAGCGTAGACGGAGCAGCAAGTCTGATGTGAAAGGCGGGGGC

TCAACCCCCGGACTGCATTGGAAACT

GTTGATCTTGAGTACCGGAGAGGTAAGCGGAATTCCTAGTGTAGC

GGTGAAATGCGTAGATATTAGGAGG

AACACCAGTGGCGAAGGCGGCTTACTGGACGGTAACTGACGTTGA

GGCTCGAAAGCGTGGGGAGCAAACA

GGATTAGATACCCTGGTAGTCCACGCCGTAAACGATGAATACTAG

GTGTCGGGTGGCAGAGCCATTCGGT

GCCGCAGCAAACGCAGTAAGTATTCCACCTGGGGGAGTACGTTCG

CAAGAATGAAACTCAAAGGAATTGA

CGGGGACCCGCACAAGCGGTGGAGCATGTGGTTTAATTCGAAGCA

ACGCGAAGAACCTTACCAAGTCTTG

ACATCCCTATGACCGGCGCGTAACGTCGCCTTTTCTTCGGAGCATC
GGAGACAGGTGGTGCATGGTTGTC
GTCAGCTCGTGTCGTGAGATGTTGGGTTAAGTCCCGCAACGAGCGC
AACCCCTATCCTTAGTAGCCAGCG
GTTTGGCCGGGCACTCTGAGGAGACTGCCAGGGATAACCTGGAGG
AAGGCGGGGATGACGTCAAATCATC
ATGCCCCTTATGATTTGGGCTACACACGTGCTACAATGGCGTAAAC
AAAGGGAAGCGAGAGTGTGAGCTT
AAGCAAATCCCAAAAATAACGTCCCAGTTCGGACTGCAGTCTGCA
ACTCGACTGCACGAAGCTGGAATCG
CTAGTAATCGCGGATCAGAATGCCGCGGTGAATACGTTCCCGGGT
CTTGTACACACCGCCCGTCACACCA
TGGGAGTCAGTAACGCCCGAAGTCAGGACCGAACCGAAAGGACG
GAGC

>AJ867050.1 Bacteroides vulgatus 16S rRNA gene, strain ATCC 8482:

CAAACTAGGACCGAGTCCTACTTGCGATCGATGTCCGAATTGTGTA
CGTTCAGCTCCCCGTCGTACCAGA
ATCGAACGATTCCGGCTACCGCTGGCCGCGTGCCCACTCATTGTGC
ATAGGTTGGACGGCAGATGAGAAC
CTGTCGGAAGACTTTCCTTCTAATTATGTTCTACCGTAGTACTCAG
GCGTACAAGTGTACTAATTTCCAT
AAGGCCATCTGCTACCCCTACGCAAGGTAATCTATCATCCGCCCCA
TTGCCGGGTGGATCAGAAGCTACC
TATCCCAAGACTCTCCTTCAGGGGGTGTAACCTTGACTCTGTGCCA
GGTTTGAGGATGCCCTCCGTCGTC
ACTCCTTATAACCAGTTACCCGCTCTCGGACTTGGTCGGTTCATCG
CACTTCCTACTGACGGGATACCCA

ACATTTGAAGAAAATATTTCCTTATTTCAGCCCATACCTATGGGCA
AACGTACATGAAATACTTATTCCT

AGCCGATTGAGGCACGGTCGTCGGCGCCATTATGCCTCCTAGGCTC
GCAATAGGCCTAAATAACCCAAAT

TTCCCTCGCATCTACCTACAAATTCAGTCAACACTTTCAAACGCCG
AGTTGGCATTTTAACGTCAACTAT

GACCTATAGAACTCACGTCAACTCCGTCCGCCTTAAGCACCACATC
GCCACTTACGAATCTATAGTGCT

TCTTGAGGCTAACGCTTCCGTCGGACGATTCGACGTTGACTGTAAC
TCCGAGCTTTCACACCCATAGTTT

GTCCTAATCTATGGGACCATCAGGTGTGCCATTTGCTACTTATGAG
CGACAAACGCTATATGCCGTTCGC

CGGTTCGCTTTCGCAATTCATAAGGTGGACCCCTCATGCGGCCGTT
GCCACTTTGAGTTTCCTTAACTGC

CCCCGGGCGTGTTCGCCTCCTTGTACACCAAATTAAGCTACTATGC
GCTCCTTGGAATGGGCCCGAATTT

AACGTCTACTTAATGCCACTTTCGGCATTCGGCGTTCCGTAGACAC
TTCCACGACGTACCAACAGCAGTC

GAGCACGGCACTCCACAGCCGAATTCACGGTATTGCTCGCGTTGG
GAACAACAGTCAATGATTGTCCAAA

ACGACTCCTGAGACTGTTCTGACGGTAGCATTCTACACTCCTTCCA
CCCCTACTGCAGTTTAGTCGTGCC

GGGAATGCAGGCCCCGATGTGTGCACAATGTTACCCCCCATGTCTC
CCGGCGATGGTGCGCTCACTACGG

TTAGGGGTTTTGGAGAGAGTCAAGCCTGACCTCAGACGTTGGGCT
GAGGTGCTTCGACCTAAGCGATCAT

TAGCGCGTAGTCGGTGCCGCGCCACTTATGCAAGGGCCCGGAACA
TGTGTGGCGGGCAGTTCGGTACCT

CGGCCCCCCATGGACTTCACGCATTGGCGCTCCTCGCGGGATCCCA
TTTTGACCACTGACCCCGATTCAG
CATTGT

>NR_040865.1 Bacteroides ovatus strain JCM 5824 16S ribosomal RNA, partial sequence:

GATCATGGCTCAGGATGAACGCTAGCTACAGGCTTAACACATACA

AGTCGTGGGGCAGCATTTTATGTTT

GTTTGCAAACTGAAGATGGCGACCGGCGCACGGGTGAGTAACACG

TATCCAACCTGCCGATAACTCCGGA

ATAGCCTTTCGAAAGAAAGATTAATACCGGATAGCATACGATATC

GCATGATATTTTTATTAAAGAATTT

CGGTTATCGATGGGGATGCGTTCCATTAGTTTGTTGGCGGGGTAAC

GGCCCACCAAGACTACGATGGATA

GGGGTTCTGAGAGGAAGGTCCCCCACATTGGAACTGAGACACGGT

CCAAACTCCTACGGGAGGCAGCAGT

GAGGAATATTGGTCAATGGGCGAGAGCCTGAACCAGCCAAGTAGC

GTGAAGGATGAAGGCTCTATGGGTC

GTAAACTTCTTTTATATGGGAATAAAGTTTTCCACGTGTGGAATTTT

GTATGTACCATATGAATAAGGAT

CGGACTACTCCGTGCCAGCAGCCGCGGTAATACGGAGGATCCGAG

CGTTATCCGGATTTATTGGGTTTAA

AGGGAGCGTAGGTGGATTGTTAAGTCAGTTGTGAAAGTTTGCGGCT

CAACCGTAAAATTGCAGTTGAAAC

TGGCAGTCTTGAGTACAGTAGAGGTGGGCGGAATTCGTGGTGTAG

CGGTGAAATGCTTAGATATCACGAA

GAACTCCGATTGCGAAGGCAGCTCACTAGACTGTTACTGACACTG

ATGCTCGAAAGTGTGGGTATCAAAC

AGGATTAGATACCCTGGTAGTCCACACAGTAAACGATGAATACTC
GCTGTTTGCGATATACAGTAAGCGG

CCAAGCGAAAGCATTAAGTATTCCACCTGGGGAGTACGCCGGCAA
CGGTGAAACTCAAAGGAATTGACGG

GGGCCCGCACAAGCGGAGGAACATGTGGTTTAATTCGATGATACG
CGAGAACCTTACCCGGGCTTAAATT

GCAACAGAATATATTGGAAACAGTATAGCCGTAAGGCTGTTGTGA
AGGTGCTGCATGGTTGTCGTCAGCT

CGTGCCGTGAGGTGTCGGCTTAAGTGCCATAACGAGCGCAACCCTT
ATCTTTAGTTACTAACAGGTTATG

CTGAGGACTCTAGAGAGACTGCCGTCGTAAGATGTGAGGAAGGTG
GGGATGACGTCAAATCAGCACGGCC

CTTACGTCCGGGGCTACACACGTGTTACAATGGGGGGTACAGAAG
GCAGCTACCTGGTGACATGATGCTA

ATCCCAAAAACCTCTCTCAGTTCGGATCGAAGTCTGCAACCCGACT
TCGTGAAGCTGGATTCGCTAGTAA

TCGCGCATCAGCCATGGCGCGGTGAATACGTTCCCGGGCCTTGTAC
ACACCGCCCGTCAAGCCATGAAAG

CCGGGGGTACCTGAAGTACGTAACCGCAAGGAGCGTCCTAGGGTA
AAACTGGTAA

>NR_074784.2 Bacteroides fragilis NCTC 9343 16S ribosomal RNA, complete sequence:

ACAATGAAGAGTTTGATCCTGGCTCAGGATGAACGCTAGCTACAG
GCTTAACACATGCAAGTCGAGGGGC

ATCAGGAAGAAAGCTTGCTTTCTTTGCTGGCGACCGGCGCACGGGT
GAGTAACACGTATCCAACCTGCCC

TTTACTCGGGGATAGCCTTTCGAAAGAAAGATTAATACCCGATAGC
ATAATGATTCCGCATGGTTTCATT

ATTAAAGGATTCCGGTAAAGGATGGGGATGCGTTCCATTAGGTTGT
TGGTGAGGTAACGGCTCACCAAGC

CTTCGATGGATAGGGGTTCTGAGAGGAAGGTCCCCCACATTGGAA
CTGAGACACGGTCCAAACTCCTACG

GGAGGCAGCAGTGAGGAATATTGGTCAATGGGCGCTAGCCTGAAC
CAGCCAAGTAGCGTGAAGGATGAAG

GCTCTATGGGTCGTAAACTTCTTTTATATAAGAATAAAGTGCAGTA
TGTATACTGTTTTGTATGTATTAT

ATGAATAAGGATCGGCTAACTCCGTGCCAGCAGCCGCGGTAATAC
GGAGGATCCGAGCGTTATCCGGATT

TATTGGGTTTAAAGGGAGCGTAGGTGGACTGGTAAGTCAGTTGTG
AAAGTTTGCGGCTCAACCGTAAAAT

TGCAGTTGATACTGTCAGTCTTGAGTACAGTAGAGGTGGGCGGAAT
TCGTGGTGTAGCGGTGAAATGCTT

AGATATCACGAAGAACTCCGATTGCGAAGGCAGCTCACTGGACTG
CAACTGACACTGATGCTCGAAAGTG

TGGGTATCAAACAGGATTAGATACCCTGGTAGTCCACACAGTAAA
CGATGAATACTCGCTGTTTGCGATA

TACAGTAAGCGGCCAAGCGAAAGCATTAAGTATTCCACCTGGGGA
GTACGCCGGCAACGGTGAAACTCAA

AGGAATTGACGGGGGCCCGCACAAGCGGAGGAACATGTGGTTTAA
TTCGATGATACGCGAGGAACCTTAC

CCGGGCTTAAATTGCAGTGGAATGATGTGGAAACATGTCAGTGAG
CAATCACCGCTGTGAAGGTGCTGCA

TGGTTGTCGTCAGCTCGTGCCGTGAGGTGTCGGCTTAAGTGCCATA
ACGAGCGCAACCCTTATCTTTAGT

TACTAACAGGTTATGCTGAGGACTCTAGAGAGACTGCCGTCGTAA
GATGTGAGGAAGGTGGGGATGACGT

CAAATCAGCACGGCCCTTACGTCCGGGGCTACACACGTGTTACAAT
GGGGGGTACAGAAGGCAGCTAGCG

GGTGACCGTATGCTAATCCCAAAATCCTCTCTCAGTTCGGATCGAA
GTCTGCAACCCGACTTCGTGAAGC

TGGATTCGCTAGTAATCGCGCATCAGCCACGGCGCGGTGAATACG
TTCCCGGGCCTTGTACACACCGCCC

GTCAAGCCATGGGAGCCGGGGGTACCTGAAGTACGTAACCGCAAG
GATCGTCCTAGGGTAAAACTGGTGA

CTGGGGCTAAGTCGTAACAAGGTAGCCGTACCGGAAGGTGCGGCT
GGAACACCTCCTTT

>NR_027573.1 Intestinibacter bartlettii strain WAL 16138 16S ribosomal RNA, partial sequence:

AGGATGAACGCTGGCGGCGTGCCTAACACATGCAAGTCGAGCGAT
TNTCTTCGGAGAAGAGCGGCGGACG

GGTGAGTAACGCGTGGGTAACCTGCCCTGTACACACGGATAACAT
ACCGAAAGGTATGCTAATACGGGAT

AACATAAGAAATTCGCATGTTTTTCTTATCAAAGCTCCGGCGGTAC
AGGATGGACCCGCGTCTGATTAGC

TAGTTGGTGAGGTAACGGCTCACCAAGGCGACGATCAGTAGCCGA
CCTGAGAGGGTGATCGGCCACATTG

GAACTGAGACACGGTCCAAACTCCTACGGGAGGCAGCAGTGGGGA
ATATTGCACAATGGGCGAAAGCCTG

ATGCAGCAACGCCGCGTGAGCGATGAAGGCCTTCGGGTCGTAAAG
CTCTGTCCTCAAGGAAGATAATGAC

GGTACTTGAGGAGGAAGCCCCGGCTAACTACGTGCCAGCAGCCGC
GGTAATACGTAGGGGGCTAGCGTTA

TCCGGATTTACTGGGCGTAAAGGGTGCGTAGGCGGTCTTTTAAGTC
AGGAGTGAAAGGCTACGGCTCAAC

CGTAGTAAGCTCTTGAAACTGGAGGACTTGAGTGCAGGAGAGGAG
AGTGGAATTCCTAGTGTAGCGGTGA

AATGCGTAGATATTAGGAGGAACACCAGTAGCGAAGGCGGCTCTC
TGGACTGTAACTGACGCTGAGGCAC

GAAAGCGTGGGGAGCAAACAGGATTAGATACCCTGGTAGTCCACG
CCGTAAACGATGAGTACTAGCTGTC

GGAGGTTACCCCCTTCGGTGGCGCAGCTAACGCATTAAGTACTCCG
CCTGGGGAGTACGCTCGCAAGAGT

GAAACTCAAAGGAATTGACGGGGACCCGCACAAGTAGCGGAGCAT
GTGGTTTAATTCGAAGCAACGCGAA

GAACCTTACCTAAGCTTGACATCCTTTTGACCGATGCCTAATCGCA
TCTTTCCCTTCGGGGACAGAAGTG

ACAGGTGGTGCATGGTTGTCGTCAGCTCGTGTCGTGAGATGTTGGG
TTAAGTCCCGCAACGAGCGCAACC

CTTGCCTTTAGTTGCCATCATTAAGTTGGGCACTCTAGAGGGACTG
CCAGGGATAACCTGGAGGAAGGTG

GGGATGACGTCAAATCATCATGCCCCTTATGCTTAGGGCTACACAC
GTGCTACAATGGGTGGTACAGAGG

GCAGCGAAGTCGTGAGGCCAAGCTAATCCCTTAAAGCCATTCTCA
GTTCGGATTGTAGGCTGAAACTCGC

CTACATGAAGCTGGAGTTACTAGTAATCGCAGATCAGAATGCTGC
GGTGAATGCGTTCCCGGGTCTTGTA

CACACCGCCCGTCACACCATGGGAGTTGGGGGCGCCCGAAGCCGG
CTAGCTAACCTTTTGGAAGCGGTCG

TCGAAGGTGAAACCAATAACTGGGGTGAA

>NR_176800.1 Bilophila wadsworthia strain CCUG 32349 16S ribosomal RNA, partial sequence:

TGCTTAACACATGCAAGTCGAACGTGAAAGTCCTTCGGGGCGAGT
AAAGTGGCGCACGGGTGAGTAACGC
GTGGATAATCTACCCTTAAGATGGGGATAACGGCTGGAAACGGTC
GCTAATACCGAATACGCTCCCGATT
TTATCATTGGGGGGAAAGATGGCCTCTGCTTGCAAGCTATCGCTTA
AGGATGAGTCCGCGTCCCATTAGC
TAGTTGGCGGGGTAACGGCCCACCAAGGCAACGATGGGTAGCCGG
TCTGAGAGGATGACCGGCCACACTG
GAACTGGAACACGGTCCAGACTCCTACGGGAGGCAGCAGTGGGGA
ATATTGCGCAATGGGCGAAAGCCTG
ACGCAGCGACGCCGCGTGAGGGATGAAGGTTCTCGGATCGTAAAC
CTCTGTCAGGGGGGAAGAAACCCCC
TCGTGTGAATAATGCGAGGGCTTGACGGTACCCCCAAAGGAAGCA
CCGGCTAACTCCGTGCCAGCAGCCG
CGGTAATACGGAGGGTGCAAGCGTTAATCGGAATCACTGGGCGTA
AAGCGCACGTAGGCGGCTTGGTAAG
TCAGGGGTGAAATCCCACAGCCCAACTGTGGAACTGCCTTTGATAC
TGCCAGGCTTGAGTACCGGAGAGG
GTGGCGGAATTCCAGGTGTAGGAGTGAAATCCGTAGATATCTGGA
GGAACACCGGTGGCGAAGGCGGCCA
CCTGGACGGTAACTGACGCTGAGGTGCGAAAGCGTGGGTAGCAAA
CAGGATTAGATACCCTGGTAGTCCA
CGCTGTAAACGATGGGTGCTGGGTGCTGGGATGTATGTCTCGGTGC
CGTAGCTAACGCGATAAGCACCCC

GCCTGGGGAGTACGGTCGCAAGGCTGAAACTCAAAGAAATTGACG
GGGGCCCGCACAAGCGGTGGAGTAT

GTGGTTTAATTCGATGCAACGCGAAGAACCTTACCCAGGCTTGACA
TCTAGGGAACCCTTCGGAAATGAA

GGGGTGCCCTTCGGGGAGCCCTAAGACAGGTGCTGCATGGCTGTC
GTCAGCTCGTGCCGTGAGGTGTTGG

GTTAAGTCCCGCAACGAGCGCAACCCCTATCTTCAGTTGCCAGCAG
GTAAGGCTGGGCACTCTGGAGAGA

CCGCCCCGGTCAACGGGGAGGAAGGTGGGGACGACGTCAAGTCAT
CATGGCCCTTACGCCTGGGGCTACA

CACGTACTACAATGGCGCGCACAAAGGGTAGCGAGACCGCGAGGT
GGAGCCAATCCCAAAAAACGCGTCC

CAGTCCGGATTGGAGTCTGCAACTCGACTCCATGAAGTCGGAATC
GCTAGTAATTCGAGATCAGCATGCT

CGGGTGAATGCGTTCCCGGGCCTTGTACACACCGCCCGTCACACCA
CGAAAGTCGGTTTTACCCGAAGCC

GGTGAGCTAACTCGCAAGAGGAGCAGCCGTCTACGGTAGGGCCGA
TGATTGGGG

The quantitative abundance is determined from the extracted DNA, using a set of probes specific to each of the plurality of predetermined microbes associated with the biological sample. The set of probes includes a plurality of probes where each probe is utilized for each of the plurality of predetermined microbes (one probe for one predetermined microbe) associated with the biological sample.

In an embodiment, a multiplexed quantitative Polymerase Chain Reaction (qPCR) technique is employed for determining the quantitative abundance. More specifically, the multiplexed quantitative Polymerase Chain Reaction (qPCR) technique define an experimental design and arrangement of the plurality of probes that are of the set of probes for detecting and determining the quantitative abundance associated with the biological sample.

More specifically, the set of probes specific to each of the plurality of predetermined microbes associated with the biological sample are utilized in three sequential multiplexed qPCR runs (defined by the multiplexed quantitative Polymerase Chain Reaction (qPCR) technique), to determine the quantitative abundance of each of the plurality of predetermined microbes associated with the biological sample.

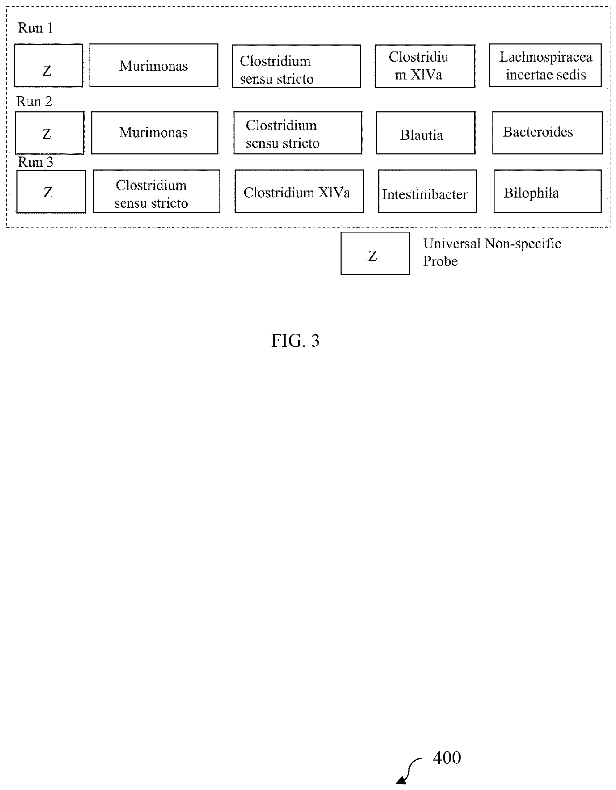

FIG. 3 illustrates an exemplary probe and multiplexed qPCR design for detecting and determining a quantitative abundance of each of the plurality of predetermined microbes associated with the biological sample, according to some embodiments of the present disclosure. As shown in FIG. 3, the three sequential multiplexed qPCR runs namely a first multiplexed qPCR run (Run 1), a second multiplexed qPCR run (Run 2), and a third multiplexed qPCR run (Run 3) are defined for determining quantitative abundance of each of the plurality of predetermined microbes associated with the biological sample. Each Run of the first multiplexed qPCR run (Run 1), the second multiplexed qPCR run (Run 2), and the third multiplexed qPCR run (Run 3) includes five probes (hence it is also called as five-plex qPCR run) where each probe is utilized for the plurality of predetermined microbes associated with the biological sample, from the list having: *Murimonas, Clostridium sensu stricto, Clostridium XIVa, Lachnospiracea incertae sedis, Blautia, Bacteroides, Intestinibacter, and Bilophila.* Also, each run of the first multiplexed qPCR run (Run 1), the second multiplexed qPCR run (Run 2), and the third multiplexed qPCR run (Run 3) contains a non-specific probe or a universal probe (denoted as 'Z' in FIG. 3).

Further, as shown in FIG. 3, the plurality of predetermined microbes, the quantitative abundance of which are being determined through the first multiplex qPCR run are: *Murimonas, Clostridium sensu stricto, Clostridium XIVa,* and *Lachnospiracea incertae sedis.* The plurality of predetermined microbes, the quantitative abundance of which are being determined through the second multiplex qPCR run are: *Murimonas, Clostridium sensu stricto, Blautia,* and *Bacteroides.*

The plurality of predetermined microbes, the quantitative abundance of which are being determined through the third multiplex qPCR run are: *Clostridium sensu stricto, Clostridium XIVa, Intestinibacter,* and *Bilophila.*

In an embodiment, the plurality of predetermined microbes associated with the biological samples are captured from features of a pre-determined machine learning (ML) model. In an embodiment, the pre-determined machine learning (ML) model is an ensemble machine learning (ML) model that is built using a microbial abundance data corresponding to a plurality of training biological samples. The plurality of training biological samples are the biological samples used for training a machine learning model to obtain the corresponding pre-determined machine learning (ML) model. In an embodiment, the microbial abundance data corresponding to the plurality of training biological samples is the quantitative abundance of all the microbes present in each of the plurality of training biological samples.

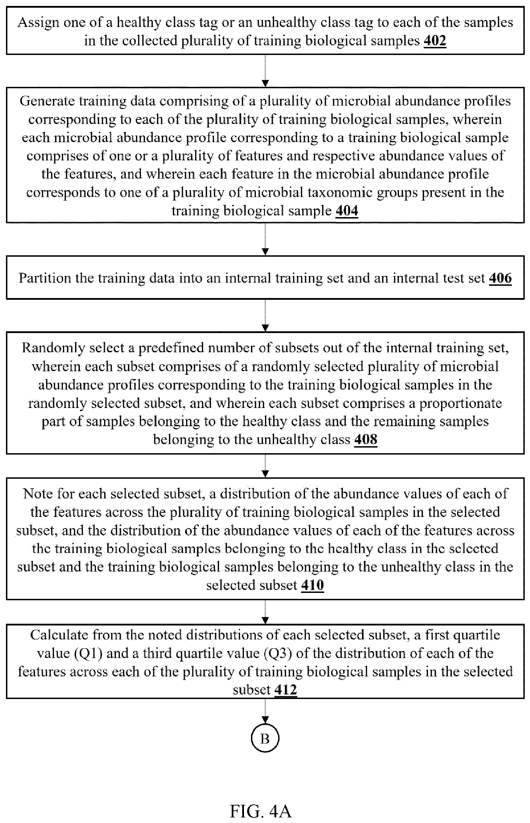

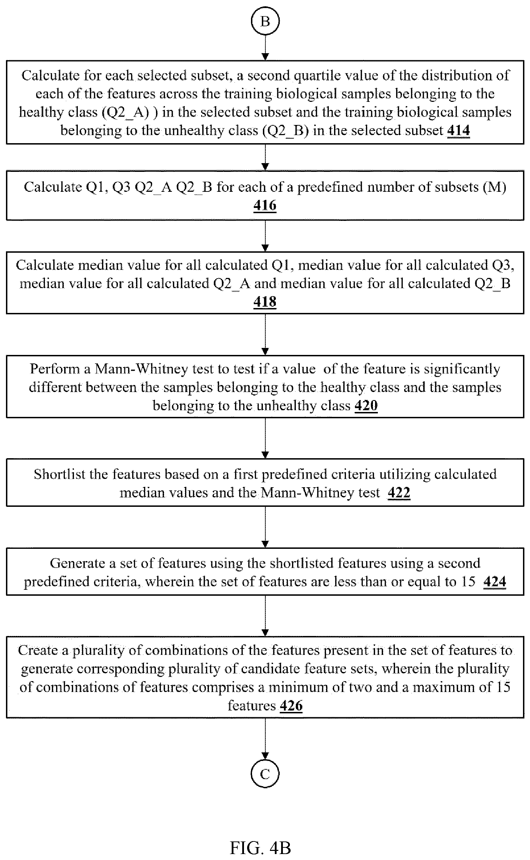

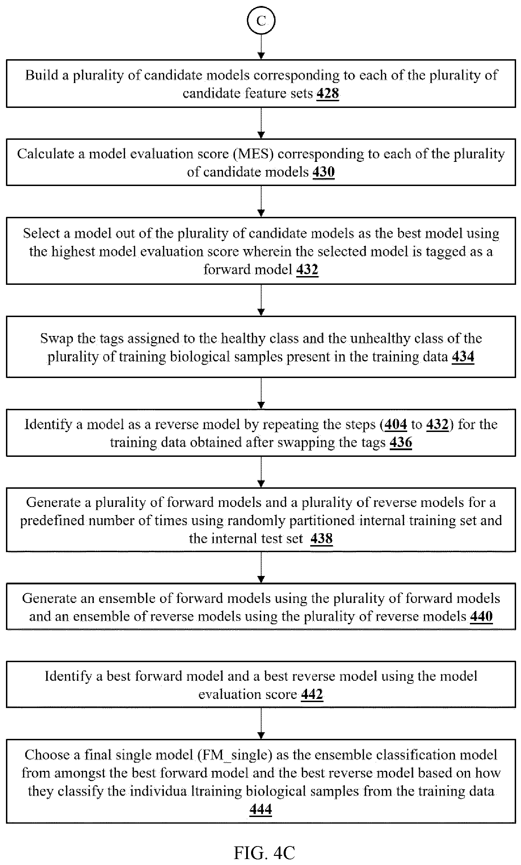

The ensemble ML model is built using the plurality of training biological samples, to obtain the pre-determined machine learning model. FIGS. 4A, 4B and 4C are flowcharts illustrating steps 400 involved in building the pre-determined machine learning model, according to some embodiments of the present disclosure. The technique for building the ensemble ML model accepts data in form of a feature table for multiple observations (the plurality of training biological samples) wherein each observation/ training biological sample is defined by '$N$' features ($F$) which are either or both of continuous and counted variables with ($N \geq 1$). In case of training data ($TR$), each of the training biological samples/observations further have a preassigned class/category which is binary in nature, i.e., a healthy class (A) (e.g., affiliating to healthy samples category obtained from subjects/ individuals detected/ clinically assessed as having no risk of breast cancer including information about whether these subjects/ individuals have MBD category as one of a low, a medium and a high category) and an unhealthy class or diseased class (B) (e.g., affiliating to unhealthy or disordered or diseased samples category obtained from subjects/ individuals having the risk of breast cancer including information about whether these subjects/ individuals have MBD category as one of a low, a medium and a high category). In case of test data ($TS$) or data received during actual deployment of the method, the model(s) built based on training data predicts the class/category of the training biological samples/observations. During training process, the following steps are followed:

Initially at step 402, a healthy class tag or an unhealthy class tag is assigned to each of the training biological samples in the collected plurality of training biological samples. The healthy class tag indicates absence of risk of breast cancer, and the unhealthy class tag indicates presence of risk of breast cancer.

At step 404, the training data comprises of a plurality of microbial abundance profiles corresponding to each of the collected plurality of training biological samples, wherein each microbial abundance profile corresponding to a training biological sample comprises of one or a plurality of feature (s) and respective abundance value (s) of the feature (s), wherein each feature in the microbial abundance profile corresponds to one of a plurality of microbial taxonomic groups present in the plurality of training biological samples.

In the next step 406, the training data ($TR$) is randomly partitioned into two sets - namely, an internal-train ($ITR$) and an internal-test ($ITS$), based on a parameter '$L_1$', wherein $L_1$% training biological samples from the total training data constitute the $ITR$ set and (100 - $L_1$) % of the training biological samples constitute the $ITS$ set. Furthermore, the random partitioning into $ITR$ and $ITS$ sets is performed using a stratified sampling approach with the intent of preserving the relative proportion of training biological samples belonging to the healthy class (A) or the unhealthy class (B) in the total training data in these newly drawn subsets.

In the next step 408, a predefined number of subsets are randomly selected out of the internal training set based on a second parameter ($L_2$). Each of the subset comprises a randomly selected plurality of microbial abundance profiles corresponding to the plurality of training biological samples in the randomly selected subset, and wherein each of the subset comprises a proportionate part of training biological samples belonging to the healthy class (A) and the remaining training biological samples belonging to the unhealthy class (B). Thus, from $ITR$, '$M$' randomly drawn subsets $ITRS_i$ (e.g., $ITRS_1, ITRS_2, ITRS_3 .... ITRS_M$), each containing $S$ training biological samples are further generated, wherein $S = L_2$% of the training biological samples present in $ITR$. For example, the values of $L_2$ and $M$ are 80 % and 100 respectively for present disclosure. Other values are within the scope of this invention.

In the next step 410, for each selected subset, a distribution of the abundance values of each of the features across the plurality of training biological samples in the selected subset, and the distribution of the abundance values of each of the features across the training biological samples belonging to the healthy class (A) in the selected subset and the training biological samples belonging to the unhealthy class (B) in the selected subset are noted. Thus, from each subset $ITRS_i$ (where $i$ = 1, 2, 3, ..., $M$), wherein there are total $S$ training biological samples, each of which are described by $N$ features ($F_j$) (where $j$ = 1, 2, 3, ..., $N$), the distributions of each of the features ($ITRS_iDF_j$) across $S$ training biological samples are noted. Similarly, from each subset $ITRS_i$, wherein there are $S_A$ training biological samples belonging to the healthy class (A) and $S_B$ training biological samples belonging to the unhealthy class (B), each of the training biological samples being described by $N$ features ($F_j$; $j$ = 1, 2, 3, ..., $N$), the distributions of each of the features ($ITRS_iD_AF_j$) across $S_A$ training biological samples, and the distributions of each of the features ($ITRS_iD_BF_j$) across $S_B$ training biological samples are noted.

In the next step 412, from the noted distributions of each selected subset, a first quartile value ($Q1$) and a third quartile value ($Q3$) of the distribution of each of the features is calculated across each of the plurality of training biological samples in the selected subset. In an example, the respective first quartile value ($Q1$) and the third quartile value ($Q3$) of $ITRS_iDF_j$ may also be referred as $Q1ITRS_iDF_j$ and $Q3ITRS_iDF_j$.

Furthermore, in the next step 414, for each selected subset, a second quartile value of the distribution of each of the features across the training biological samples belonging to the healthy class ($Q2_A$) in the selected subset and the training biological samples belonging to the unhealthy class ($Q2_B$) in the selected subset is calculated. Thus, in an example, the median value (in other words, the second quartile value) of ($ITRS_iD_AF_j$) is referred as $Q2ITRS_iD_AF_j$, and the median value of ($ITRS_iD_BF_j$) is referred as $Q2ITRS_iD_BF_j$.

In the next step 416, for the M subsets of $ITRS_i$, a total of M values for each of $Q1ITRS_iDF_j$, $Q3ITRS_iDF_j$, $Q2ITRS_iD_AF_j$, and $Q2ITRS_iD_BF_j$, are calculated. Further at step 418, median value ($\widetilde{Q1}_J$) is calculated for all calculated $Q1$, median value ($\widetilde{Q3}_J$) is calculated for all calculated Q3, median value ($\widetilde{Q2_A}_J$) is calculated for all calculated Q2$_A$ and median value ($\widetilde{Q2_B}_J$) is calculated for all calculated Q2$_B$. Thus,

$$\widetilde{Q1}_J = \text{median of } \{Q1ITRS_1DF_j, Q1ITRS_2DF_j, Q1ITRS_3DF_j, \ldots Q1ITRS_MDF_j\}$$

$$\widetilde{Q3}_J = \text{median of } \{Q3ITRS_1DF_j, Q3ITRS_2DF_j, Q3ITRS_3DF_j, \ldots Q3ITRS_MDF_j\}$$

$$\widetilde{Q2_A}_J = \text{median } \{Q2ITRS_1D_AF_j, Q2ITRS_2D_AF_j, Q2ITRS_3D_AF_j, \ldots . Q2ITRS_MD_AF_j\}$$

$$\widetilde{Q2_B}_J = \text{median } \{Q2ITRS_1D_BF_j, Q2ITRS_2D_BF_j, Q2ITRS_3D_BF_j, \ldots . Q2ITRS_MD_BF_j\} \quad \text{(where } i = 1,2,3, \ldots,$$
$M$; and $j$ = 1, 2, 3, ..., $N$)

In the next step 420, a Mann-Whitney test is performed to test if a value of the feature ($F_j$) is significantly (p<0.1) different between the training biological samples belonging to the healthy class ($S_A$) and the training biological samples belonging to the unhealthy class ($S_B$) in each of the M randomly drawn subsets $ITRS_j$. Other statistical tests based on the nature of distribution (e.g., t-test for normal distribution), nature of sampling (e.g., Wilcoxon signed rank test for paired case and control samples) or other methods of statistical comparison relevant for microbiome datasets (e.g., ALDEx2) can also be adopted.

In the next step 422, the features are shortlisted based on a first predefined criteria utilizing calculated median values and the Mann-Whitney test. The first predefined criteria comprises if a feature $F_j$ is observed to have significantly (p<0.1) different values in $S_A$ compared to $S_B$ in more than 70% of $M$ subsets, and if $\widetilde{Q2_A}_J >= Q2_{min}$ OR $\widetilde{Q2_B}_J >= Q2_{min}$ (a predefined feature 'abundance' threshold and Q2$_{min}$ threshold as described in the case study). $F_j$ is added to a set of shortlisted features ($SF$).

In the next step 424, a set of features is generated using the shortlisted features ($SF$) using a second predefined criteria, wherein the set of features are less than or equal to 15. If the number of shortlisted features ($SF$) obtained in previous step satisfies the criteria $1 \le SF \le 15$, then the training process proceeds to model building with all the features in $SF$. If no shortlisted features ($SF$) are obtained in previous (i.e., $SF < 1$) then following step is performed with all the features $F_j$ for evaluating the ability of the features, when considered independently, to distinguish between training biological samples belonging to the healthy class (A) and the unhealthy class (B). Similarly, if the number of shortlisted features ($SF$) obtained in previous step exceeds fifteen ($SF > 15$) then following step is performed with all the shortlisted features ($SF$) for evaluating the ability of the features, when considered independently, to distinguish between the training biological samples belonging to the healthy class (A) and the unhealthy class (B).

Steps for shortlisting the features in case of $SF < 1$ or $SF > 15$: For each of the features (obtained previously) taken individually, different threshold values are used to classify the samples belonging to the set $ITR$, and the results are cumulated to construct a receiver operating characteristic curve (ROC curve) for each of the features. The area under the curve (AUC) of the ROC curve of any feature (AUC$^F$) is indicative of the utility of the feature to distinguish between the training biological samples belonging to the healthy class (A) and the unhealthy class (B), and the same is computed for every feature. The shortlisted features ($SF$) set is modified to include only the top fifteen features from a list of features arranged in a descending order of the AUC$^F$ values.

In the next step 426, a plurality of combinations of the features present in the set of features is created to generate corresponding plurality of candidate feature sets ($CF$), wherein the plurality of combinations of features comprises a minimum of one and a maximum of 15 features. In an embodiment, the maximum possible candidate feature sets that can be created in this process is K = $2^{15}$ - 1 = 32767 (i.e., maximum value of K = 32767).

In the next step 428, a plurality of candidate models is built corresponding to each of the plurality of candidate feature sets. At step 430, a model evaluation score (*MES*) is calculated corresponding to each of the plurality of candidate models. For each candidate feature set $CF_K$, a corresponding candidate model $CM_K$ is built and evaluated as mentioned in the steps mentioned below.

Steps for evaluating the candidate model:

- Step 1: The values of the features $F_j$ constituting a candidate feature set defining the training biological samples in *ITR* are transformed to $F_j'$ such that - $-\widetilde{Q1}_j$, $\widetilde{Q3}_j$, $\widetilde{Q2_{A_j}}$ and $\widetilde{Q2_{B_j}}$

$$F_j' = 0 \ldots\ldots\ldots\ldots\ldots \text{ if } F_j < \widetilde{Q1}_j$$

$$F_j' = 1 \ldots\ldots\ldots\ldots\ldots \text{ if } F_j > \widetilde{Q3}_j$$

$$F_j' = 0.5 \ldots\ldots\ldots\ldots\ldots \text{ if } \widetilde{Q1}_j = \widetilde{Q3}_j$$

$$F_j' = \frac{F_j - \widetilde{Q1}_j}{\widetilde{Q3}_j - \widetilde{Q1}_j} \ldots\ldots\ldots\ldots\ldots \text{ if } \widetilde{Q1}_j < F_j < \widetilde{Q3}_j$$

- Step 2: If for a feature $F_j$, it is observed that $\widetilde{Q2_{B_J}} > \widetilde{Q2_{A_J}}$, then the feature $F_j$ is tagged as a 'numerator' feature and added to a set of numerator features $F_{numerator}$. Else, feature $F_j$ is tagged as a 'denominator' feature and added to a set of denominator features $F_{denominator}$.
- Step 3: Each candidate model ($CM_K$) is constituted as a simple ratio function given below -

$$CM_K = \frac{\sum F_{numerator}}{\sum F_{denominator}} \ldots \text{ when } F_{numerator} > 0 \text{ and } F_{denominator} > 0$$

or,

$$CM_K = \frac{\sum F_{numerator} + 1}{\sum F_{denominator} + 1} \ldots \text{ when either } F_{numerator} \text{ or } F_{denominator} = 0$$

wherein, $\sum F_{numerator}$ represents the sum of values of all numerator features for a particular sample, and,
wherein, $\sum F_{denominator}$ represents the sum of values of all denominator features for a particular sample.

For each of the features, a transformed value $F_j'$ as obtained above is used in the candidate model equation.
- Step 4: A candidate model c is used to generate candidate model scores ($CMS_K$) for each of the samples in the set *ITR*. From the set of scores $CMS_K$, the top 10 percentile and bottom 10 percentile scores are removed as outliers and thereafter the maximum and minimum scores from the set $CMS_K$ are noted as $CMS_{K_{max}}$ and $CMS_{K_{min}}$ respectively.
- Step 5: Considering each of the scores in the set $CMS_K$ as a threshold ($T$), the model $CM_K$ is used to (re)classify the samples in the training set (*ITR.*) such that -

  ○ The training biological sample is classified into the healthy class (A) if $CMS >= T$
  ○ or the training biological sample is classified into the unhealthy class (B) if $CMS < T$

and based on a comparison of these classifications and the true/original classes of the training biological samples, Matthew's correlation coefficients (*MCC*) for each of the thresholds are calculated, to evaluate how well each of the thresholds can distinguish between training biological samples between the healthy class (A) and the unhealthy class (B).
- Step 6: The threshold ($T_{max}$) which provides the maximum absolute *MCC* value ($|MCC_{max}|$) is noted. If $|MCC_{max}| < 0.4$ for a candidate model $CM_K$, then the candidate model is discarded from further evaluation. Else, the $|MCC_{max}|$ value is considered as the 'train-*MCC*' value ($|MCC_{train}|$) for the model *ITS* and the model and its corresponding $T_{max}$ threshold is used to classify the training biological samples in the internal-test set (*ITS*). In another implementation of the

process, the $MCC_{max}$ threshold may not be applied for retaining the candidate model for subsequent evaluation. Before classifying the each of the training biological samples in the *ITS* set, the values of features characterizing the training biological samples of the *ITS* set are transformed using the method mentioned in step 418 while using the earlier obtained values of $\widetilde{Q1}_J$, $\widetilde{Q3}_J, \widetilde{Q2_{A_J}}$ and $\widetilde{Q2_{B_J}}$ from the *ITR* set.

- Step 7: The classification results on the training biological samples from the *ITS* set are compared against the true/original classes of the training biological samples (with pre-assigned labels), and the *MCC* for the model $CM_K$ and its corresponding $T_{max}$ threshold on the *ITS* samples is calculated ($MCC_{test}$).
- Step 8: A model evaluation score (*MES*) for candidate model $CM_K$ is calculated as $MES =|(MCC_{train} + MCC_{test})| - | (MCC_{train} - MCC_{test})|$

In the next step 432, the model $CM_K$ is tagged as a "strong model" if all the features in the corresponding candidate feature set satisfies the Mann-Whitney test based shortlisting criteria described above. Otherwise, if any of the features in the corresponding feature set fails to satisfy the Mann-Whitney test, the model $CM_K$ is tagged as a "weak model".

Further, the above process is repeated for candidate models and respective *MES* scores are used to rank all the models. The best model is subsequently chosen based on the *MES* score. In case, there are more than one model with the best *MES* score, the best model is chosen based on the following criteria (in order of preference):

(a) the model with fewer number of features (i.e., based on a smaller candidate feature set) is chosen.
(b) the model with lower $T_{max}$ (threshold value) is chosen.

Further, the best model obtained through above steps is tagged as a forward model ($MD_{fwd}$). The model $MD_{fwd}$ additionally constitutes its corresponding $T_{max}$ threshold, the $CMS_{Kmax}$ and $CMS_{Kmin}$ values, and the $\widetilde{Q1}_J$, $\widetilde{Q3}_J$, $\widetilde{Q2_{A_J}}$ and $\widetilde{Q2_{B_J}}$ values corresponding to the *ITR* set.

In the next step 434, the tags assigned to the healthy class (A) and the unhealthy class (B) of the plurality of samples present in the training data are swapped. At step 436, all of the above steps 404 to 432 to determine the best model are repeated after swapping the class labels (A <-> B) for the entire training set (*TR*) to obtain a best model tagged as the reverse model ($MD_{rev}$). The reverse model ($MD_{rev}$) additionally constitutes its corresponding $T_{max}$ threshold, the $CMS_{Kmax}$ and $CMS_{Kmin}$ values, and the $\widetilde{Q1}_J, \widetilde{Q3}_J, \widetilde{Q2_{A_J}}$ and $\widetilde{Q2_{B_J}}$ values corresponding to the *ITR* set.

At step 438, a plurality of forward models and a plurality of reverse models are generated by repeating step (404) through (436) for a predefined number of times using randomly partitioned internal training set and the internal test set. The steps (404) through (436) are iterated 'R' times using multiple randomly partitioned *ITR* and *ITS* sets generated initially. After each iteration, (i) the features constituting the models $MD_{fwd}$ and the models $MD_{rev}$ obtained in the current iteration (r) are compared against, and if necessary, appended to, a set of unique features $F_{unq}$ that consists of respective features constituting the $MD_{fwd}$ and $MD_{rev}$ obtained in earlier iterations (i.e., up to iteration r - 1). After 'R' iterations, a plurality of forward models and a plurality of reverse models are generated for a predefined number of times using randomly partitioned internal training set and the internal test set. The iterations proceed while the value of R satisfies the following criteria -

(i)

$$R \leq R_{max}$$

(ii)

$$(|F_{unq}| \text{ after iteration R}) > (|F_{unq}| \text{ after iteration } R - R_{unq})$$

(iii)

$$|F_{unq}| \text{ after iteration no. } R <= Fet_{max}$$

Wherein, $R_{max}$ is a parameter indicating the maximum number of iterations allowed;

$R_{unq}$ is a parameter indicating the maximum number of iterations allowed without any cumulative increase in the number of unique features $|F_{unq}|$ in the models being generated in consecutive iterations; and

$Fet_{max}$ is a parameter indicating the maximum allowed value of $|F_{unq}|$ (i.e., the no. of unique features cumulated through the iterative process).

In an embodiment, the exemplary values of $R_{max}$, $R_{unq}$, and $Fet_{max}$ are 100, 10, 100 respectively for the present disclosure. Other values of these and other parameters here for finetuning and suitability for other datasets are within the scope of the present invention.

In the next step at 440, an ensemble of forward models is generated using the plurality of forward models and an ensemble of reverse models is generated using the plurality of reverse models. This is referred as an ensemble of forward models (ENS-$MD_{fwd}$)) and an ensemble of reverse models (ENS-$MD_{rev}$).

At step 442, the best models from each of these ensembles, i.e., the best of the forward models ($BMD_{fwd}$) and the best of the reverse models ($BMD_{rev}$) respectively, are identified.

If all models in an ensemble are weak models, the best model from the ensemble ($BMD$) is chosen by ranking the models based on their model evaluation scores and associated criteria. Also, if an ensemble contains more than one strong model, then only those strong models are considered for ranking based on their model evaluation scores and associated criteria as mentioned above, and the best model from the ensemble ($BMD$) is thereby chosen.

In the next step 444, a final single model ($FM_{single}$) is chosen as the ensemble classification model from amongst the best forward model and the best reverse model based on how they classify the individual samples from the training data. Once the best models from each of the ensemble of forward models and the ensemble of reverse models, i.e., the best of the forward models ($BMD_{fwd}$) and the best of the reverse models ($BMD_{rev}$) are identified, the final single model ($FM_{single}$) is chosen from amongst $BMD_{fwd}$ and $BMD_{rev}$ based on how well they can classify the individual training biological samples from the entire training set ($TR$). The AUC value for ROC curves for each of these two models are computed based on the predicted model scores for the training set (TR) samples and their pre-assigned classes (the healthy class (A) and the unhealthy class (B)). The model having the best AUC for ROC value is selected as the final single model ($FM_{single}$). If both $BMD_{fwd}$ and $BMD_{rev}$ have the same AUC value, $BMD_{fwd}$ is chosen as $FM_{single}$.

In an alternate implementation $FM_{single}$ can be chosen based whether $BMD_{fwd}$ or $BMD_{rev}$ obtains a higher $MCC$ value while classifying the $TR$ training biological samples. Once the $FM_{single}$ model has been chosen, for classification of any samples from a test set (TS) or any sample data received during actual deployment, the $FM_{single}$ model is used after:

(a) appropriately transforming the features corresponding to the training biological sample being classified using the

$\widetilde{Q1}_J$, $\widetilde{Q3}_J$, $\widetilde{Q2_{A_J}}$ and $\widetilde{Q2_{B_J}}$ values corresponding to the $FM_{single}$ model,

(b) limiting the model score between a maximum of $CMS_{K_{max}}$ and a minimum of $CMS_{K_{min}}$ values corresponding to the $FM_{single}$ model, and

(c) classification based on the model score using its corresponding threshold $T_{max}$.

According to an embodiment of the disclosure, the ensemble of forward models (ENS-$MD_{fwd}$) and the ensemble of reverse models (ENS-$MD_{rev}$) are also evaluated for respective collective classification efficiencies using an ensemble model scoring. In the ensemble scoring method, each of the models ($MD$) constituting an ensemble (ENS) are used to generate a model score ($MS$) for each of the samples from the entire $TR$ set. For any specific training biological sample, the values of the features corresponding to the training biological sample are appropriately transformed using the

$\widetilde{Q1}_J$, $\widetilde{Q3}_J$, $\widetilde{Q2_{A_J}}$ and $\widetilde{Q2_{B_J}}$ values corresponding to the model MD. The model scores ($MS$) are then transformed into scaled model scores ($SMS$) having values between -1 and +1, using the following procedure:

$$SMS = (MS - T_{max})/(CMS_{K_{max}} - T_{max}), \ldots\ldots \text{ when } MS >= T_{max},$$

and

$$SMS = (MS - T_{max})/(T_{max} - CMS_{K_{min}}), \ldots\ldots \text{ when } MS < T_{max},$$

Wherein, $T_{max}$, $CMS_{K_{max}}$, and $CMS_{K_{min}}$ values corresponding to the respective model is used.

Let $SMS_{avg}$ be the average of all $SMS$ obtained using all models in ENS for a particular sample.

When using Forward model [ENS-$MD_{fwd}$],

$$SMS_{avg} = SMS_{avg} * (+1)$$

If $SMS_{avg}$ >= 0, sample is classified as the unhealthy class (B)
If $SMS_{avg}$ < 0, sample is classified as the healthy class (A)
When using Reverse model [ENS-$MD_{rev}$]:

$$SMS_{avg} = SMS_{avg} * (-1)$$

If $SMS_{avg}$ > 0, sample is classified as the unhealthy class (B)
If $SMS_{avg}$ <= 0, sample is classified as the healthy class (A)

If all models in one of the ensembles are weak models, then the other one having (one or more) strong models is selected as a final ensemble model ($FM_{ens}$), and subsequently used for classification of any of training biological samples from a test set *(TS)* or any sample data received during actual deployment of the method, using the scoring and classification process mentioned in above paragraph. If both ensembles have constituent strong models, then both the ensembles are evaluated for their efficiency by scoring them on all individual samples in *TR.* The AUC value for ROC curves for each of these two ensembles are computed based on the predicted $SMS_{avg}$ for all the training set (*TR*) samples and their pre-assigned classes. The ensemble of models having the best AUC for ROC value is selected as the final ensemble model ($FM_{ens}$). In case both ENS-$MD_{fwd}$ and ENS-$MD_{rev}$ exhibit equal AUC values then ENS-$MD_{fwd}$ is chosen as the final ensemble model ($FM_{ens}$). In an alternate implementation, $FM_{ens}$ can be chosen based whether ENS-$MD_{fwd}$ and ENS-$MD_{rev}$ obtains a higher average MCC value for their respective constituent models while classifying the *TR* samples.

Thus, either the $FM_{single}$ model or $FM_{ens}$ ensemble of models is considered as the pre-determined machine learning (ML) model and can be used for classification of any of training biological samples from a test set (*TS*) or any training biological sample data received during actual deployment.

In an embodiment, one or more predetermined microbes out of the plurality of predetermined microbes associated with the biological sample, are common to the first multiplexed qPCR run (Run 1), the second multiplexed qPCR run (Run 2), and the third multiplexed qPCR run (Run 3) for determining the associated quantitative abundance. In an embodiment, the one or more predetermined microbes that are common to the first multiplexed qPCR run (Run 1), the second multiplexed qPCR run (Run 2), and the third multiplexed qPCR run (Run 3) are determined based on (i) a median abundance (obtained from microbial abundance data) of each of the plurality of predetermined microbes obtained from the plurality of training biological samples, (ii) a frequency of occurrence of each of the plurality of predetermined microbes constituting the ensemble ML model associated with the biological sample. More specifically, the one or more predetermined microbes (from amongst the set of predetermined microbes) has/ have the highest (or relatively higher) median abundance or frequency of occurrence (as compared to the median abundance(s) or the frequency of occurrence of each microbe in the remaining set of predetermined microbes) across the plurality of training biological samples is/ are common to the first multiplexed qPCR run (Run 1), the second multiplexed qPCR run (Run 2), and the third multiplexed qPCR run (Run 3).

For example, a predetermined microbe having a high median abundance or a high frequency of occurrence from the microbial abundance data is determined and utilized in more than one Run. As shown in FIG. 3, the predetermined microbe *Murimonas* is common for both the first multiplexed qPCR run (Run 1) and the second multiplexed qPCR run (Run 2). Similarly, the predetermined microbe *Clostridium sensu stricto* is common for all the first multiplexed qPCR run (Run 1), the second multiplexed qPCR run (Run 2), and the third multiplexed qPCR run (Run 3).

In an embodiment, the quantitative abundance determination involves creating abundance or feature table and generation of the percent normalized abundance or feature table having percent normalized abundance values of the predetermined microbes or operational taxonomic units (OTUs) or taxa in each sample. In another embodiment, Multicolour Combinatorial Probe Coding (MCPC) qPCR or real-time PCR based measurement of abundance of the microbial OTUs or taxa can also be considered for quantification of a predefined set of taxa. Alternatively, any other pre-processing techniques or data normalization techniques known in the state of art can be used for normalization and feature selection from the main feature table.

**Design configuration & number of multiplexed qPCR runs required for quantifying the abundance of target microbes or microbial taxonomic groups or microbial taxa/ features:**

The quantitative abundance of each of the microbial taxonomic groups or microbes, that are common to each of the multiplexed qPCR runs (the first multiplexed qPCR run, the second multiplexed qPCR run, and the, the third multiplexed qPCR run), is determined based on a normalizing factor ($NF_{run}$) associated with each multiplexed qPCR run and the quantitative abundance of associated microbial taxonomic group in the corresponding multiplexed qPCR run.

For example, considering a maximum of five unique DNA fragments, each representing a microbial taxa or spike DNA, can be quantified in a one multiplexed qPCR run. Therefore, to analyze a disease signature (captured in an ML model) comprising of 'n' microbial taxa/ features, a minimum of $(1 + \lceil (n-4)/4 \rceil)$ multiplexed qPCR runs would be required wherein *'n'* is the unique number of microbial taxonomic groups constituting the frugal set of markers, and wherein each multiplexed qPCR run is configured to determine, in the test biological sample (received at step 202 of the method 200), the relative abundance of a predetermined subset of the microbial taxonomic groups constituting the disease signature. This minimum number is based on assumptions that:

(a) the spike DNA should be analyzed at least once in one of the '$(1 + \lceil (n-4)/4 \rceil)$' multiplexed qPCR runs; and

(b) an overlap of at least one microbial taxa/ features was done between two corresponding runs.

For example, if a disease signature comprises of 8 microbial taxa (A, B, C, D, E, F, G, and H), then at least TWO multiplexed qPCR runs would be required, where Z is the spike DNA of known concentration and taxa 'D' is analyzed in both multiplexed qPCR runs. Here, $\lceil (n-4)/4 \rceil$ indicates a ceiling value of the expression. Thus, the minimum no. of required qPCR runs would be:

1 for 1-4 signatures/ features
2 for 5-8 signatures/ features
3 for 9-12 signatures/ features
4 for 13-16 signatures/ features, and so on...

**Example A: Run 1:** Z A B C **D; Run2: D** E F G H

Similarly, for a feature size of 12 (A, B, C, D, E, F, G, H, I, J, K, and L), at least THREE multiplexed qPCR runs would be required, where Z is the spike DNA of known concentration and taxa 'D' and 'H' are analyzed in twice.

**Example B: Run 1:** Z A B C **D; Run 2: D** E F G **H; Run 3: H** I J K L

If the number of features constituting the signature is not optimal for the above condition, i.e., for e.g., the number of features is 10, then more than one microbial taxon can be analyzed twice. The same is exemplified below, wherein taxa C and D are analyzed twice (in Runs 1 and 2). Similarly, taxa F and G are also analyzed twice (in Runs 2 and 3).

**Example C: Run 1:** Z A B **C D; Run 2: C D** E **F G; Run3: F G** H I J

In alternate implementations, the spike DNA (Z) can be analyzed in each of the runs. In that scenario, the first multiplexed qPCR will be able to accommodate up to FOUR features. Each additional multiplexed qPCR run will accommodate up to THREE new/ additional features as shown by underlining in the example below. Thus, two multiplexed qPCR runs would be required for a feature set of up to seven; three qPCR runs for a feature set of up to ten and so on.

**Run 1: Z** A B C **D** ; **Run 2: Z D** E F **G** ; **Run 3: Z G** H I J

Furthermore, if the number of features is not optimal for the above condition, then two or more taxa/ features can be analyzed multiple times as shown in example C.

**Methodology to interpret/ quantify the abundance of a microbial taxon or microbes or microbial taxonomic groups from data obtained from above qPCR configurations:**

Given that the concentration of the spike DNA (Z) is previously known - say $X_1$. If the measured concentration of Z in the multiplexed qPCR is $X_2$, then all the measured concentration in a single multiplexed qPCR run can be normalized multiplying by a normalizing factor ($NF_{run}$) of $X_1/X_2$.

In cases where the spike DNA is only analyzed in only one of the multiplexed qPCR runs (as shown in examples A, B and C), then the normalized values of the taxa/ feature in the first run which is/ are re-analyzed in the Run 2, can be used for adjusting the concentrations inferred from the Run 2 of the multiplexed qPCR. Following Example-A (described previously),

Actual conc of $Z$: $X_1$
Measured conc of $Z$: $X_2$
Normalizing factor $NF_{run1}$: $X_1/X_2$
Inferred conc. of A (from Run 1): $A'_{run1} \times NF_{run1}$
Inferred conc. of B (from Run 1): $B'_{run1} \times NF_{run1}$
Inferred conc. of C (from Run 1): $C'_{run1} \times NF_{run1}$
Inferred conc. of D (from Run 1): $D'_{run1} \times NF_{run1}$

Where $A'_{run1}$, $B'_{run1}$, $C'_{run1}$, and $D'_{run1}$ are the measured/ analyzed concentrations of taxa/ feature A, B, C and D respectively.
Normalizing factor $NF_{run2}$: Inferred conc. of D from Run 1/ Measured concentrations of feature D in Run 2

Inferred conc. of E: $E'_{run1} \times NF_{run2}$
Inferred conc. of F: $F'_{run1} \times NF_{run2}$
Inferred conc. of G: $G'_{run1} \times NF_{run2}$
Inferred conc. of H: $H'_{run1} \times NF_{run2}$

The same protocol may be repeated for normalizing/ adjusting the concentrations measured from all subsequent runs (as in example B). In case wherein more than once feature is analyzed in subsequent runs (as in example C), a median Normalizing factor ($NF$) - derived from the $NFs$ for each of the replication features may be used for computing the inferred concentrations from that run.

In alternate implementations, wherein the spike DNA (Z) is analyzed in each of the runs (as in example D), Normalizing factor ($NF$) corresponding to each of the runs may be computed and used for inferring the concentrations of the constituent features. In cases, where the measured spike DNA (Z) concentration varies by more than 25% from the actual concentration, it is suggested that the observations from the said multiplexed qPCR run be discarded, and a fresh multiplexed qPCR run for the sub-set of features be performed.

In an alternate implementation using multiplexed qPCR runs, the marker feature (marker microbe or taxa) having the lowest variance in relative abundance in training data across both the classes, is selected as the anchor marker ($AM$), and the relative abundance of each of the markers is computed by multiplying the ratio of their estimated/inferred DNA concentrations and the estimated/inferred DNA concentration of $AM$ with the median abundance of $AM$ across all training data. For example, if the marker features are A, B, C and D. wherein A is the anchor marker ($AM$) having a median abundance of $ABN_{AM}$, then the abundances of the marker features B, C and D will be computed as;

$$ABN_B = (Inferred\ conc.\ of B / Inferred\ conc.\ of A) \times ABN_{AM}$$

$$ABN_C = (Inferred\ conc.\ of C / Inferred\ conc.\ of A) \times ABN_{AM}$$

$$ABN_D = (Inferred\ conc.\ of D / Inferred\ conc.\ of A) \times ABN_{AM}$$

At step 208 of the method 200, the quantitative abundance is collated through the abundance determining module 112. The quantitative abundance of each of the plurality of predetermined microbes associated with the biological sample, determined at step 206 of the method 200 is collated to obtain a microbial abundance matrix.

At step 210 of the method 200, a model score is determined based on the microbial abundance matrix obtained at step 208 of the method 200, through the ML module 114. The ML module 114 includes a pre-determined machine learning (ML) model explained and obtained at step 206 of the method 200 is employed to determine the model score based on the microbial abundance matrix.

At step 212 of the method 200, the category of MBD for the subject is predicted through the assessment module 116. The categorization is predicted based on the model score obtained at step 210 of the method 200 and a pair of predefined threshold values. More specifically the category of Mammographic Breast Density (MBD) for the subject, is predicted into one of (i) a high category, (ii) a medium category, and (iii) a low category, based on the model score and the pair of predefined threshold values. The pair of predefined threshold values includes a first predefined threshold value and a second predefined threshold value. For example, if the model score obtained at step 210 of the method 200 is less than or equal to the first predefined threshold value, then the subject is predicted to be having low category of MBD. If the model score obtained at step 210 of the method 200 is greater than or equal to the second predefined threshold value, then the subject is predicted as having the high category of MBD. If the model score obtained at step 210 of the method 200 is greater than the first predefined threshold value and less than the second predefined threshold value, then the subject is predicted as having the medium category of MBD.

The predicted category helps in choosing one or more downstream techniques for assessing the presence of one or more breast lesions in the subject. In an embodiment, the one or more downstream techniques are selected from a list comprising of a mammogram, an ultrasound scan, a breast magnetic resonance imaging (MRI) scan, a computed tomography (CT) scan, and a positron emission tomography (PET) scan.

Further, the one of more downstream techniques of the ultrasound scan, the breast MRI scan, the CT scan, or the PET scan are suggested for the subject having the predicted MBD category as the high category. Further, in one embodiment, the one of more downstream techniques of the ultrasound scan, the breast MRI scan, the CT scan, or the PET scan are suggested for the subject having the predicted MBD category as either the medium category or the high category. The downstream technique of the mammography is selected for the subject having the predicted MBD category as the low category.

At step 214 of the method 200, the category of breast cancer risk for the subject is additionally predicted through the assessment module 116, into one of (i) a healthy category, and (ii) a breast cancer risk category, based on the model score and a predefined threshold value. The predefined threshold value at this step is different to the pair of the predefined threshold values described at step 212 of the method 200. The model score is compared with the predefined threshold value at this step, to predict the category of breast cancer risk for the subject and to classify into one of (i) the healthy category, and (ii) the breast cancer risk category. For example, if the model score is less than or equal to the predefined threshold value then the subject is predicted under the healthy category, else the subject is predicted under the breast cancer risk category.

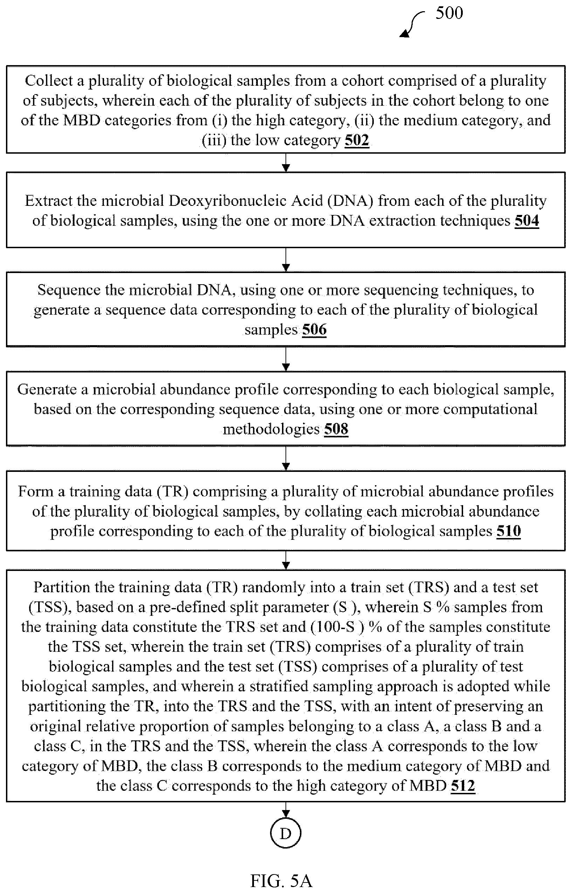

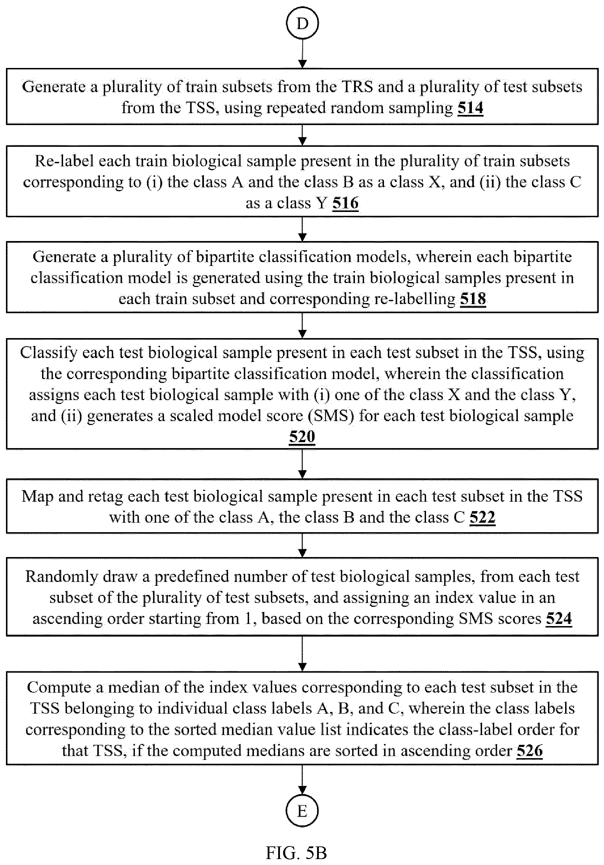

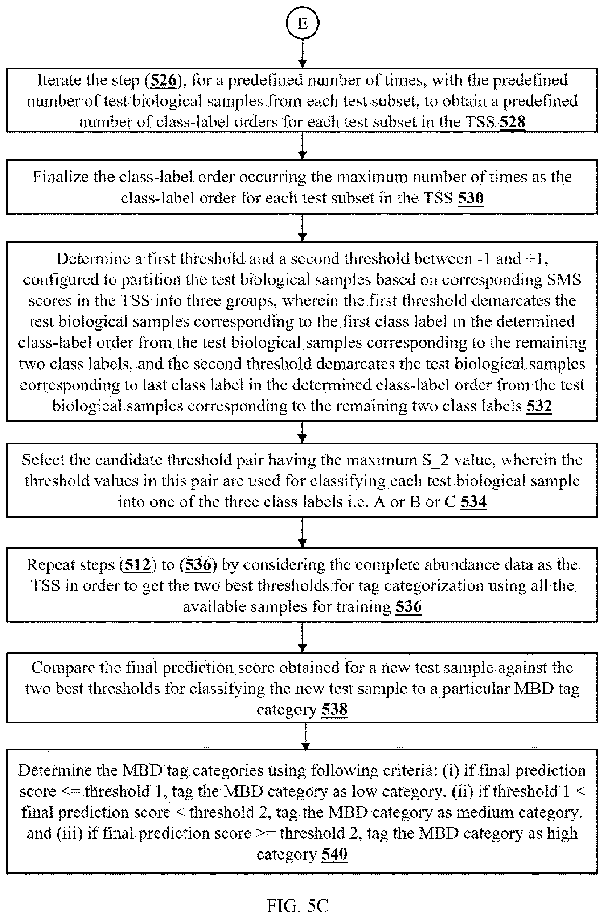

FIGS. 5A, 5B and 5C are flowcharts illustrating steps 500 involved in calculating the pair of predefined threshold values described at step 212 of the method 200, according to some embodiments of the present disclosure. Initially, at step 502, a plurality of biological samples is collected from a cohort comprised of a plurality of subjects. Each of the plurality of subjects in the cohort are belong to one of the MBD categories from one of (i) the high category, (ii) the medium category, and (iii) the low category. The plurality of biological samples is similar are same as that of the plurality of training biological samples specified while generating the predetermined ML model.

At step 504, the microbial Deoxyribonucleic Acid (DNA) is extracted from each of the collected plurality of biological samples, using the one or more DNA extraction techniques explained at step 204 of the method 200. At step 506, the extracted microbial DNA is sequenced using the one or more sequencing techniques to generate a sequence data corresponding to each of the plurality of biological samples.

Further at step 508, one or more microbial abundance profiles corresponding to each of the plurality of biological samples, are generated based on the corresponding sequence data, using one or more computational methodologies. At step 510, a training data (TR) comprising a plurality of microbial abundance profiles, is obtained by collating the one or more microbial abundance profiles corresponding to each of the plurality of biological samples

At step 512, the training data (TR) is randomly partitioned into a train set (TRS) and a test set (TSS), based on a pre-defined split parameter (S). Wherein, S % samples from the training data constitute the TRS set and (100 - S) % of the samples constitute the TSS set. A stratified sampling approach is adopted while partitioning the TR, into the TRS and the TSS, with an intent of preserving an original relative proportion of samples belonging to a class A, a class B and a class C, in the TRS and the TSS, wherein the class A corresponds to the low category of MBD, the class B corresponds to the medium category of MBD and the class C corresponds to the high category of MBD. After the partitioning, the train set (TRS) comprises (referred as) of a plurality of train biological samples and the test set (TSS) comprises (referred as) of a plurality of test biological samples. At step 514, a plurality of train subsets from the TRS and a plurality of test subsets from the TSS are generated using repeated random sampling. For example, 100 subsets of TRS and TSS are generated using repeated random sampling. At step 516, each train biological sample present in the plurality of train subsets corresponding to (i) the class A and the class B are re-labelled as a class X, and (ii) the class C re-labelled as a class Y.

In the next step 518, a plurality of bipartite classification models, are generated using the plurality of train subsets in the TSS. More specifically, each bipartite classification model is generated using the train biological samples present in each train subset and corresponding re-labelling. In an embodiment, the plurality of bipartite classification models is generated in the similar process as used for generating the pre-determined machine learning (ML) model.

At step 520, each test biological sample present in each test subset in the TSS, is classified using the corresponding bipartite classification model of the plurality of bipartite classification models. The classification in this step assigns each test biological sample with (i) one of the class X and the class Y, and (ii) generates a scaled model score *(SMS)* for each test biological sample. For example, each sample in each of the 100 TSS subsets obtain a classification label (X or Y) and a corresponding scaled model scores (*SMS*) ranging between -1 to +1. At step 522, each test biological sample present in each test subset in the TSS is mapped and retagged with one of the class A, the class B, and the class C,

At step 524, a predefined number of test biological samples are randomly drawn from each test subset of the plurality of test subsets in the TSS, and an index value is assigned in an ascending order starting from 1, based on the corresponding *SMS* scores. For example, 70% of samples are randomly drawn and sorted from each TSS set based on their *SMS* scores in an ascending order. If *N* is the number of samples drawn, then each sample in the sorted list is tagged to an index value ranging between 1 and *N.*

At step 526, a median of the index values corresponding to each test subset in the TSS belonging to individual class labels A, B, and C, is computed. The class labels corresponding to the sorted median value list indicates the class-label order for that TSS, if the computed medians are sorted in ascending order. Further the step 528, the step (526) is iterated for a predefined number of times, with the predefined number of test biological samples from each test subset in the TSS, to obtain a predefined number of class-label orders for each test subset in the TSS. For example, the step (526) is iterated 100 times with 70% of samples being drawn randomly each time, resulting in generation of 100 class-label orders for each TSS set.

At step 530, the class label order occurring the maximum number of times is finalized as the class-label order for each test subset in the TSS (Tie).

In the next step 532, a first threshold and a second threshold are determined between -1 and +1. The first threshold and the second threshold are configured to partition the test biological samples based on the corresponding *SMS* scores in the test set into three groups. The first threshold demarcates the test biological samples corresponding to the first class label in the determined class-label order from the samples corresponding to the remaining two class labels, and the second threshold demarcates the test biological samples corresponding to last class label in the determined class-label order from the test biological samples corresponding to the remaining two class labels, wherein the first threshold and the second threshold are determined using following steps through 532a to 532d.

At step 532a, the *SMS* scores are sorted corresponding to each test sample in the TSS set in an ascending order. At step 532b, averages of each consecutive pair of *SMS* scores are computed in the sorted list. In the next step 532c, test biological samples in the TSS set are grouped into three groups (F, M and L) using a candidate threshold pair comprising of a pair of average scores (from all possible pairs of average scores in the sorted list), wherein F, M, and L correspond to the first, middle and last elements in the previously determined class-label order for a particular TSS set, wherein the elements (F, M, and L) correspond to one of the original class labels i.e. the high category, the medium category or the low category. In the next step 532d, two confusion matrices (a first confusion matrix and a second confusion matrix) are created by comparing the original class labels in step 516 and the labels as obtained in step 532c.

The values in the first confusion matrix indicate the prediction accuracy of test biological samples corresponding to the first element in the class-label order, wherein the two categories for determining TP, TN, FN, FP values are (F vs !F), wherein F and !F indicates test biological samples falling under group F and group (M and L). The values in the second confusion matrix indicate the prediction accuracy of test biological samples corresponding to the last element in the class-label order, wherein the two categories for determining TP, TN, FN, FP values are (L vs !L), wherein L and !L indicates test biological samples falling under group L and group (F and M). Based on the values in the confusion matrices, a pair of *MCC* (Mathew's correlation coefficient) values ($MCC_1$ and $MCC_2$) are computed (and thus available for each candidate threshold pair).

At step 534, a first score $S_1$ and a second score $S_2$ are computed using the pair of *MCC* values using the following formulae:

$$S_1 = |MCC_1| + |MCC_2|$$

$$S_2 = |MCC_1| + |MCC_2| - ||MCC_1| - |MCC_2||$$

In the next step 536, the candidate threshold pair having the maximum $S_2$ value is selected. In case of a tie in $S_2$ values, the candidate threshold pair having maximum $S_1$ value is selected and the threshold values in this pair are used for classifying the test biological sample into one of the three class labels i.e. A or B or C. Further at step 438, steps 412 to 436 are repeated by considering the complete abundance data as the TSS in order to get the two best thresholds for tag categorization using all the available samples for training. In the next step 540, the final prediction score obtained is compared for a new test sample against the two best thresholds for classifying the new test sample to a particular MBD tag category.

And finally at step 542, the MBD tag categories are determined using following criteria:

If final prediction score <= threshold 1, the MBD tag category is low category;
If threshold 1 < final prediction score < threshold 2, the MBD tag category is medium category;
If final prediction score >= threshold 2, the MBD tag category is high category.

At step 216 of the method 200, a personalized therapeutic recommendation for the based on the category predicted for the subject (one of the high or the medium or the low category) at step 212 and 214 of the method 200, is designed through the recommendation module 118. In an embodiment, the personalized therapeutic recommendation includes utilizing a set of rules for the set of microbes that constitute the pre-determined machine learning model to identify one or more personalized probiotic and antibiotic target candidates that may be employed to ameliorate disease symptoms in the subject predicted as having the breast cancer risk category.

In an embodiment, the microbes (organisms) contributing to generation of model score at step 210 are mapped to a predefined set of antibiotic target candidates and probiotic candidates, and appropriate personalized targets for treatment and recommendation are identified accordingly.

More specifically, the personalized recommendation includes utilizing the plurality of predetermined microbes constituting the pre-determined machine learning model to identify the one or more antibiotic target candidates and the probiotic candidates that ameliorate the risk of breast cancer. The designing of the one or more antibiotic target candidates is performed by mapping the features constituting the ML model to the complete set of microbes using the following steps:

At step 1, pair-wise correlations (using the Pearson's and/or spearman's correlation index) are computed between abundances of features (i.e., organisms/ taxa/ microbes) constituting the ML model and the abundances corresponding to the complete set of microbes computed individually from (a) the subset of biological samples corresponding to the healthy class i.e. the class of samples that are taken from individuals not having the risk of breast cancer, and (b) the diseased class i.e. the class of samples that are taken from individuals having the risk of the breast cancer). Wherein the samples belonging to the healthy and diseased classes are used as training data for generating the ML model.

At step 2, positive and negative interactions between features (i.e., organisms/ taxa/ microbes) constituting the ML model and all other taxa in the healthy and the diseased class of training samples (individually) are deduced using critical correlation (r) value as the cut-off (as taught in Batushansky et al., 2016), such that inter-taxa correlation index values greater than $+r$ value are affiliated as 'positive interactions', while those less than $-r$ value are affiliated as 'negative interactions'.

At step 3, the steps 1 and 2 are repeated 1000 times and only those interactions are considered relevant that appear in at least 70% of iterations with a BH (Benjamini-Hochberg) corrected p-value cut-off of 0.1 are retained (hereafter referred to as model taxa interactions corresponding to healthy and diseased class of samples).

At step 4, thereafter, following set of rules (indicated in Table 1 below) are used to arrive at the relevant candidate using the retained model taxa interactions:

Table 1

| Probiotic Candidates | Antibiotic Candidates |
|---|---|
| $(M_H - C_T)_{HP}$ && $(M_H - C_T)_{DP}$ <br> $(M_H - C_T)_{DP}$ <br> $(M_D - C_T)_{HN}$ && $(M_D - C_T)_{DN}$ | $(M_H - C_A)_{HN} \parallel (M_H - C_A)_{DN}$ <br> $(M_D - C_A)_{HP} \parallel (M_D - C_A)_{DP}$ |

From Table 1,

$M_H$ represents a model taxon having significantly higher abundance in healthy class;
$M_D$ represents a model taxon having significantly higher abundance in diseased (unhealthy) class;
$C_T$ represents a potential candidate for recommendation;
$C_A$ represents a potential antibiotic target candidate;
$M_H - C_T$ represents an interaction between a model taxon (abundant in healthy class) with a potential candidate for recommendation;
$M_D - C_T$ represents an interaction between a model taxon (abundant in diseased class) with a potential candidate for recommendation;
$M_D - C_A$ represents an interaction between a model taxon (abundant in diseased class) with a potential antibiotic target candidate;
$M_H - C_A$ represents an interaction between a model taxon (abundant in healthy class) with a potential antibiotic target candidate;
$HP$ represents a positive interaction in a healthy environment population;
$HN$ represents a negative interaction in a healthy environment population;
$DP$ represents a positive interaction in a diseased environment population; and
$DN$ represents a negative interaction in a diseased environment population.

One or more of the set of microbes constituting the identified probiotic candidates may be recommended (individually or in combination) as probiotic formulations for treating (or ameliorating the symptoms of or the disease severity of) individuals identified as having the risk of breast cancer. Furthermore, the mentioned probiotic formulations may help in promoting development of a healthy gut microbiome (in the individuals administered with the probiotic) which may be employed to ameliorate the symptoms of, or the disease severity of individuals identified as having the risk of breast cancer.

One or more of the set of microbes constituting the identified antibiotic microbial candidates may be targeted (individually or in combination) via antibiotics or other treatment methodologies that can reduce the abundance of the identified antibiotic microbial candidate(s) and may be recommended for ameliorating the symptoms of or the disease severity of individuals identified as having the risk of the breast cancer. Furthermore, such antibiotic recommendation (as detailed above) may also help in promoting development of a healthy gut microbiome, which (may) ameliorate the symptoms of, or the disease severity of individuals identified as having the risk of the breast cancer.

The approach of the personalized recommendation is aimed at alleviating the disease symptoms, arresting the progression of disease, cure, or prevention of disease. The microbes abundant in disease class are expected to play significant role in the disease progression and hence are possible antibiotic targets. Alternatively, microbes more abundant in controls are expected to have pro-health benefits. The therapy is likely to shift the unstable/ perturbed microbiome towards a healthy equilibrium.

The approach relates to suggesting a therapeutic/ prophylactic microbial isolate or a specific consortia/ combination of microbial isolates (derived from the model ensemble) that can treat or ameliorate symptoms related to breast cancer in the subject at-risk. The recommendation can be administered in form of injection, syrup, pills, sprays, chewing gums, mouth wash etc. In current implementation, the features which are more abundant in controls in the model ensemble are suggested to be administered as probiotic candidates. In contrast, the features which are found to be abundant in cases in the model ensemble are suggested to be utilized as antibiotic targets.

In another embodiments, the role of microbiome in breast cancer for therapy are as follows:

- Combining the presently discussed approach of screening with other currently available screening methods to further improve on precision of diagnosis
- Using multiomics approach such as, metabolomics and meta-transcriptomics along with metagenomics approach to get an ensemble of markers, including both probiotics (microbes) and pre-biotics (metabolites) that can improve the therapy regimen.

In another embodiment, alternate utilization of identified microbes/ biomarkers for therapy of breast cancer are detailed below:

For microbes abundant in disease class:

- If the feature(s) is abundant in diseased the feature(s) can be termed as Disease Microbe (DM) and any antibiotic(s) currently known in the literature against DMs can be utilized as therapy.
- Any microbe(s) showing similar population trend (positive correlation) to identified DMs and are currently known in literature to be pathogenic, can also be targeted.
- Any microbial product(s) effective for supporting the microbial population inversely correlated to DMs can also be utilized appropriately.
- Any microbe(s) or microbial product(s) which are not reported to be pathogenic or toxic and reported to be effective against a microbe which has positive correlation to DMs can be targeted.

For microbes abundant in healthy class:

- If the feature(s) is abundant in healthy the feature(s) can be termed as Healthy Microbe (HM) and any probiotic(s) currently known in the literature for HMs can be utilized as therapy.
- Any microbe(s) showing similar population trend to identified HMs (positive correlation) and not known in the literature to be pathogenic, can be administered as probiotic for breast cancer.
- Any microbial product(s) effective for supporting population of the positively correlated microbe(s).
- Any microbe(s) or microbial product(s) which are not reported to be pathogenic or toxic and reported to be effective against a microbe which has a negative correlation to HMs can be targeted.

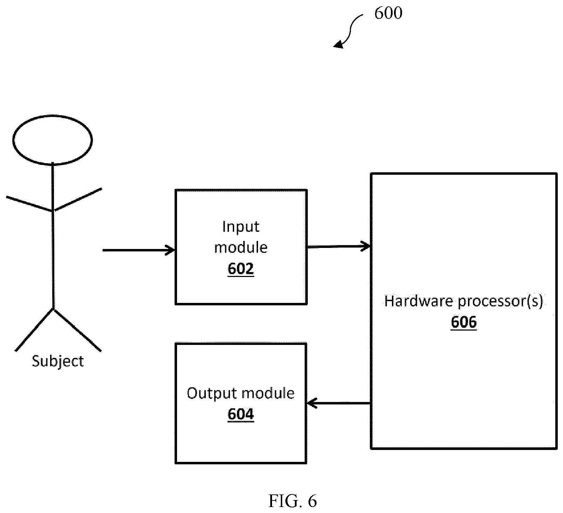

Further, a kit for predicting a category of mammographic breast density (MBD) for a subject, is disclosed. FIG. 6 illustrates an exemplary block diagram of a kit 600 for predicting the category of mammographic breast density (MBD) for the subject, according to some embodiments of the present disclosure. As shown in FIG. 6, the kit 600 includes an input module 602, one or more hardware processors 604 and an output module 606. The input module 602 is used for receiving the biological sample of the subject whose category of MBD is to be predicted. In an embodiment, the input module 602 may be a medium, a carrier, a set of mediums, or a set of carries that can hold the biological sample.

The one or more hardware processors 606 are configured to analyze the biological sample, using the one or more steps of the method 200. In an embodiment, the one or more hardware processors 606 are equivalent or same that of the one or more hardware processors 106 of the system 100. The output module 606 is used for displaying the category of mammographic breast density (MBD) for the subject, based on the analysis of the one or more hardware processors 606. In other words, the output module 606 is used for indicating the category of mammographic breast density. In an embodiment, the output module 606 includes but are not limited to a display device, an indicator, a color indicator, or any other equipment that can show the result representation on the category of mammographic breast density to the subject.

According to an embodiment of the disclosure, the method also provides a sequence of decision-making steps and based on the predicted risk/ tag, the woman at risk is recommended/ suggested a proper guidance on the next steps of the screening process. Additionally, the individual at risk is also directed towards the right confirmatory tests based on risk (calculated using mammographic breast density) to be taken and/ or further treatment modality to follow for best diagnosis and treatment.

The decision support protocol can be applied for the following scenarios:

- A woman opts for assessing risk of breast cancer as a part of routine health check-up. In case of an indication of 'risk', the proposed protocol provides follow-up recommendations that can aid in prevention or accelerated therapy driven by appropriate diagnostic measure (for the individual).
- A woman at high risk of breast cancer (predicted by gene mutation, family history etc.) opts for screening/ risk-assessment test. The proposed microbiome-based method provides the individual with an opportunity to have an idea about the current risk of development of the disease without getting exposed to any radiation-based screening techniques, especially for young women (under 40) where mammography is not recommended according to clinical guidelines. The decision support protocol further guides the healthcare provider/ the individual at risk through follow-up steps for preventive measure or appropriate diagnostic approaches aiding better management of the disease.
- A woman having physical anomalies visits a clinic. In this case, the decision support protocol can guide the healthcare provider to follow certain decision-making steps to arrive on an appropriate diagnostic technique (for the individual), which can significantly reduce the chance of faulty prediction (by the state of art imaging techniques), thus facilitating improved preventive care or prompt initiation of therapeutic procedures.

Example scenario:

According to an embodiment of the present disclosure, the method 200 can also be explained with the help of following example. The example below shows the steps involved in execution of the present disclosure.

**Step 1:** Stool sample is obtained from the subject for whom we intend to screen for breast cancer risk.

**Step 2:** The raw abundances of various microbial taxonomic groups is quantified in the stool sample. Methodology for this involves extraction of microbial DNA contents from the collected stool sample followed by amplification and sequencing of either full-length or specific variable regions of the bacterial 16S rRNA marker genes using a next-generation sequencing platform or by using the multiplex qPCR-based quantification methodology. In either case, the sequencing depth (i.e., number of reads obtained by sequencing the microbial DNA content of the stool sample) obtained should exceed a pre-defined threshold, wherein the threshold refers to the rarefaction depth that needs to be determined and/ or applied to adjust for differences in library sizes across samples in current the sequencing run or for removing the sequencing bias). In an example, the raw abundance of features in a test sample is shown in Table 2.

Table 2: Raw abundance of features in a test sample

| Features | Raw Abundance |
|---|---|
| Butyricicoccus | 159 |
| Clostridium_IV | 95 |
| Clostridium_sensu_stricto | 0 |
| Faecalibacterium | 5936 |
| Flavonifractor | 40 |
| Gemmiger | 465 |
| Lachnospiracea_incertae_sedis | 899 |
| Murimonas | 1 |
| Oscillibacter | 148 |
| .. | |

(continued)

| Features | Raw Abundance |
|---|---|
| .. | |
| Ruminococcus | 329 |
| Sporobacter | 0 |

In an embodiment, the plurality of predetermined microbes associated with the biological sample comprises *Murimonas, Clostridium sensu stricto, Clostridium XIVa*, *Lachnospiracea incertae sedis*, *Blautia, Bacteroides*, *Intestinibacter*, and *Bilophila.*

**Step 3:** The abundances of various taxa employing rarefaction depth is rarified, i.e., minimum library size determined after the sequencing run wherein, rarefaction was done using 'qiime feature-table rarefy' function in qiime tools of qiime2 package. Table 3 shows rarefied abundance of features in the test sample.

Table 3: Rarefied abundance of features in the test sample

| Features | Rarefied Abundance |
|---|---|
| Butyricicoccus | 50 |
| Clostridium_IV | 34 |
| Clostridium_sensu_stricto | 0 |
| Faecalibacterium | 2221 |
| Flavonifractor | 10 |
| Gemmiger | 165 |
| Lachnospiracea_incertae_sed is | 306 |
| Murimonas | 0 |
| Oscillibacter | 46 |
| .. | |
| .. | |
| Ruminococcus | 131 |
| Sporobacter | 0 |

**Step 4:** From the rarefied abundance table, abundances of only the subset of taxa is retained for which overlap with the list of three taxa that are provided against 'Single Best Training Model' as mentioned below in Table 4.

Table 4: Model characteristics of features in the Single Best Training Model

| Features | Murimonas | Clostridium_sensu_ stricto | Lachnospiracea_incertae_ sedis |
|---|---|---|---|
| $Q1$ | 0 | 1 | 209.5 |
| $Q3$ | 1 | 5 | 436.5 |
| $Q2_A$ | 0 | 0 | 336 |
| $Q2_B$ | 1 | 3 | 252 |
| Numerator/ Denominator Feature | Numerator | Numerator | Denominator |
| Min Model Score | 0.5 | | |
| Max Model Score | 5.321305 | | |
| Threshold | 1.402786 | | |
| Model Type | Reverse | | |

As an example, assume that the three taxa in the taxonomic abundance profile obtained by processing the stool sample (in the manner mentioned in Steps 1 and 2) had the following rarefied abundances:

Abundance of Murimonas (i.e., feature 1 in training model) in collected stool sample: 0.000000
Abundance of Clostridium_sensu_stricto (i.e., feature 2 in training model) in collected stool sample: 0.000000
Abundance of Lachnospiracea_incertae_sedis (i.e., feature 3 in training model) in collected stool sample: 306.000000

**Step 5:** Using $Q1$ and $Q3$ values corresponding to each training model feature in the single best model (as mentioned in Table 3), and applying the transformation, to above rarefied abundances, results in the following:

Transformed abundance (FMurimonas): 0.000000
Transformed abundance ($F_{Clostridium\_sensu\_stricto}$): 0.000000
Transformed abundance ($F_{Lachnospiracea\_incertae\_sedis}$): 0.425110

The transformed abundance of individual features as obtained above are then used appropriately in the candidate model equation ($CM_K$) (as replicated below), and numerator and denominator sums are computed. In this case, the values obtained are as follows:

Since Numerator sum = 0 and Denominator sum = 0.425110 in this case, a value of 1 is added to both numerator and denominator

$$CM_K = \frac{\sum F_{numerator}}{\sum F_{denominator}} \ ... \ \text{when } F_{numerator} > 0 \text{ and } F_{denominator} > 0$$

or,

$$CM_K = \frac{\sum F_{numerator} + 1}{\sum F_{denominator} + 1} \ ... \ \text{when either } F_{numerator} \text{ or } F_{denominator} = 0$$

Numerator sum: 1.000000
Denominator sum: 1.425110

**Step 6:** The sample model score ($MS$) is computed next using above Numerator sum and Denominator sum. The sample model score ($MS$) is then transformed into scaled model score ($SMS$) (having values between -1 and +1, using following rules.

$$SMS = (MS - T_{max})/(CMS_{K_{max}} - T_{max}), \ ..... \ \text{when } MS >= T_{max},$$

and

$$SMS = (MS - T_{max})/(T_{max} - CMS_{K_{min}}), \ ..... \ \text{when } MS < T_{max},$$

Wherein, $T_{max}$, $CMS_{K_{max}}$, and $CMS_{K_{min}}$ values corresponding to the respective model. For this purpose, the values of threshold: 1.402786,
Maximum model score: 5.321305,
Minimum model score: 0.500000 for single best model (as mentioned in Table 3) are employed.
Model score ($MS$): 0.701700
Scaled model score ($SMS$): -0.776580

**Step 7:** The $SMS$ is then used for predicting the category of risk for the individual from whom the stool sample was obtained i.e., healthy or 'at-risk for breast cancer'. Since both forward model and reverse model are evaluated (as explained earlier, wherein the final selected model is then used for classification or prediction). Here in this case, final selected single best model is a reverse model, hence the final prediction score value is calculated as ($SMS$ * -1).

Final pred_score is 0.776580.
Since the value is >0, the prediction class is "B" i.e., risk category is breast cancer risk category

Following the same series of steps, if the value of *SMS* is less than 0 then the prediction class will be "A" and the category of risk for the individual from whom the stool sample was obtained will be healthy.

**Step 8:** Similarly, for ensemble model, all the steps are repeated for all the single models in the ensemble and finally mean of all the Final prediction score calculated using sample model scores (*SMS*) and the class prediction is done based on final mean prediction score obtained.

**Step 9:** The final prediction score obtained in Step 7 is then compared against the best threshold pair obtained using the sequential method explained earlier. The best threshold pair consists of threshold 1 = -0.014513 and threshold 2 = 0.134146, in order to determine the Tag category i.e., high/ medium/ low category. Since the final prediction score is 0.776580, which is greater than both threshold 1 and threshold 2, the predicted tag category for the individual from whom the stool sample was obtained is 'high'.

Table 5 shows the performance of the single best ML model and the ensemble ML model. As shown in table 5, significant performance of both the models are observed.

Table 5

| Model Type | CV (100 iterations) Mean AUC | Training AUC | External Test AUC Validated |
|---|---|---|---|
| Ensemble ML model | 0.73 | 0.828429 | 0.685202 |
| Single best ML Model | 0.73 | 0.827976 | 0.806894 |

The embodiments of present disclosure herein address unresolved problem of predicting the risk category of the breast cancer of the subject effectively and invasively, using the microbial abundance profile of the biological sample. As minimum number of the predetermined microbes are considered, the proposed method is fast and requires less resource utilization.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A method (200) for predicting a category of mammographic breast density (MBD) for a subject, the method comprising:

   extracting, microbial Deoxyribonucleic Acid (DNA) from a biological sample collected from a subject, using one or more DNA extraction techniques (204);
   determining, a quantitative abundance of each of a plurality of predetermined microbes associated with the biological sample, from the microbial DNA, using a set of probes specific to each of the plurality of predetermined microbes through a multiplex quantitative Polymerase Chain Reaction (qPCR) technique, wherein the plurality of predetermined microbes comprises *Murimonas, Clostridium sensu stricto, Clostridium XIVa, Lachnospiracea incertae sedis*, *Blautia, Bacteroides*, *Intestinibacter*, and *Bilophila* (206);
   collating, via one or more hardware processors, the quantitative abundance of each of the plurality of pre-determined microbes, to obtain a microbial abundance matrix (208);
   determining, via the one or more hardware processors, a model score, based on the microbial abundance matrix, using a pre-determined machine learning (ML) model (210); and
   predicting, via the one or more hardware processors, the category of MBD of the subject, from one of (i) a high category, (ii) a medium category, and (iii) a low category, based on the model score and a pair of predefined threshold values, wherein the predicted category helps in choosing one or more downstream techniques for assessing the presence of one or more breast lesions in the subject (212).

2. The method (200) as claimed in claim 1, further comprising predicting, via the one or more hardware processors, the category of breast cancer risk for the subject, from one of (i) a healthy category, and (ii) a breast cancer risk category, based on the model score and a predefined threshold value (214).

3. The method (200) as claimed in claim 2, further comprising designing, a personalized therapeutic recommendation for the subject, based on the breast cancer risk category predicted for the subject, by utilizing a set of rules for a set of microbes that constitute the pre-determined machine learning model to identify one or more personalized probiotic and antibiotic candidates that ameliorate disease symptoms in the subject predicted as having the breast cancer risk category (216).

4. The method (200) as claimed in claim 1, wherein the set of probes specific to each of the plurality of predetermined microbes are utilized in a first multiplex qPCR run, a second multiplex qPCR run, and a third multiplex qPCR run to determine the quantitative abundance of each of the plurality of predetermined microbes associated with the biological sample, and wherein:

   the plurality of predetermined microbes, the quantitative abundance of which are being determined through the first multiplex qPCR run are: *Murimonas*, *Clostridium sensu stricto*, *Clostridium XIVa*, and *Lachnospiracea incertae sedis*;
   the plurality of predetermined microbes, the quantitative abundance of which are being determined through the second multiplex qPCR run are: *Murimonas*, *Clostridium sensu stricto*, *Blautia*, and *Bacteroides*; and
   the plurality of predetermined microbes, the quantitative abundance of which are being determined through the third multiplex qPCR run are: *Clostridium sensu stricto*, *Clostridium XIVa*, *Intestinibacter*, and *Bilophila.*

5. The method (200) as claimed in claim 1, wherein the pre-determined machine learning (ML) model is an ensemble ML model built using a microbial abundance data corresponding to a plurality of training biological samples.

6. The method (200) as claimed in claim 1, wherein the plurality of predetermined microbes associated with the biological sample are features of the pre-determined machine learning (ML) model.

7. The method (200) as claimed in claim 4, wherein one or more predetermined microbes out of the plurality of predetermined microbes associated with the biological sample, are common to one or more of the first multiplex qPCR run, the second multiplex qPCR run, and the third multiplex qPCR run, for determining the quantitative abundance, and wherein the one or more predetermined microbes that are common to one or more of the first multiplex qPCR run, the second multiplex qPCR run, and the third multiplex qPCR run are determined based on (i) a median abundance of each of the plurality of predetermined microbes obtained from the plurality of training biological samples, (ii) a frequency of occurrence of each of the plurality of predetermined microbes constituting the ensemble ML model.

8. The method (200) as claimed in claim 1, wherein the pair of predefined threshold values are obtained by:

(a) collecting a plurality of biological samples from a cohort comprised of a plurality of subjects, wherein each of the plurality of subjects in the cohort belong to one of the MBD categories from (i) the high category, (ii) the medium category, and (iii) the low category (502);

(b) extracting the microbial Deoxyribonucleic Acid (DNA) from each of the plurality of biological samples, using the one or more DNA extraction techniques (504);

(c) sequencing, the microbial DNA, using one or more sequencing techniques, to generate a sequence data corresponding to each of the plurality of biological samples (506);

(d) generating, a microbial abundance profile corresponding to each biological sample, based on the corresponding sequence data, using one or more computational methodologies (508);

(e) forming a training data (TR) comprising a plurality of microbial abundance profiles of the plurality of biological samples, by collating each microbial abundance profile corresponding to each of the plurality of biological samples (510);

(f) partitioning the training data (TR) randomly into a train set (TRS) and a test set (TSS), based on a pre-defined split parameter (S), wherein S % samples from the training data constitute the TRS set and (100 - S) % of the samples constitute the TSS set, wherein the train set (TRS) comprises of a plurality of train biological samples and the test set (TSS) comprises of a plurality of test biological samples, and wherein a stratified sampling approach is adopted while partitioning the TR, into the TRS and the TSS, with an intent of preserving an original relative proportion of samples belonging to a class A, a class B and a class C, in the TRS and the TSS, wherein the class A corresponds to the low category of MBD, the class B corresponds to the medium category of MBD and the class C corresponds to the high category of MBD (512);

(g) generating a plurality of train subsets from the TRS and a plurality of test subsets from the TSS, using repeated random sampling (514);

(h) re-labelling each train biological sample present in the plurality of train subsets corresponding to (i) the class A and the class B as a class X, and (ii) the class C as a class Y (516);

(i) generating a plurality of bipartite classification models, wherein each bipartite classification model is generated using the train biological samples present in each train subset and corresponding re-labelling (518);

(j) classifying each test biological sample present in each test subset in the TSS, using the corresponding bipartite classification model, wherein the classification assigns each test biological sample with (i) one of the class X and the class Y, and (ii) generates a scaled model score (*SMS*) for each test biological sample (520);

(k) mapping and retagging each test biological sample present in each test subset in the TSS with one of the class A, the class B and the class C (522);

(l) randomly drawing a predefined number of test biological samples, from each test subset of the plurality of test subsets, and assigning an index value in an ascending order starting from 1, based on the corresponding *SMS* scores (524);

(m) computing a median of the index values corresponding to each test subset in the TSS belonging to individual class labels A, B, and C, wherein the class labels corresponding to the sorted median value list indicates the class-label order for that TSS, if the computed medians are sorted in ascending order (526);

(n) iterating the step (m), for a predefined number of times, with the predefined number of test biological samples from each test subset, to obtain a predefined number of class-label orders for each test subset in the TSS (528);

(o) finalizing the class-label order occurring the maximum number of times as the class-label order for each test subset in the TSS (530);

(p) determining a first threshold and a second threshold between -1 and +1, configured to partition the test biological samples based on corresponding *SMS* scores in the TSS into three groups, wherein the first threshold demarcates the test biological samples corresponding to the first class label in the determined class-label order from the test biological samples corresponding to the remaining two class labels, and the second threshold demarcates the test biological samples corresponding to last class label in the determined class-label order from the test biological samples corresponding to the remaining two class labels (532), wherein the first threshold and the second threshold are determined by:

(i) sorting the *SMS* scores corresponding to each train biological sample in the TSS in an ascending order (532a);

(ii) computing averages of each consecutive pair of *SMS* scores in the sorted list (532b);

(iii) grouping the test biological samples in the TSS into a F group, a M group, and a L group, using a candidate threshold pair comprising of a pair of average scores obtained from all possible pairs of average scores in the sorted list, wherein the F group, the M group, and the L group corresponds to the first, middle and last elements in the previously determined class-label order for a particular TSS, wherein the elements correspond to one of the class A, the class B, and the class C (532c); and

(iv)creating two confusion matrices by comparing the original class labels in step h and the labels as obtained in previous step, wherein (i) values in the first confusion matrix indicate the prediction accuracy of samples corresponding to the first element in the class-label order, wherein the two categories for determining TP, TN, FN, FP values are (F vs !F), wherein F and !F indicates samples falling under (a) the group F, (b) the group M and the group L), and (ii) values in the second confusion matrix indicate the prediction accuracy of samples corresponding to the last element in the class-label order, wherein the two categories for determining TP, TN, FN, FP values are (L vs !L), wherein L and !L indicates samples falling under (c) the group L and, (d) the group F and the group M, and computing a pair of *MCC* (Mathew's correlation coefficient) values ($MCC_1$ and $MCC_2$), are computed, based on the values in the confusion matrices (532d);

(q) computing a first score $S_1$ and a second score $S_2$ using the pair of MCC values using the following formulae:

$$S_1 = |MCC_1| + |MCC_2|$$

$$S_2 = |MCC_1| + |MCC_2| - \big||MCC_1| - |MCC_2|\big|$$

(r) selecting the candidate threshold pair having the maximum $S_2$ value, wherein the threshold values in this pair are used for classifying each test biological sample into one of the three class labels i.e. A or B or C (536);
(s) repeating steps (f) to (r) by considering the complete abundance data as the TSS in order to get the two best thresholds for tag categorization using all the available samples for training (538);
(t) comparing the final prediction score obtained for a new test sample against the two best thresholds for classifying the new test sample to a particular MBD tag category (540); and
(u) determining the MBD tag categories using following criteria:

(i) if final prediction score <= threshold 1, tag the MBD category as low category,
(ii) if threshold 1 < final prediction score < threshold 2, tag the MBD category as medium category, and
(iii) if final prediction score >= threshold 2, tag the MBD category as high category (542).

9. The method (200) as claimed in claim 1, wherein the biological sample is at least one of a stool sample, a gastrointestinal tract (gut) sample, a saliva sample, and a urine sample.

10. The method (200) as claimed in claim 1, wherein the one or more downstream techniques are selected from a list comprising a mammogram, an ultrasound scan, a breast magnetic resonance imaging (MRI) scan, a computed tomography (CT) scan, and a positron emission tomography (PET) scan.

11. The method (200) as claimed in claim 10, wherein

(i) the one of more downstream techniques of the ultrasound scan, the breast MRI scan, the CT scan, or the PET scan are suggested for the subject having the predicted MBD category as the high category; and
(ii) the downstream technique of the mammography is selected for the subject having the predicted MBD category as the low category.

12. The method (200) as claimed in claim 3, wherein the personalized therapeutic recommendation includes utilizing the plurality of predetermined microbes constituting the pre-determined machine learning model to identify one or more antibiotic target candidates and one or more probiotic candidates towards ameliorating the risk of breast cancer, wherein the designing of the one or more antibiotic target candidates is performed by mapping the features constituting the ML model to the complete set of microbes, by:

computing pair-wise correlations between abundances of features constituting the ML model and the abundances corresponding to the complete set of microbial taxa computed individually from (a) the subset of biological samples corresponding to the healthy class and (b) the diseased class, wherein both the samples belonging to the healthy and diseased classes are configured to be used as training data for generating the ML model;
deducing positive and negative interactions between features constituting the ML model and taxa in the healthy and the diseased class of training samples using critical correlation (r) value as the cut-off, such that inter-taxa correlation index values greater than +r value are affiliated as 'positive interactions', while those less than -r value are affiliated as negative interactions;

repeating the previous two steps 1000 times and considering only those interactions relevant that appear in at least 70% of iterations with a BH (Benjamini-Hochberg) corrected p-value cut-off of 0.1 are retained; and

arriving at the relevant therapeutic one or more antibiotic target candidates and one or more probiotic candidates using the retained model taxa interactions and a set of predefined rules.

13. A kit for predicting a category of mammographic breast density (MBD) for a subject, comprising:

an input module for receiving a biological sample of the subject whose category of MBD is to be predicted;

a DNA extraction module to extract microbial Deoxyribonucleic acid (DNA) from the biological sample collected from the subject, using one or more DNA extraction techniques;

an abundance determining module to determine a quantitative abundance of each of a plurality of predetermined microbes associated with the biological sample, from the microbial DNA, using a set of probes specific to each of the plurality of predetermined microbes through a multiplex quantitative Polymerase Chain Reaction (qPCR) technique;

one or more hardware processors configured to analyze the biological sample using the method performed in any of the claim 1 to claim 12; and

an output module for displaying the MBD category for the subject, based on the analysis of the one or more hardware processors.

## Patentansprüche

1. Verfahren (200) zum Vorhersagen einer Kategorie der mammografischen Brustdichte (Mammographic Breast Density, MBD) für ein Subjekt, wobei das Verfahren Folgendes umfasst:

Extrahieren von mikrobieller Desoxyribonukleinsäure (DNS) aus einer von einem Subjekt entnommenen biologischen Probe unter Verwendung einer oder mehrerer DNS-Extraktionstechniken (204);

Bestimmen einer quantitativen Häufigkeit von jeder einer Mehrzahl von vorbestimmten Mikroben, die mit der biologischen Probe assoziiert sind, aus der mikrobiellen DNS unter Verwendung eines Satzes von Sonden, die für jede der Mehrzahl von vorbestimmten Mikroben spezifisch sind, durch eine quantitative Multiplex-Polymerasekettenreaktion (Multiplex Quantitative Polymerase Chain Reaction, qPCR)-Technik, wobei die Mehrzahl von vorbestimmten Mikroben *Murimonas, Clostridium sensu stricto, Clostridium XIVa, Lachnospiracea incertae sedis, Blautia, Bacteroides, Intestinibacter* und *Bilophila* (206) umfasst;

Zusammenstellen, über einen oder mehrere Hardwareprozessoren, der quantitativen Häufigkeit von jeder der Mehrzahl von vorbestimmten Mikroben, um eine mikrobielle Häufigkeitsmatrix (208) zu erhalten;

Bestimmen, über den einen oder die mehreren Hardwareprozessoren, einer Modellbewertung, basierend auf der mikrobiellen Häufigkeitsmatrix, unter Verwendung eines vorbestimmten Maschinenlernmodells (Machine Learning Model, ML-Modell) (210); und

Vorhersagen, über den einen oder die mehreren Hardwareprozessoren, der Kategorie der MBD des Subjekts aus einer von (i) einer hohen Kategorie, (ii) einer mittleren Kategorie und (iii) einer niedrigen Kategorie, basierend auf der Modellbewertung und einem Paar von vordefinierten Schwellenwerten, wobei die vorhergesagte Kategorie dabei hilft, eine oder mehrere nachgeschaltete Techniken zum Beurteilen des Vorhandenseins einer oder mehrerer Brustläsionen in dem Subjekt auszuwählen (212).

2. Verfahren (200) nach Anspruch 1, ferner umfassend das Vorhersagen, über den einen oder die mehreren Hardwareprozessoren, der Kategorie des Brustkrebsrisikos für das Subjekt aus einer von (i) einer gesunden Kategorie und (ii) einer Brustkrebsrisikokategorie, basierend auf der Modellbewertung und einem vordefinierten Schwellenwert (214).

3. Verfahren (200) nach Anspruch 2, ferner umfassend das Entwerfen einer personalisierten therapeutischen Empfehlung für das Subjekt, basierend auf der für das Subjekt vorhergesagten Brustkrebsrisikokategorie, durch Verwenden eines Satzes von Regeln für einen Satz von Mikroben, die das vorbestimmte Maschinenlernmodell bilden, um einen oder mehrere personalisierte probiotische und antibiotische Kandidaten zu identifizieren, die Krankheitssymptome in dem Subjekt lindern, von denen vorhergesagt wird, dass sie die Brustkrebsrisikokategorie aufweisen (216).

4. Verfahren (200) nach Anspruch 1, wobei der Satz von Sonden, die für jede der Mehrzahl von vorbestimmten Mikroben spezifisch sind, in einem ersten Multiplex-qPCR-Lauf, einem zweiten Multiplex-qPCR-Lauf und einem dritten Multiplex-qPCR-Lauf verwendet wird, um die quantitative Häufigkeit von jeder der Mehrzahl von vorbestimmten Mikroben, die mit der biologischen Probe assoziiert sind, zu bestimmen, und wobei:

die Mehrzahl von vorbestimmten Mikroben, deren quantitative Häufigkeit durch den ersten Multiplex-qPCR-Lauf bestimmt wird, Folgendes sind: *Murimonas, Clostridium sensu stricto, Clostridium XIVa* und *Lachnospiracea incertae sedis;*
die Mehrzahl von vorbestimmten Mikroben, deren quantitative Häufigkeit durch den zweiten Multiplex-qPCR-Lauf bestimmt wird, Folgendes sind: *Murimonas, Clostridium sensu stricto, Blautia* und *Bacteroides*; und
die Mehrzahl von vorbestimmten Mikroben, deren quantitative Häufigkeit durch den dritten Multiplex-qPCR-Lauf bestimmt wird, Folgendes sind: *Clostridium sensu stricto, Clostridium XIVa, Intestinibacter* und *Bilophila.*

5.  Verfahren (200) nach Anspruch 1, wobei das vorbestimmte Maschinenlernmodell (ML-Modell) ein Ensemble-ML-Modell ist, das unter Verwendung von mikrobiellen Häufigkeitsdaten, die einer Mehrzahl von biologischen Trainingsproben entsprechen, erstellt wurde.

6.  Verfahren (200) nach Anspruch 1, wobei die Mehrzahl von vorbestimmten Mikroben, die mit der biologischen Probe assoziiert sind, Merkmale des vorbestimmten Maschinenlernmodells (ML-Modells) sind.

7.  Verfahren (200) nach Anspruch 4, wobei ein oder mehrere vorbestimmte Mikroben aus der Mehrzahl von vorbestimmten Mikroben, die mit der biologischen Probe assoziiert sind, einem oder mehreren des ersten Multiplex-qPCR-Laufs, des zweiten Multiplex-qPCR-Laufs und des dritten Multiplex-qPCR-Laufs gemeinsam sind, um die quantitative Häufigkeit zu bestimmen, und wobei das eine oder die mehreren vorbestimmten Mikroben, die einem oder mehreren des ersten Multiplex-qPCR-Laufs, des zweiten Multiplex-qPCR-Laufs und des dritten Multiplex-qPCR-Laufs gemeinsam sind, basierend auf (i) einer mittleren Häufigkeit von jeder der Mehrzahl von vorbestimmten Mikroben, die aus der Mehrzahl von biologischen Trainingsproben erhalten wurden, (ii) einer Häufigkeit des Auftretens von jeder der Mehrzahl von vorbestimmten Mikroben, die das Ensemble-ML-Modell bilden, bestimmt werden.

8.  Verfahren (200) nach Anspruch 1, wobei das Paar von vordefinierten Schwellenwerten erhalten wird durch:

(a) Sammeln einer Mehrzahl von biologischen Proben aus einer Kohorte, die aus einer Mehrzahl von Subjekten besteht, wobei jedes der Mehrzahl von Subjekten in der Kohorte zu einer der MBD-Kategorien aus (i) der hohen Kategorie, (ii) der mittleren Kategorie und (iii) der niedrigen Kategorie gehört (502);
(b) Extrahieren der mikrobiellen Desoxyribonukleinsäure (DNS) aus jeder der Mehrzahl von biologischen Proben unter Verwendung der einen oder der mehreren DNS-Extraktionstechniken (504);
(c) Sequenzieren der mikrobiellen DNS unter Verwendung einer oder mehrerer Sequenzierungstechniken, um Sequenzdaten zu erzeugen, die jeder der Mehrzahl von biologischen Proben entsprechen (506);
(d) Erzeugen eines mikrobiellen Häufigkeitsprofils, das jeder biologischen Probe entspricht, basierend auf den entsprechenden Sequenzdaten unter Verwendung einer oder mehrerer Rechenmethoden (508);
(e) Bilden von Trainingsdaten (TR), die eine Mehrzahl von mikrobiellen Häufigkeitsprofilen der Mehrzahl von biologischen Proben umfassen, durch Zusammenstellen jedes mikrobiellen Häufigkeitsprofils, das jeder der Mehrzahl von biologischen Proben entspricht (510);
(f) Aufteilen der Trainingsdaten (TR) zufällig in einen Trainingssatz (TRS) und einen Testsatz (TSS) basierend auf einem vordefinierten Aufteilungsparameter (S), wobei S % der Proben aus den Trainingsdaten den TRS-Satz bilden und (100 - S) % der Proben den TSS-Satz bilden, wobei der Trainingssatz (TRS) eine Mehrzahl von biologischen Trainingsproben umfasst und der Testsatz (TSS) eine Mehrzahl von biologischen Testproben umfasst, und wobei ein Stratified-Sampling-Ansatz angewendet wird, während die TR in den TRS und den TSS aufgeteilt wird, mit der Absicht, einen ursprünglichen relativen Anteil von Proben, die zu einer Klasse A, einer Klasse B und einer Klasse C gehören, in dem TRS und dem TSS zu bewahren, wobei die Klasse A der niedrigen Kategorie von MBD entspricht, die Klasse B der mittleren Kategorie von MBD entspricht und die Klasse C der hohen Kategorie von MBD entspricht (512);
(g) Erzeugen einer Mehrzahl von Trainingsteilsätzen aus dem TRS und einer Mehrzahl von Testteilsätzen aus dem TSS unter Verwendung von wiederholtem Zufallssampling (514);
(h) Neumarkieren jeder biologischen Trainingsprobe, die in der Mehrzahl von Trainingsteilsätzen vorhanden ist, die (i) der Klasse A und der Klasse B als eine Klasse X und (ii) der Klasse C als eine Klasse Y entspricht (516);
(i) Erzeugen einer Mehrzahl von zweiteiligen Klassifizierungsmodellen, wobei jedes zweiteilige Klassifizierungsmodell unter Verwendung der biologischen Trainingsproben, die in jedem Trainingsteilsatz vorhanden sind, und entsprechender Neumarkierung erzeugt wird (518);
(j) Klassifizieren jeder biologischen Testprobe, die in jedem Testteilsatz in dem TSS vorhanden ist, unter Verwendung des entsprechenden zweiteiligen Klassifizierungsmodells, wobei die Klassifizierung jeder biologischen Testprobe (i) eine der Klasse X und der Klasse Y zuweist und (ii) eine skalierte Modellbewertung (Scaled Model Score, *SMS*) für jede biologische Testprobe erzeugt (520);

(k) Zuordnen und Neumarkieren jeder biologischen Testprobe, die in jedem Testteilsatz in dem TSS vorhanden ist, zu einer der Klasse A, der Klasse B und der Klasse C (522);

(l) zufälliges Zeichnen einer vordefinierten Anzahl von biologischen Testproben aus jedem Testteilsatz der Mehrzahl von Testteilsätzen und Zuweisen eines Indexwerts in einer aufsteigenden Reihenfolge beginnend mit 1 basierend auf den entsprechenden *SMS* Bewertungen (524);

(m)Berechnen eines Medians der Indexwerte, die jedem Testteilsatz in dem TSS entsprechen, der zu individuellen Klassenmarkierungen A, B und C gehört, wobei die Klassenmarkierungen, die der sortierten Medianwertliste entsprechen, die Klassenmarkierungsreihenfolge für diesen TSS anzeigen, wenn die berechneten Mediane in aufsteigender Reihenfolge sortiert sind (526);

(n) Wiederholen des Schritts (m) für eine vordefinierte Anzahl von Malen mit der vordefinierten Anzahl von biologischen Testproben aus jedem Testteilsatz, um eine vordefinierte Anzahl von Klassenmarkierungsreihenfolgen für jeden Testteilsatz in dem TSS zu erhalten (528);

(o) Fertigstellen der Klassenmarkierungsreihenfolge, die die maximale Anzahl von Malen auftritt, als die Klassenmarkierungsreihenfolge für jeden Testteilsatz in dem TSS (530);

(p) Bestimmen eines ersten Schwellenwerts und eines zweiten Schwellenwerts zwischen -1 und +1, die konfiguriert sind, um die biologischen Testproben basierend auf entsprechenden *SMS* Bewertungen in dem TSS in drei Gruppen zu unterteilen, wobei der erste Schwellenwert die biologischen Testproben, die der ersten Klassenmarkierung in der bestimmten Klassenmarkierungsreihenfolge entsprechen, von den biologischen Testproben, die den verbleibenden zwei Klassenmarkierungen entsprechen, abgrenzt und der zweite Schwellenwert die biologischen Testproben, die der letzten Klassenmarkierung in der bestimmten Klassenmarkierungsreihenfolge entsprechen, von den biologischen Testproben, die den verbleibenden zwei Klassenmarkierungen entsprechen, abgrenzt (532), wobei der erste Schwellenwert und der zweite Schwellenwert bestimmt werden durch:

(i) Sortieren der *SMS* Bewertungen, die jeder biologischen Zugprobe in dem TSS entsprechen, in einer aufsteigenden Reihenfolge (532a);

(ii) Berechnen von Mittelwerten jedes aufeinanderfolgenden Paars von *SMS* Bewertungen in der sortierten Liste (532b);

(iii)Gruppieren der biologischen Testproben in dem TSS in eine F-Gruppe, eine M-Gruppe und eine L-Gruppe unter Verwendung eines Kandidatenschwellenwertpaars, das ein Paar von Durchschnittsbewertungen umfasst, die aus allen möglichen Paaren von Durchschnittsbewertungen in der sortierten Liste erhalten werden, wobei die F-Gruppe, die M-Gruppe und die L-Gruppe den ersten, mittleren und letzten Elementen in der zuvor bestimmten Klassenmarkierungsreihenfolge für ein bestimmtes TSS entsprechen, wobei die Elemente einer der Klasse A, der Klasse B und der Klasse C entsprechen (532c); und

(iv)Erzeugen von zwei Konfusionsmatrizen durch Vergleichen der ursprünglichen Klassenmarkierungen in Schritt h und der Markierungen, wie sie im vorherigen Schritt erhalten wurden, wobei (i) Werte in der ersten Konfusionsmatrix die Vorhersagegenauigkeit von Proben angeben, die dem ersten Element in der Klassenmarkierungsreihenfolge entsprechen, wobei die zwei Kategorien zum Bestimmen von TP-, TN-, FN-, FP-Werten (F vs !F) sind, wobei F und !F Proben angeben, die unter (a) die Gruppe F, (b) die Gruppe M und die Gruppe L fallen, und (ii) Werte in der zweiten Konfusionsmatrix die Vorhersagegenauigkeit von Proben angeben, die dem letzten Element in der Klassenmarkierungsreihenfolge entsprechen, wobei die zwei Kategorien zum Bestimmen von TP-, TN-, FN-, FP-Werten (L vs !L) sind, wobei L und !L Proben angeben, die unter (c) die Gruppe L fallen, und (d) die Gruppe F und die Gruppe M, und Berechnen eines Paars von *MCC* (Mathews Korrelationskoeffizienten) Werten ($MCC_1$ und $MCC_2$), die basierend auf den Werten in den Konfusionsmatrizen berechnet werden (532d);

(q) Berechnen einer ersten Bewertung $S_1$ und einer zweiten Bewertung $S_2$ unter Verwendung des Paars von MCC-Werten unter Verwendung der folgenden Formeln:

$$S_1 = |MCC_1| + |MCC_2|$$

$$S_2 = |MCC_1| + |MCC_2| - ||MCC_1| - |MCC_2||$$

(r) Auswählen des Kandidatenschwellenwertpaars mit dem maximalen $S_2$ Wert, wobei die Schwellenwerte in diesem Paar zum Klassifizieren jeder biologischen Testprobe in eine der drei Klassenmarkierungen, d. h. A oder B oder C, verwendet werden (536);

(s) Wiederholen der Schritte (f) bis (r) durch Berücksichtigen der vollständigen Häufigkeitsdaten als das TSS, um die zwei besten Schwellenwerte für die Markierungskategorisierung unter Verwendung aller verfügbaren Proben für das Training zu erhalten (538);

(t) Vergleichen der endgültigen Vorhersagebewertung, die für eine neue Testprobe erhalten wird, mit den zwei besten Schwellenwerten zum Klassifizieren der neuen Testprobe in eine bestimmte MBD-Markierungskategorie (540); und

(u) Bestimmen der MBD-Markierungskategorien unter Verwendung der folgenden Kriterien:

(i) wenn die endgültige Vorhersagebewertung <= Schwellenwert 1 ist, Markieren der MBD-Kategorie als niedrige Kategorie,

(ii) wenn Schwellenwert 1 < endgültige Vorhersagebewertung < Schwellenwert 2 ist, Markieren der MBD-Kategorie als mittlere Kategorie, und

(iii) wenn die endgültige Vorhersagebewertung >= Schwellenwert 2 ist, Markieren der MBD-Kategorie als hohe Kategorie (542).

9. Verfahren (200) nach Anspruch 1, wobei die biologische Probe mindestens eine von einer Stuhlprobe, einer Probe des Magen-Darm-Trakts (Darm), einer Speichelprobe und einer Urinprobe ist.

10. Verfahren (200) nach Anspruch 1, wobei die eine oder die mehreren nachgeschalteten Techniken aus einer Liste ausgewählt sind, die ein Mammogramm, einen Ultraschallscan, einen Magnetresonanztomographie-Scan (MRT-Scan) der Brust, einen Computertomographie-Scan (CT-Scan) und einen Positronenemissionstomographie-Scan (PET-Scan) umfasst.

11. Verfahren (200) nach Anspruch 10, wobei

(i) die eine oder die mehreren nachgeschalteten Techniken des Ultraschallscans, des Brust-MRT-Scans, des CT-Scans oder des PET-Scans für das Subjekt vorgeschlagen werden, das die vorhergesagte MBD-Kategorie als die hohe Kategorie aufweist; und

(ii) die nachgeschaltete Technik der Mammographie für das Subjekt ausgewählt wird, das die vorhergesagte MBD-Kategorie als die niedrige Kategorie aufweist.

12. Verfahren (200) nach Anspruch 3, wobei die personalisierte therapeutische Empfehlung das Verwenden der Mehrzahl von vorbestimmten Mikroben beinhaltet, die das vorbestimmte Maschinenlernmodell bilden, um einen oder mehrere antibiotische Zielkandidaten und einen oder mehrere probiotische Kandidaten zum Lindern des Risikos von Brustkrebs zu identifizieren, wobei das Entwerfen des einen oder der mehreren antibiotischen Zielkandidaten durch Abbilden der Merkmale, die das ML-Modell bilden, auf den vollständigen Satz von Mikroben durchgeführt wird, durch:

Berechnen von paarweisen Korrelationen zwischen Häufigkeiten von Merkmalen, die das ML-Modell bilden, und den Häufigkeiten, die dem vollständigen Satz von mikrobiellen Taxa entsprechen, die individuell aus (a) der Teilmenge von biologischen Proben, die der gesunden Klasse entsprechen, und (b) der erkrankten Klasse berechnet werden, wobei beide Proben, die zu der gesunden und der erkrankten Klasse gehören, konfiguriert sind, um als Trainingsdaten zum Erzeugen des ML-Modells verwendet zu werden;

Ableiten von positiven und negativen Wechselwirkungen zwischen Merkmalen, die das ML-Modell bilden, und Taxa in der gesunden und der erkrankten Klasse von Trainingsproben unter Verwendung des kritischen Korrelationswerts (r) als den Cut-off, so dass Intertaxakorrelationsindexwerte, die größer als der +r-Wert sind, als "positive Wechselwirkungen" verbunden sind, während diejenigen, die kleiner als der - r-Wert sind, als negative Wechselwirkungen verbunden sind;

Wiederholen der vorhergehenden zwei Schritte 1000 Mal und Berücksichtigen nur derjenigen relevanten Wechselwirkungen, die in mindestens 70% der Iterationen mit einem BH-(Benjamini-Hochberg)-korrigierten p-Wert-Cut-off von 0,1 erscheinen, beibehalten werden; und

Erreichen des einen oder der mehreren relevanten therapeutischen antibiotischen Zielkandidaten und des einen oder der mehreren probiotischen Kandidaten unter Verwendung der beibehaltenen Modelltaxawechselwirkungen und eines Satzes von vordefinierten Regeln.

13. Kit zum Vorhersagen einer Kategorie der mammografischen Brustdichte (Mammographic Breast Density, MBD) für ein Subjekt, das Folgendes umfasst:

ein Eingabemodul zum Empfangen einer biologischen Probe des Subjekts, deren MBD-Kategorie vorhergesagt werden soll;

ein DNS-Extraktionsmodul zum Extrahieren von mikrobieller Desoxyribonukleinsäure (DNS) aus der von dem Subjekt entnommenen biologischen Probe unter Verwendung einer oder mehrerer DNS-Extraktionstechniken;

ein Häufigkeitsbestimmungsmodul zum Bestimmen einer quantitativen Häufigkeit von jeder einer Mehrzahl von vorbestimmten Mikroben, die mit der biologischen Probe assoziiert sind, aus der mikrobiellen DNS unter Verwendung eines Satzes von Sonden, die für jede der Mehrzahl von vorbestimmten Mikroben spezifisch sind, durch eine quantitative Multiplex-Polymerasekettenreaktion (Multiplex Quantitative Polymerase Chain Reaction, qPCR)-Technik;

einen oder mehrere Hardwareprozessoren, die konfiguriert sind, um die biologische Probe unter Verwendung des Verfahrens zu analysieren, das in einem der Ansprüche 1 bis 12 durchgeführt wird; und

ein Ausgabemodul zum Anzeigen der MBD-Kategorie für das Subjekt, basierend auf der Analyse des einen oder der mehreren Hardwareprozessoren.

**Revendications**

1. Procédé (200) de prédiction d'une catégorie de densité mammographique (MBD) pour un sujet, le procédé comprenant :

l'extraction d'acide désoxyribonucléique (ADN) microbien à partir d'un échantillon biologique prélevé chez un sujet, en utilisant une ou plusieurs techniques d'extraction d'ADN (204) ;

la détermination d'une abondance quantitative de chacun d'une pluralité de microbes prédéterminés associés à l'échantillon biologique, à partir de l'ADN microbien, en utilisant un ensemble de sondes spécifiques à chacun de la pluralité de microbes prédéterminés par l'intermédiaire d'une technique de réaction en chaîne par polymérase quantitative multiplex (qPCR), dans lequel la pluralité de microbes prédéterminés comprend *Murimonas, Clostridium sensu stricto, Clostridium XIVa, Lachnospiracea incertae sedis, Blautia, Bacteroides, Intestinibacter*, et *Bilophila* (206) ;

la compilation, par l'intermédiaire d'un ou plusieurs processeurs matériels, de l'abondance quantitative de chacun de la pluralité de microbes prédéterminés, pour obtenir une matrice d'abondance microbienne (208) ;

la détermination, par l'intermédiaire des un ou plusieurs processeurs matériels, d'un score de modèle, sur la base de la matrice d'abondance microbienne, en utilisant un modèle d'apprentissage machine (ML) prédéterminé (210) ; et

la prédiction, par l'intermédiaire des un ou plusieurs processeurs matériels, de la catégorie de MBD du sujet, à partir de l'une parmi (i) une catégorie élevée, (ii) une catégorie moyenne, et (iii) une catégorie basse, sur la base du score de modèle et d'une paire de valeurs de seuil prédéfinies, dans lequel la catégorie prédite aide à choisir une ou plusieurs techniques en aval pour évaluer la présence d'une ou plusieurs lésions mammaires chez le sujet (212).

2. Procédé (200) selon la revendication 1, comprenant en outre la prédiction, par l'intermédiaire des un ou plusieurs processeurs matériels, de la catégorie de risque de cancer du sein pour le sujet, à partir de l'une parmi (i) une catégorie saine, et (ii) une catégorie de risque de cancer du sein, sur la base du score de modèle et d'une valeur de seuil prédéfinie (214).

3. Procédé (200) selon la revendication 2, comprenant en outre la conception d'une recommandation thérapeutique personnalisée pour le sujet, sur la base de la catégorie de risque de cancer du sein prédite pour le sujet, en utilisant un ensemble de règles pour un ensemble de microbes qui constituent le modèle d'apprentissage machine prédéterminé pour identifier un ou plusieurs candidats probiotiques et antibiotiques personnalisés qui améliorent les symptômes de maladie chez le sujet prédit comme ayant la catégorie de risque de cancer du sein (216).

4. Procédé (200) selon la revendication 1, dans lequel l'ensemble de sondes spécifiques à chacun de la pluralité de microbes prédéterminés est utilisé dans un premier cycle de qPCR multiplex, un deuxième cycle de qPCR multiplex, et un troisième cycle de qPCR multiplex pour déterminer l'abondance quantitative de chacun de la pluralité de microbes prédéterminés associés à l'échantillon biologique, et dans lequel :

la pluralité de microbes prédéterminés, dont l'abondance quantitative est déterminée par l'intermédiaire du premier cycle de qPCR multiplex sont : *Murimonas, Clostridium sensu stricto, Clostridium XIVa,* et *Lachnospiracea incertae sedis* ;

la pluralité de microbes prédéterminés, dont l'abondance quantitative est déterminée par l'intermédiaire du deuxième cycle de qPCR multiplex sont : *Murimonas, Clostridium sensu stricto, Blautia,* et *Bacteroides* ; et la pluralité de microbes prédéterminés, dont l'abondance quantitative est déterminée par l'intermédiaire du troisième cycle de qPCR multiplex sont : *Clostridium sensu stricto, Clostridium XIVa, Intestinibacter,* et *Bilophila.*

5. Procédé (200) selon la revendication 1, dans lequel le modèle d'apprentissage machine (ML) prédéterminé est un modèle ML d'ensemble construit en utilisant des données d'abondance microbienne correspondant à une pluralité d'échantillons biologiques d'apprentissage.

6. Procédé (200) selon la revendication 1, dans lequel la pluralité de microbes prédéterminés associés à l'échantillon biologique sont des caractéristiques du modèle d'apprentissage machine (ML) prédéterminé.

7. Procédé (200) selon la revendication 4, dans lequel un ou plusieurs microbes prédéterminés parmi la pluralité de microbes prédéterminés associés à l'échantillon biologique, sont communs à un ou plusieurs parmi le premier cycle de qPCR multiplex, le deuxième cycle de qPCR multiplex, et le troisième cycle de qPCR multiplex, pour déterminer l'abondance quantitative, et dans lequel les un ou plusieurs microbes prédéterminés qui sont communs à un ou plusieurs parmi le premier cycle de qPCR multiplex, le deuxième cycle de qPCR multiplex, et le troisième cycle de qPCR multiplex sont déterminés sur la base (i) d'une abondance médiane de chacun de la pluralité de microbes prédéterminés obtenus à partir de la pluralité d'échantillons biologiques d'apprentissage, (ii) d'une fréquence d'occurrence de chacun de la pluralité de microbes prédéterminés constituant le modèle ML d'ensemble.

8. Procédé (200) selon la revendication 1, dans lequel la paire de valeurs de seuil prédéfinies est obtenue par :

(a) le prélèvement d'une pluralité d'échantillons biologiques à partir d'une cohorte composée d'une pluralité de sujets, dans lequel chacun de la pluralité de sujets dans la cohorte appartient à l'une des catégories de MBD parmi (i) la catégorie élevée, (ii) la catégorie moyenne, et (iii) la catégorie basse (502) ;
(b) l'extraction de l'acide désoxyribonucléique (ADN) microbien à partir de chacun de la pluralité d'échantillons biologiques, en utilisant les une ou plusieurs techniques d'extraction d'ADN (504) ;
(c) le séquençage de l'ADN microbien, en utilisant une ou plusieurs techniques de séquençage, pour générer des données de séquence correspondant à chacun de la pluralité d'échantillons biologiques (506) ;
(d) la génération d'un profil d'abondance microbienne correspondant à chaque échantillon biologique, sur la base des données de séquence correspondantes, en utilisant une ou plusieurs méthodologies de calcul (508) ;
(e) la formation de données d'apprentissage (TR) comprenant une pluralité de profils d'abondance microbienne de la pluralité d'échantillons biologiques, en compilant chaque profil d'abondance microbienne correspondant à chacun de la pluralité d'échantillons biologiques (510) ;
(f) le partitionnement des données d'apprentissage (TR) de manière aléatoire en un ensemble d'apprentissage (TRS) et un ensemble de tests (TSS), sur la base d'un paramètre de division prédéfini (S), dans lequel *S* % d'échantillons à partir des données d'apprentissage constituent l'ensemble TRS et (100 - S) % des échantillons constituent l'ensemble TSS, dans lequel l'ensemble d'apprentissage (TRS) comprend une pluralité d'échantillons biologiques d'apprentissage et l'ensemble de tests (TSS) comprend une pluralité d'échantillons biologiques de tests, et dans lequel une approche d'échantillonnage stratifiée est adoptée tout en partitionnant le TR, en le TRS et le TSS, avec une intention de préserver une proportion relative d'origine d'échantillons appartenant à une classe A, une classe B et une classe C, dans le TRS et le TSS, dans lequel la classe A correspond à la catégorie basse de MBD, la classe B correspond à la catégorie moyenne de MBD et la classe C correspond à la catégorie élevée de MBD (512) ;
(g) la génération d'une pluralité de sous-ensembles d'apprentissage à partir du TRS et d'une pluralité de sous-ensembles de tests à partir du TSS, en utilisant un échantillonnage aléatoire répété (514) ;
(h) le réétiquetage de chaque échantillon biologique de train présent dans la pluralité de sous-ensembles d'apprentissage correspondant à (i) la classe A et la classe B en tant que classe X, et (ii) la classe C en tant que classe Y (516) ;
(i) la génération d'une pluralité de modèles de classification bipartite, dans lequel chaque modèle de classification bipartite est généré en utilisant les échantillons biologiques d'apprentissage présents dans chaque sous-ensemble d'apprentissage et le réétiquetage correspondant (518) ;
(j) la classification de chaque échantillon biologique de test présent dans chaque sous-ensemble de tests dans le TSS, en utilisant le modèle de classification bipartite correspondant, dans lequel la classification attribue à chaque échantillon biologique de test (i) l'une parmi la classe X et la classe Y, et (ii) génère un score de modèle mis à l'échelle *(SMS)* pour chaque échantillon biologique de test (520) ;
(k) le mappage et le réétiquetage de chaque échantillon biologique de test présent dans chaque sous-ensemble de tests dans le TSS avec l'une parmi la classe A, la classe B et la classe C (522) ;

(I) le tirage aléatoire d'un nombre prédéfini d'échantillons biologiques de test, à partir de chaque sous-ensemble de tests de la pluralité de sous-ensembles de tests, et l'attribution d'une valeur d'indice dans un ordre croissant commençant à partir de 1, sur la base des scores *SMS* correspondants (524) ;

(m) le calcul d'une médiane des valeurs d'indice correspondant à chaque sous-ensemble de tests dans le TSS appartenant à des étiquettes de classe individuelles A, B, et C, dans lequel les étiquettes de classe correspondant à la liste de valeurs médianes triées indiquent l'ordre d'étiquette de classe pour ce TSS, si les médianes calculées sont triées dans l'ordre croissant (526) ;

(n) l'itération de l'étape (m), pour un nombre prédéfini de fois, avec le nombre prédéfini d'échantillons biologiques de test à partir de chaque sous-ensemble de tests, pour obtenir un nombre prédéfini d'ordres d'étiquette de classe pour chaque sous-ensemble de tests dans le TSS (528) ;

(o) la finalisation de l'ordre d'étiquette de classe survenant le nombre maximal de fois en tant qu'ordre d'étiquette de classe pour chaque sous-ensemble de tests dans le TSS (530) ;

(p) la détermination d'un premier seuil et d'un second seuil entre - 1 et +1, configurés pour diviser les échantillons biologiques de test sur la base de scores *SMS* correspondants dans le TSS en trois groupes, dans lequel le premier seuil démarque les échantillons biologiques de test correspondant à la première étiquette de classe dans l'ordre d'étiquette de classe déterminé à partir des échantillons biologiques de test correspondant aux deux étiquettes de classe restantes, et le second seuil démarque les échantillons biologiques de test correspondant à la dernière étiquette de classe dans l'ordre d'étiquette de classe déterminé à partir des échantillons biologiques de test correspondant aux deux étiquettes de classe restantes (532), dans lequel le premier seuil et le second seuil sont déterminés par :

> (i) le tri des scores *SMS* correspondant à chaque échantillon biologique d'apprentissage dans le TSS dans un ordre croissant (532a) ;
> (ii) le calcul de moyennes de chaque paire consécutive de scores *SMS* dans la liste triée (532b) ;
> (iii) le regroupement des échantillons biologiques de test dans le TSS en un groupe F, un groupe M et un groupe L, à l'aide d'une paire de seuils candidats comprenant une paire de scores moyens obtenus à partir de toutes les paires possibles de scores moyens dans la liste triée, dans lequel le groupe F, le groupe M et le groupe L correspondent aux éléments de début, de milieu et de fin dans l'ordre d'étiquette de classe déterminé précédemment pour un TSS particulier, dans lequel les éléments correspondent à l'une de la classe A, de la classe B et de la classe C (532c) ; et
> (iv) la création de deux matrices de confusion en comparant les étiquettes de classe d'origine à l'étape h et les étiquettes telles qu'obtenues à l'étape précédente, dans lequel (i) des valeurs dans la première matrice de confusion indiquent la précision de prédiction d'échantillons correspondant au premier élément dans l'ordre d'étiquette de classe, dans lequel les deux catégories pour déterminer des valeurs de TP, TN, FN, FP sont (F vs !F), dans lequel F et !F indiquent des échantillons tombant sous (a) le groupe F, (b) le groupe M et le groupe L), et (ii) des valeurs dans la seconde matrice de confusion indiquent la précision de prédiction d'échantillons correspondant au dernier élément dans l'ordre d'étiquette de classe, dans lequel les deux catégories pour déterminer des valeurs de TP, TN, FN, FP sont (L vs !L), dans lequel L et !L indiquent des échantillons tombant sous (c) le groupe L et, (d) le groupe F et le groupe M, et le calcul d'une paire de valeurs *MCC* (coefficient de corrélation de Matthews) ($MCC_1$ et $MCC_2$), sont calculés, sur la base des valeurs dans les matrices de confusion (532d) ;

(q) le calcul d'un premier score $S_1$ et d'un second score $S_2$ en utilisant la paire de valeurs de MCC en utilisant les formules suivantes :

$$S_1 = |MCC_1| + |MCC_2|$$

$$S_2 = |MCC_1| + |MCC_2| - \left| |MCC_1| - |MCC_2| \right|$$

(r) la sélection de la paire de seuils candidats ayant la valeur maximale $S_2$, dans lequel les valeurs de seuil dans cette paire sont utilisées pour classer chaque échantillon biologique de test dans l'une des trois étiquettes de classe, c'est-à-dire A ou B ou C (536) ;

(s) la répétition des étapes (f) à (r) en considérant les données d'abondance complètes comme le TSS afin d'obtenir les deux meilleurs seuils pour la catégorisation d'étiquette en utilisant tous les échantillons disponibles pour l'apprentissage (538) ;

(t) la comparaison du score de prédiction final obtenu pour un nouvel échantillon de test par rapport aux deux meilleurs seuils pour classer le nouvel échantillon de test dans une catégorie d'étiquette MBD particulière (540) ; et

(u) la détermination des catégories d'étiquette MBD en utilisant les critères suivants :

(i) si le score de prédiction final <= seuil 1, marquer la catégorie MBD comme catégorie basse,
(ii) si le seuil 1 < score de prédiction final < seuil 2, marquer la catégorie MBD comme catégorie moyenne, et
(iii) si le score de prédiction final >= seuil 2, marquer la catégorie MBD comme catégorie élevée (542).

9. Procédé (200) selon la revendication 1, dans lequel l'échantillon biologique est au moins l'un parmi un échantillon de selles, un échantillon de tractus gastro-intestinal (intestin), un échantillon de salive, et un échantillon d'urine.

10. Procédé (200) selon la revendication 1, dans lequel les une ou plusieurs techniques en aval sont sélectionnées dans une liste comprenant une mammographie, un balayage ultrasonore, un balayage d'imagerie par résonance magnétique (IRM) mammaire, un balayage de tomodensitométrie (TDM), et un balayage de tomographie par émission de positons (TEP).

11. Procédé (200) selon la revendication 10, dans lequel

(i) les une ou plusieurs techniques en aval du balayage ultrasonore, du balayage d'IRM mammaire, du balayage TDM, ou du balayage TEP sont suggérées pour le sujet ayant la catégorie MBD prédite comme catégorie élevée ; et
(ii) la technique en aval de la mammographie est sélectionnée pour le sujet ayant la catégorie MBD prédite comme catégorie basse.

12. Procédé (200) selon la revendication 3, dans lequel la recommandation thérapeutique personnalisée inclut l'utilisation de la pluralité de microbes prédéterminés constituant le modèle d'apprentissage machine prédéterminé pour identifier un ou plusieurs candidats cibles antibiotiques et un ou plusieurs candidats probiotiques pour améliorer le risque de cancer du sein, dans lequel la conception des un ou plusieurs candidats cibles antibiotiques est effectuée en mappant les caractéristiques constituant le modèle ML à l'ensemble complet de microbes, par :

le calcul de corrélations par paires entre des abondances de caractéristiques constituant le modèle ML et les abondances correspondant à l'ensemble complet de taxons microbiens calculés individuellement à partir (a) du sous-ensemble d'échantillons biologiques correspondant à la classe saine et (b) à la classe malade, dans lequel les deux échantillons appartenant aux classes saine et malade sont configurés pour être utilisés comme données d'apprentissage pour générer le modèle ML ;
la déduction d'interactions positives et négatives entre des caractéristiques constituant le modèle ML et des taxons dans la classe saine et la classe malade d'échantillons d'apprentissage en utilisant une valeur de corrélation critique (r) comme seuil, de sorte que des valeurs d'indice de corrélation inter-taxons supérieures à la valeur +r sont affiliées en tant qu'« interactions positives », tandis que celles inférieures à la valeur -r sont affiliées en tant qu'interactions négatives ;
la répétition des deux étapes précédentes 1000 fois et la considération que seules les interactions pertinentes qui apparaissent dans au moins 70 % d'itérations avec un seuil de valeur p corrigé BH (Benjamini-Hochberg) de 0,1 sont retenues ; et
l'arrivée aux un ou plusieurs candidats cibles antibiotiques et un ou plusieurs candidats probiotiques thérapeutiques pertinents en utilisant les interactions de taxons de modèle retenues et un ensemble de règles prédéfinies.

13. Kit de prédiction d'une catégorie de densité mammographique (MBD) pour un sujet, comprenant :

un module d'entrée pour recevoir un échantillon biologique du sujet dont la catégorie de MBD doit être prédite ;
un module d'extraction d'ADN pour extraire de l'acide désoxyribonucléique (ADN) microbien à partir de l'échantillon biologique prélevé chez le sujet, en utilisant une ou plusieurs techniques d'extraction d'ADN ;
un module de détermination d'abondance pour déterminer une abondance quantitative de chacun d'une pluralité de microbes prédéterminés associés à l'échantillon biologique, à partir de l'ADN microbien, en utilisant un ensemble de sondes spécifiques à chacun de la pluralité de microbes prédéterminés par l'intermédiaire d'une technique de réaction en chaîne par polymérase quantitative multiplex (qPCR) ;
un ou plusieurs processeurs matériels configurés pour analyser l'échantillon biologique en utilisant le procédé effectué dans l'une quelconque de la revendication 1 à la revendication 12 ; et

un module de sortie pour afficher la catégorie de MBD pour le sujet, sur la base de l'analyse des un ou plusieurs processeurs matériels.

FIG. 1

200

Collect a biological sample from a subject **202**

Extract microbial Deoxyribonucleic Acid (DNA) from the biological sample, using one or more DNA extraction techniques **204**

Determine a quantitative abundance of each of a plurality of predetermined microbes associated with the biological sample, from the microbial DNA, using a set of probes specific to each of the plurality of predetermined microbes through a multiplex quantitative Polymerase Chain Reaction (qPCR) technique **206**

Collate the quantitative abundance of each of the plurality of predetermined microbes, to obtain a microbial abundance matrix **208**

Determine a model score, based on the microbial abundance matrix, using a pre-determined machine learning (ML) model **210**

A

FIG. 2A

200

(A)

Predict the category of MBD of the subject, from one of (i) a high category, (ii) a medium category, and (iii) a low category, based on the model score and a pair of predefined threshold values, wherein the predicted category helps in choosing one or more downstream techniques for assessing the presence of one or more breast lesions in the subject **212**

Predict the category of breast cancer risk for the subject, from one of (i) a healthy category, and (ii) a breast cancer risk category, based on the model score and a predefined threshold value **214**

Design a personalized therapeutic recommendation for the subject, based on the breast cancer risk category predicted for the subject, by utilizing a set of rules for a set of microbes that constitute the pre-determined machine learning model to identify one or more personalized probiotic and antibiotic candidates that ameliorate disease symptoms in the subject predicted as having the breast cancer risk category **216**

FIG. 2B

Run 1

| Z | Murimonas | Clostridium sensu stricto | Clostridium XIVa | Lachnospiracea incertae sedis |

Run 2

| Z | Murimonas | Clostridium sensu stricto | Blautia | Bacteroides |

Run 3

| Z | Clostridium sensu stricto | Clostridium XIVa | Intestinibacter | Bilophila |

| Z | Universal Non-specific Probe |

FIG. 3

400

Assign one of a healthy class tag or an unhealthy class tag to each of the samples in the collected plurality of training biological samples **402**

Generate training data comprising of a plurality of microbial abundance profiles corresponding to each of the plurality of training biological samples, wherein each microbial abundance profile corresponding to a training biological sample comprises of one or a plurality of features and respective abundance values of the features, and wherein each feature in the microbial abundance profile corresponds to one of a plurality of microbial taxonomic groups present in the training biological sample **404**

Partition the training data into an internal training set and an internal test set **406**

Randomly select a predefined number of subsets out of the internal training set, wherein each subset comprises of a randomly selected plurality of microbial abundance profiles corresponding to the training biological samples in the randomly selected subset, and wherein each subset comprises a proportionate part of samples belonging to the healthy class and the remaining samples belonging to the unhealthy class **408**

Note for each selected subset, a distribution of the abundance values of each of the features across the plurality of training biological samples in the selected subset, and the distribution of the abundance values of each of the features across the training biological samples belonging to the healthy class in the selected subset and the training biological samples belonging to the unhealthy class in the selected subset **410**

Calculate from the noted distributions of each selected subset, a first quartile value (Q1) and a third quartile value (Q3) of the distribution of each of the features across each of the plurality of training biological samples in the selected subset **412**

( B )

FIG. 4A

B

Calculate for each selected subset, a second quartile value of the distribution of each of the features across the training biological samples belonging to the healthy class (Q2_A) ) in the selected subset and the training biological samples belonging to the unhealthy class (Q2_B) in the selected subset **414**

Calculate Q1, Q3 Q2_A Q2_B for each of a predefined number of subsets (M) **416**

Calculate median value for all calculated Q1, median value for all calculated Q3, median value for all calculated Q2_A and median value for all calculated Q2_B **418**

Perform a Mann-Whitney test to test if a value of the feature is significantly different between the samples belonging to the healthy class and the samples belonging to the unhealthy class **420**

Shortlist the features based on a first predefined criteria utilizing calculated median values and the Mann-Whitney test **422**

Generate a set of features using the shortlisted features using a second predefined criteria, wherein the set of features are less than or equal to 15 **424**

Create a plurality of combinations of the features present in the set of features to generate corresponding plurality of candidate feature sets, wherein the plurality of combinations of features comprises a minimum of two and a maximum of 15 features **426**

C

FIG. 4B

$$C$$

Build a plurality of candidate models corresponding to each of the plurality of candidate feature sets **428**

Calculate a model evaluation score (MES) corresponding to each of the plurality of candidate models **430**

Select a model out of the plurality of candidate models as the best model using the highest model evaluation score wherein the selected model is tagged as a forward model **432**

Swap the tags assigned to the healthy class and the unhealthy class of the plurality of training biological samples present in the training data **434**

Identify a model as a reverse model by repeating the steps (**404** to **432**) for the training data obtained after swapping the tags **436**

Generate a plurality of forward models and a plurality of reverse models for a predefined number of times using randomly partitioned internal training set and the internal test set **438**

Generate an ensemble of forward models using the plurality of forward models and an ensemble of reverse models using the plurality of reverse models **440**

Identify a best forward model and a best reverse model using the model evaluation score **442**

Choose a final single model (FM_single) as the ensemble classification model from amongst the best forward model and the best reverse model based on how they classify the individua ltraining biological samples from the training data **444**

FIG. 4C

500

Collect a plurality of biological samples from a cohort comprised of a plurality of subjects, wherein each of the plurality of subjects in the cohort belong to one of the MBD categories from (i) the high category, (ii) the medium category, and (iii) the low category 502

Extract the microbial Deoxyribonucleic Acid (DNA) from each of the plurality of biological samples, using the one or more DNA extraction techniques 504

Sequence the microbial DNA, using one or more sequencing techniques, to generate a sequence data corresponding to each of the plurality of biological samples 506

Generate a microbial abundance profile corresponding to each biological sample, based on the corresponding sequence data, using one or more computational methodologies 508

Form a training data (TR) comprising a plurality of microbial abundance profiles of the plurality of biological samples, by collating each microbial abundance profile corresponding to each of the plurality of biological samples 510

Partition the training data (TR) randomly into a train set (TRS) and a test set (TSS), based on a pre-defined split parameter (S ), wherein S % samples from the training data constitute the TRS set and (100-S ) % of the samples constitute the TSS set, wherein the train set (TRS) comprises of a plurality of train biological samples and the test set (TSS) comprises of a plurality of test biological samples, and wherein a stratified sampling approach is adopted while partitioning the TR, into the TRS and the TSS, with an intent of preserving an original relative proportion of samples belonging to a class A, a class B and a class C, in the TRS and the TSS, wherein the class A corresponds to the low category of MBD, the class B corresponds to the medium category of MBD and the class C corresponds to the high category of MBD 512

(D)

FIG. 5A

(D)

Generate a plurality of train subsets from the TRS and a plurality of test subsets from the TSS, using repeated random sampling **514**

Re-label each train biological sample present in the plurality of train subsets corresponding to (i) the class A and the class B as a class X, and (ii) the class C as a class Y **516**

Generate a plurality of bipartite classification models, wherein each bipartite classification model is generated using the train biological samples present in each train subset and corresponding re-labelling **518**

Classify each test biological sample present in each test subset in the TSS, using the corresponding bipartite classification model, wherein the classification assigns each test biological sample with (i) one of the class X and the class Y, and (ii) generates a scaled model score (SMS) for each test biological sample **520**

Map and retag each test biological sample present in each test subset in the TSS with one of the class A, the class B and the class C **522**

Randomly draw a predefined number of test biological samples, from each subset of the plurality of test subsets, and assigning an index value in an ascending order starting from 1, based on the corresponding SMS scores **524**

Compute a median of the index values corresponding to each test subset in the TSS belonging to individual class labels A, B, and C, wherein the class labels corresponding to the sorted median value list indicates the class-label order for that TSS, if the computed medians are sorted in ascending order **526**

(E)

FIG. 5B

E

Iterate the step (**526**), for a predefined number of times, with the predefined number of test biological samples from each test subset, to obtain a predefined number of class-label orders for each test subset in the TSS **528**

Finalize the class-label order occurring the maximum number of times as the class-label order for each test subset in the TSS **530**

Determine a first threshold and a second threshold between -1 and +1, configured to partition the test biological samples based on corresponding SMS scores in the TSS into three groups, wherein the first threshold demarcates the test biological samples corresponding to the first class label in the determined class-label order from the test biological samples corresponding to the remaining two class labels, and the second threshold demarcates the test biological samples corresponding to last class label in the determined class-label order from the test biological samples corresponding to the remaining two class labels **532**

Select the candidate threshold pair having the maximum S_2 value, wherein the threshold values in this pair are used for classifying each test biological sample into one of the three class labels i.e. A or B or C **534**

Repeat steps (**512**) to (**536**) by considering the complete abundance data as the TSS in order to get the two best thresholds for tag categorization using all the available samples for training **536**

Compare the final prediction score obtained for a new test sample against the two best thresholds for classifying the new test sample to a particular MBD tag category **538**

Determine the MBD tag categories using following criteria: (i) if final prediction score <= threshold 1, tag the MBD category as low category, (ii) if threshold 1 < final prediction score < threshold 2, tag the MBD category as medium category, and (iii) if final prediction score >= threshold 2, tag the MBD category as high category **540**

FIG. 5C

600

Subject

Input
module
**602**

Output module
**604**

Hardware processor(s)
**606**

FIG. 6